# EUROPEAN PATENT SPECIFICATION

(11) **EP 3 619 207 B1**
(45) Date of publication and mention of the grant of the patent: **23.06.2021**
(21) Application number: 18718474.2
(22) Date of filing: 25.04.2018
(51) Int. Cl.: C07D 413/12, C07D 261/04, A01N 43/80, A01P 3/00

(54) **SUBSTITUTED 5-(HALOALKYL)-5-HYDROXY-ISOXAZOLINES FOR COMBATING PHYTOPATHOGENIC FUNGI**
SUBSTITUIERTE 5-(HALOGENALKYL)-5-HYDROXY-ISOXAZOLINE ZUR BEKÄMPFUNG VON PHYTOPATHOGENEN PILZEN
ISOXAZOLINES SUBSTITUÉES 5-(HALOALKYL)-5-HYDROXY POUR LUTTER CONTRE DES CHAMPIGNONS PHYTOPATHOGÈNES

(30) Priority: 04.05.2017 EP 17169490; 11.05.2017 EP 17170597
(43) Date of publication of application: 11.03.2020
(73) Proprietor: BASF SE, 67056 Ludwigshafen am Rhein (DE)
(72) Inventor: WINTER, Christian Harald, 67056 Ludwigshafen (DE); ESCRIBANO CUESTA, Ana, 67056 Ludwigshafen (DE); CRAIG, Ian Robert, 67056 Ludwigshafen (DE); GRAMMENOS, Wassilios, 67056 Ludwigshafen (DE); TERTERYAN-SEISER, Violeta, 67056 Ludwigshafen (DE); GROTE, Thomas, 67056 Ludwigshafen (DE); WIEBE, Christine, 67056 Ludwigshafen (DE); CAMBEIS, Erica, 67056 Ludwigshafen (DE); LOHMANN, Jan Klaas, 67056 Ludwigshafen (DE); SEET, Michael, 67056 Ludwigshafen (DE); MUELLER, Bernd, 67056 Ludwigshafen (DE)
(74) Representative: BASF IP Association
(86) International application number: PCT/EP2018/060542
(87) International publication number: WO 2018/202487

(56) References cited:
- WO-A1-99/05130
- WO-A1-2008/006561
- WO-A1-2015/129773
- WO-A1-2017/055469

## Description

The present invention relates to 5-(haloalkyl)-5-hydroxy-isoxazoles of the formula I, or the N-oxides, or the agriculturally useful salts thereof, and the use thereof for controlling phytopathogenic fungi; to a method for combating phytopathogenic harmful fungi, which process comprises treating the fungi, the plants, the soil or seeds to be protected against fungal attack, with an effective amount of at least one compound of the formula I, or an N-oxide, or an agriculturally acceptable salt thereof; and to agrochemical compositions comprising at least one compound of the formula I, or an N-oxide, or an agriculturally acceptable salt thereof, and further comprising seeds.

EP 276432 A2 relates to 3-phenyl-5-trifluoromethyloxadiazole derivatives and to their use to combat phytopathogenic microorganisms. WO 97/30047 A1, WO 2015/185485 A1, WO 2017/055469 A1 and WO 2017/055473 A1 describe other derivatives of trifluoromethyloxadiazoles and their use to combat phytopathogenic microorganisms.

WO 2008/006561 A1 relates to substituted 4H-isoxazoles and to their use as pharmaceuticals for the treatment of diseases known to be mediated by HDAC (histone deacetylase) activity.

In many cases, in particular at low application rates, the fungicidal activity of known fungicidal compounds is unsatisfactory. Based on this, it was an objective of the present invention to provide compounds having improved activity and/or a broader activity spectrum against phytopathogenic fungi. This objective is achieved by the isoxazoles of the formula I or their agriculturally useful salts for controlling phytopathogenic fungi.

Accordingly, the present invention relates to the use of compounds of the formula I, or the N-oxides, or the agriculturally acceptable salts thereof, for combating phytopathogenic harmful fungi, 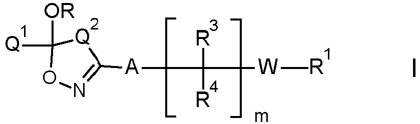 wherein:
- Q¹: is CHF₂ or CF₃;
- Q²: is -CH₂- or -CF₂-;
- R: is hydrogen, C₁-C₄-alkyl, C₃-C₈-cycloalkyl, C₁-C₆-alkyl-C(=O)-O-CH₂-, C₃-C₆-cycloalkyl-C(=O)-O-CH2-, -Si(C₁-C₄-alkyl)₃ or -(C=O)-R^{x};
R^{X} is C₁-C₆-alkyl, C₂-C₆-alkenyl, C₁-C₆-alkoxy, C₁-C₄-alkoxy-C₁-C₄-alkyl, C₃-C₈-cycloalkyl, -N(R^{Xa})₂, phenyl or a 3- to 6-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein any of the above-mentioned aliphatic or cyclic groups are unsubstituted or substituted with 1, 2, 3 or up to the maximum possible number of groups R^{xb}; wherein
R^{xa} is independently selected from the group consisting of hydrogen, C₁-C₆-alkyl, C₂-C₆-alkenyl, C₂-C₆-alkynyl, C₃-C₈-cycloalkyl, C₃-C₈-cycloalkenyl, C₁-C₆-alkoxy, C₁-C₄-alkoxy-C₁-C₄-alkyl and C₁-C₆-alkylthio;
R^{xb} is independently selected from the group consisting of halogen, C₁-C₆-alkyl, C₁-C₆-alkoxy, C₁-C₆-haloalkyl, C₁-C₆-haloalkoxy and C₃-C₈-cycloalkyl;
- A: is phenyl or a 5- or 6-membered aromatic heterocycle; wherein the ring member atoms of the aromatic heterocycle include besides carbon atoms 1, 2, 3 or 4 heteroatoms selected from N, O and S as ring member atoms with the provision that the heterocycle cannot contain 2 contiguous atoms selected from O and S; and wherein the phenyl ring or the aromatic heterocycle is unsubstituted or substituted with 1, 2, 3 or 4 identical or different groups R^{A}; wherein
R^{A} is halogen, cyano, C₁-C₆-alkyl, C₁-C₆-haloalkyl, C₁-C₆-alkoxy or C₁-C₆-haloalkoxy;
- W: is -(C=O)-NR²-#, -(C=S)-NR²-#, -S(=O)ₚ-NR²-#, -NR²-(C=O)-#, -NR²-(C=S)-#, -NR²-S(=O)ₚ-#, -NR²-(C=O)-NR²-#, -NR²-(C=S)-NR²-#, -NR²-S(=O)ₚ-NR²-#, -(C=O)-NR²-NR²-#, -(C=S)-NR²-NR²-#, -S(=O)ₚ-NR²-NR²-#, -NR²-NR²-(C=O)-#, -NR²-NR²-(C=S)-#, -NR²-NR²-S(=O)ₚ-#, -NR²-(C=O)-NR²-NR²-#, -NR²-(C=S)-NR²-NR²#, -NR²-S(=O)ₚ-NR²-NR²#, -NR²-NR²-(C=O)-NR²-#, -NR²-NR²-(C=S)-NR²-#, -NR²-NR²-S(=O)ₚ-NR²-#, -O-(C=O)-NR²-#, -O-(C=S)-NR²-#, -NR²-(C=O)-O-# or -NR²-(C=S)-O-#, wherein # denotes the position, which is attached to R¹;
- p: is 0, 1 or 2;
- R²: is independently selected from the group consisting of hydrogen, C₁-C₆-alkyl, C₂-C₆-alkenyl, C₂-C₆-alkynyl, C₁-C₆-alkoxy, C₃-C₈-cycloalkyl, C₃-C₈-cycloalkenyl, C₃-C₈-cycloalkyl-C₁-C₄-alkyl, phenyl-C₁-C₄-alkyl, phenyl, pyridinyl, C(=O)-(C₁-C₆-alkyl), C(=O)-(C₁-C₆-alkoxy) and -N(R^{2a})₂; wherein
R^{2a} is independently selected from the group consisting of hydrogen, OH, C₁-C₆-alkyl, C₂-C₆-alkenyl, C₂-C₆-alkynyl, C₃-C₈-cycloalkyl, C₃-C₈-cycloalkenyl, C₁-C₆-alkoxy, C₁-C₄-alkoxy-C₁-C₄-alkyl and C₁-C₆-alkylthio;
and wherein any of the aliphatic or cyclic groups in R² are unsubstituted or substituted with 1, 2, 3 or up to the maximum possible number of identical or different radicals selected from the group consisting of halogen, hydroxy, oxo, cyano, C₁-C₆-alkyl, C₁-C₆-alkoxy and C₃-C₈-cycloalkyl;
- R¹: is C₁-C₆-alkyl, C₁-C₆-alkoxy, C₃-C₈-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O and S as ring member atoms with the provision that the heterocycle cannot contain 2 contiguous atoms selected from O and S; and wherein any of the above-mentioned aliphatic or cyclic groups are unsubstituted or substituted with 1, 2, 3 or up to the maximum possible number of identical or different groups R^{1a}; or R¹ is a bicyclic carbocycle of the formula R^{a} 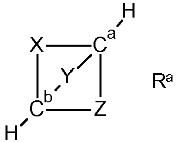
wherein
- C^{a} and C^{b}: are bridgehead carbon atoms;
- X: is a direct single bond or a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-;
- Y and Z: independently of each other are a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-; or R¹ is a tricyclic carbocycle of the formula R^{b} 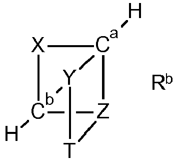
wherein
- C^{a} and C^{b}: are bridgehead carbon atoms;
- X: is a direct single bond or a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-;
- Y and Z: independently of each other are a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-; and wherein groups Y and Z are attached to the bridgehead carbon atoms C^{a} and C^{b};
- T: is a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-; and wherein the group T is attached to one carbon atom in each of the groups Y and Z; and with the proviso that, if R¹ is a tricyclic carbocycle of the formula R^{b}, wherein X is a direct single bond or a divalent group -CH₂-, the groups T and Z independently of each other are a divalent group selected from the group consisting of -CH₂-CH₂-, -(CH₂)₃-, -(CH₂)₄-, -CH=CH-, -CH₂-CH=CH-, -CH=CH-CH₂- and -CH=CH-CH=CH-; and wherein the groups R^{a} or R^{b} are connected to the group W through one of the ring carbon atoms; and wherein the groups R^{a} or R^{b} are unsubstituted or substituted with 1, 2, 3, 4 or up to the maximum possible number of radicals selected from the group consisting of oxo, hydroxy, halogen, C₁-C₃-alkyl, C₁-C₃-haloalkyl, C₃-C₆-cycloalkyl, vinylidene and dichlorovinylidene;
- or R¹: and one of the groups R², together with the nitrogen atom to which R² is attached, and together with interjacent groups, if any, which are located between said nitrogen atom and the group R¹, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O and S as ring member atoms with the provision that the heterocycle cannot contain 2 contiguous atoms selected from O and S; and wherein the heterocycle is unsubstituted or substituted with 1, 2, 3, 4 or up to the maximum possible number of identical or different groups R^{1a};
- or, if R²: is -N(R^{2a})₂, R¹ and one of the two groups R^{2a}, together with the nitrogen atom to which R^{2a} is attached, and together with interjacent groups, which are located between said nitrogen atom and the group R¹, form a saturated or partially unsaturated mono- or bicyclic 3- to 10-membered heterocycle, wherein the heterocycle includes beside two nitrogen atoms and one or more carbon atoms no further heteroatoms or 1, 2 or 3 further heteroatoms independently selected from N, O and S as ring member atoms with the provision that the heterocycle cannot contain 2 contiguous atoms selected from O and S; and wherein the heterocycle is unsubstituted or substituted with 1, 2, 3, 4 or up to the maximum possible number of identical or different groups R^{1a};
- R^{1a}: is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, (C=O)-C₁-C₄-alkyl, C(=O)-C₁-C₄-alkoxy, C₁-C₆-alkylsulfonyl, hydroxyC₁-C₄-alkyl, C(=O)-NH₂, C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl or C₁-C₄-alkoxy-C₁-C₄-alkyl;
- m: is 0 or 1;
- R³, R⁴: independently of each other are selected from the group consisting of hydrogen, halogen, cyano, C₁-C₄-alkyl, C₁-C₄-alkenyl, C₁-C₄-alkynyl, C₁-C₄-haloalkyl and C₁-C₄-alkoxy; or
- R³ and: R⁴ together with the carbon atom to which they are bound form a saturated 3- to 7-membered carbocycle or a saturated 3- to 6-membered heterocycle; wherein the saturated heterocycle includes beside carbon atoms 1, 2 or 3 heteroatoms independently selected from the group consisiting of N, O and S as ring member atoms with the provision that the heterocycle cannot contain 2 contiguous atoms selected from O and S; and wherein said N ring member atom is substituted with the group R^{N}; wherein
- R^{N}: is hydrogen, C₁-C₆-alkyl or halogen; and wherein said S ring member atom is unsubstituted or substituted with 1 or 2 oxo radicals; and wherein one or two CH₂ groups of the saturated carbocycle or of the saturated heterocycle may be replaced by one or two groups independently selected from -C(=O)- and -C(=S)-; and wherein the carbon ring member atoms of the saturated carbocycle or of the saturated heterocycle are unsubstituted or substituted with a total number of 1, 2, 3, 4 or up to the maximum possible number of identical or different radicals selected from the group consisting of halogen, cyano, C₁-C₆-alkyl, C₁-C₆-alkoxy and C₃-C₈-cycloalkyl.

Agriculturally acceptable salts of the compounds of the formula I encompass especially the salts of those cations or the acid addition salts of those acids whose cations and anions, respectively, have no adverse effect on the fungicidal action of the compounds I. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may be substituted with one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of acceptable acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound I with an acid of the corresponding anion, preferably of hydrochloric acid, hydrobromic acid, sulfuric acid, phosphoric acid or nitric acid. Compounds of the formula I can exist as one or more stereoisomers. The various stereoisomers include enantiomers, diastereomers, atropisomers arising from restricted rotation about a single bond of asymmetric groups and geometric isomers. They also form part of the subject matter of the present invention. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, e.g. a racemate, individual stereoisomers, or as an optically active form. Compounds of the formula I can be present in different crystal modifications whose biological activity may differ. They also form part of the subject matter of the present invention.

In respect of the variables, the embodiments of the intermediates obtained during preparation of compounds I correspond to the embodiments of the compounds of formula I. The term "compounds I" refers to compounds of the formula I.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "oxo" refers to an oxygen atom =O, which is bound to a carbon atom or sulfur atom, thus forming, for example, a ketonyl -C(=O)- or sulfinyl -S(=O)- group.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₄-alkylthio-C₁-C₄-alkyl" refers to alkyl having 1 to 4 carbon atoms (as defined above), wherein one hydrogen atom of the alkyl radical is replaced by a C₁-C₄-alkylthio group.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group.

The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethylimino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms, wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms, wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms, wherein two hydrogen atoms of one carbon atom of the alkyl radical are replaced by a divalent C₂-C₆-alkynyloxyimino radical (C₂-C₆-alkynyl-O-N=).

The term "C₃-C₈-cycloalkyl-C₁-C₆-alkyl" refers to alkyl having 1 to 6 carbon atoms, wherein one hydrogen atom of the alkyl radical is replaced by a C₃-C₈-cycloalkyl group.

The term "hydroxyC₁-C₄-alkyl" refers to alkyl having 1 to 4 carbon atoms, wherein one hydrogen atom of the alkyl radical is replaced by a OH group.

The term "aminoC₁-C₄-alkyl" refers to alkyl having 1 to 4 carbon atoms, wherein one hydrogen atom of the alkyl radical is replaced by a NH₂ group.

The term "C₁-C₆-alkylamino" refers to an amino group, which is substituted with one residue independently selected from the group that is defined by the term C₁-C₆-alkyl. Likewise the term "diC₁-C₆-alkylamino" refers to an amino group, which is substituted with two residues independently selected from the group that is defined by the term C₁-C₆-alkyl.

The term "C₁-C₄-alkylamino-C₁-C₄-alkyl" refers to refers to alkyl having 1 to 4 carbon atoms (as defined above), wherein one hydrogen atom of the alkyl radical is replaced by a C₁-C₄-alkyl-NH- group which is bound through the nitrogen. Likewise the term "diC₁-C₄-alkylamino-C₁-C₄-alkyl" refers to refers to alkyl having 1 to 4 carbon atoms (as defined above), wherein one hydrogen atom of the alkyl radical is replaced by a (C₁-C₄-alkyl)₂N- group which is bound through the nitrogen.

The term "aminocarbonyl-C₁-C₄-alkyl" refers to alkyl having 1 to 4 carbon atoms, wherein one hydrogen atom of the alkyl radical is replaced by a -(C=O)-NH₂ group.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₈-cycloalkyl" refers to monocyclic saturated hydrocarbon radicals having 3 to 8 carbon ring members such as cyclopropyl (C₃H₅), cyclobutyl, cyclopentyl, cyclohexyl, cycloheptyl or cyclooctyl.

The term "C₃-C₈-cycloalkyl" refers to monocyclic saturated hydrocarbon radicals having 3 to 8 carbon ring members such as cyclopropyl (C₃H₅), cyclobutyl, cyclopentyl, cyclohexyl, cycloheptyl or cyclooctyl.

The terms "C(=O)-C₁-C₄-alkyl" or "C(=O)-C₁-C₄-alkoxy" refer to a radical which is attached through the carbon atom of the -C(=O)- group as indicated by the number valence of the carbon atom.

The term "aliphatic" refers to compounds or radicals composed of carbon and hydrogen and which are non-aromatic compounds. An "alicyclic" compound or radical is an organic compound that is both aliphatic and cyclic. They contain one or more all-carbon rings which may be either saturated or unsaturated, but do not have aromatic character.

The terms "cyclic moiety" or "cyclic group" refer to a radical which is an alicyclic ring or an aromatic ring, such as, for example, phenyl or heteroaryl.

The term "and wherein any of the aliphatic or cyclic groups are unsubstituted or substituted with..." refers to aliphatic groups, cyclic groups and groups, which contain an aliphatic and a cyclic moiety in one group, such as in, for example, C₃-C₈-cycloalkyl-C₁-C₄-alkyl; therefore a group which contains an aliphatic and a cyclic moiety both of these moieties may be substituted or unsubstituted independently of each other.

The term "vinylidene" refers to a group =CH₂, the term "dichlorovinylidene" refers to a group =CCl₂.

The term "wherein R¹ is connected to the group W through one of the ring carbon atoms of the groups R^{a} or R^{b}" in the context of this invention means that R¹ is attached to the group W through one carbon atom of the groups R^{a} or R^{b}, which includes any carbon atom of groups X, Y, Z, T and the bridgehead carbon atoms C^{a} and C^{b}, thereby substituting a hydrogen atom on said carbon atom.

The term "phenyl" refers to an aromatic ring systems incuding six carbon atoms (commonly referred to as benzene ring. In association with the group A the term "phenyl" is to be interpreted as a benzene ring or phenylene ring, which is attached to both, the isoxazole moiety and the -CR³R⁴- or W group.

The term "heteroaryl" refers to aromatic monocyclic or polycyclic ring systems incuding besides carbon atoms, 1, 2, 3 or 4 heteroatoms independently selected from the group consisting of N, O and S.

The term "saturated 3- to 7-membered carbocycle" is to be understood as meaning monocyclic saturated carbocycles having 3, 4 or 5 carbon ring members. Examples include cyclopropyl, cyclopentyl, cyclohexyl, cycloheptyl, and the like.

The term "3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S", is to be understood as meaning both, aromatic mono- and bicyclic heteroaromatic ring systems, and also saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine;
and a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

In respect of the variables, the embodiments of the intermediates correspond to the embodiments of the compounds I. Preference is given to those compounds I and, where applicable, also to compounds of all subformulae provided herein, e. g. formulae I.1, 1.2, I.1a, I.A, I.B, I.C, I.D, I.E, I.F, I.G, I.H, I.J, I.K, I.L, I.M, I.N, I.O, I.P, I.Q, I.R, I.S, I.T, I.U, I.V, I.W, I.X, I.Y, I.Z1, I.Z2, I.Z3, I.Z4, I.Z5, I.Z6, I.Z7, I.Z8, I.Z9, I.Z10, I.Z11, I.Z12, I.Z13 and I.Z14, wherein the variables have independently of each other or more preferably in combination (any possible combination of 2 or more substituents as defined herein) the following meanings:
In one aspect of the invention Q¹ is CHF₂ or CF₃; particularly CF₃. In one embodiment Q² is - CF₂-. In one aspect of the invention Q² is -CH₂-.

In another embodiment Q¹ is CF₃ and Q² is -CF₂-. In a further embodiment Q¹ is CHF₂ and Q² is -CF₂-. In another embodiment Q¹ is CF₃ and Q² is -CH₂-. In a further embodiment Q¹ is CHF₂ and Q² is -CH₂-.

In one embodiment of the invention R is hydrogen, C₁-C₄-alkyl, C₁-C₆-alkyl-C(=O)-O-CH₂-, C₃-C₆-cycloalkyl-C(=O)-O-CH₂-, C₃-C₈-cycloalkyl, -Si(C₁-C₄-alkyl)₃ or -(C=O)-R^{X}; wherein
- R^{X}: is C₁-C₆-alkyl, C₂-C₆-alkenyl, C₁-C₆-alkoxy, C₁-C₄-alkoxy-C₁-C₄-alkyl, C₃-C₈-cycloalkyl, -N(R^{Xa})₂, phenyl or a 5- or 6-membered saturated, partially unsaturated or aromatic mono- or bicyclic heterocycle, wherein the ring member atoms of said mono- or bicyclic heterocycle include besides carbon atoms further 1 or 2 heteroatoms selected from N, O and S as ring member atoms with the provision that the heterocycle cannot contain 2 contiguous atoms selected from O and S; and wherein any of the above-mentioned aliphatic or cyclic groups are unsubstituted or substituted with 1, 2, 3 or up to the maximum possible number of groups R^{xb}; wherein
R^{xa} is independently selected from the group consisting of hydrogen and C₁-C₆-alkyl;
R^{xb} is independently selected from the group consisting of halogen, C₁-C₆-alkyl, C₁-C₆-alkoxy, C₁-C₆-haloalkyl, C₁-C₆-haloalkoxy and C₃-C₈-cycloalkyl.

Embodiment R.1: R is hydrogen, C₁-C₄-alkyl, C₁-C₆-alkyl-C(=O)-O-CH₂-, C₃-C₆-cycloalkyl-C(=O)-O-CH₂-, C₃-C₈-cycloalkyl, -Si(C₁-C₄-alkyl)₃ or -(C=O)-R^{X}, wherein R^{X} is C₁-C₄-alkyl, C₁-C₄-alkoxy, C₁-C₄-alkoxy-C₁-C₄-alkyl, C₃-C₈-cycloalkyl, -N(R^{xa})₂, a 5- or 6-membered saturated, partially unsaturated or aromatic mono- or bicyclic heterocycle, wherein the ring member atoms of said mono- or bicyclic heterocycle include besides carbon atoms further 1 or 2 heteroatoms selected from N, O and S as ring member atoms with the provision that the heterocycle cannot contain 2 contiguous atoms selected from O and S; and wherein R^{xa} is independently selected from hydrogen and C₁-C₄-alkyl.

Embodiment R.2: R is hydrogen or C₁-C₄-alkyl; preferably hydrogen, methyl or ethyl; more preferably hydrogen.

Embodiment R.3: R is (C=O)-C₁-C₄-alkyl or C(=O)-C₁-C₄-alkoxy.

Embodiment R.4: R is C₁-C₆-alkyl-C(=O)-O-CH₂-, C₃-C₆-cycloalkyl-C(=O)-O-CH₂-. Embodiment R.5: R is -(C=O)-N(R^{xa})₂, wherein R^{xa} is independently selected from hydrogen and C₁-C₄-alkyl; preferably from hydrogen, methyl and ethyl.

Embodiment R.6: R is -(C=O)-R^{X}, wherein R^{X} is C₁-C₄-alkyl, C₁-C₄-alkoxy, C₁-C₄-alkoxy-C₁-C₄-alkyl, C₃-C₈-cycloalkyl or -N(R^{xa})₂, wherein R^{xa} is independently selected from hydrogen and C₁-C₄-alkyl.

Embodiment R.7: R is -(C=O)-R^{x}, wherein R^{X} is a 5- or 6-membered saturated, partially unsaturated or aromatic mono- or bicyclic heterocycle, wherein the ring member atoms of said mono- or bicyclic heterocycle include besides carbon atoms further 1 or 2 heteroatoms selected from N, O and S as ring member atoms with the provision that the heterocycle cannot contain 2 contiguous atoms selected from O and S.

In one aspect of the invention A is phenyl which is unsubstituted or substituted with 1, 2, 3 or 4 identical or different groups R^{A} as defined or preferably defined herein and wherein the group -CR³R⁴- or W is attached to the phenyl ring in para-position with regard to the isoxazole group.

In one aspect of the invention A is phenyl which is unsubstituted or substituted with 1, 2, 3 or 4 identical or different groups R^{A} as defined or preferably defined herein and wherein the group -CR³R⁴- or W is attached to the phenyl ring in *meta*-position with regard to the isoxazole group.

In a further aspect of the invention A is phenyl which is substituted with 1 or 2 identical or different groups R^{A} as defined or preferably defined herein and wherein the group -CR³R⁴- or W is attached to the phenyl ring in *para*-position with regard to the isoxazole group.

In another aspect of the invention A is phenyl which is unsubstituted and wherein the group -CR³R⁴- or W is attached to the phenyl ring in *para*-position with regard to the isoxazole group.

In one embodiment A is a 6-membered aromatic heterocycle, wherein the ring member atoms of the aromatic heterocycle include besides carbon atoms 1 or 2 nitrogen atoms as ring member atoms with the provision that the heterocycle cannot contain 2 contiguous atoms selected from O and S; and wherein the aromatic heterocycle is unsubstituted or substituted with 1 or 2 identical or different groups R^{A} as defined or preferably defined herein; particularly R^{A} is chlorine, fluorine or methyl.

In a further embodiment A is a 6-membered aromatic heterocycle, wherein the ring member atoms of the aromatic heterocycle include besides carbon atoms 1 or 2 nitrogen atoms as ring member atoms with the provision that the heterocycle cannot contain 2 contiguous atoms selected from O and S; and wherein the aromatic heterocycle is unsubstituted or substituted with 1 or 2 identical or different groups R^{A} as defined or preferably defined herein; particularly R^{A} is chlorine, fluorine or methyl; and wherein the group -CR³R⁴- or W is attached to the 6-membered aromatic heterocycle in *para*-position with regard to the isoxazole group. In a further preferred embodiment A is a 5-membered aromatic heterocycle, in particular a thiophene ring, more particularly a 2,5-thiophenyl ring, wherein the ring member atoms of the heterocycle include besides carbon atoms 1, 2, 3 or 4 heteroatoms selected from N, O and S as ring member atoms with the provision that the heterocycle cannot contain 2 contiguous atoms selected from O and S; and wherein the cyclic groups A are unsubstituted or substituted with 1 or 2 identical or different groups R^{A} as defined or preferably defined herein; particularly R^{A} is chlorine, fluorine or methyl.

In one embodiment the invention relates to use of compounds of the formula I, wherein the cyclic moiety A is defined as in subformulae (A.1) to (A.30), 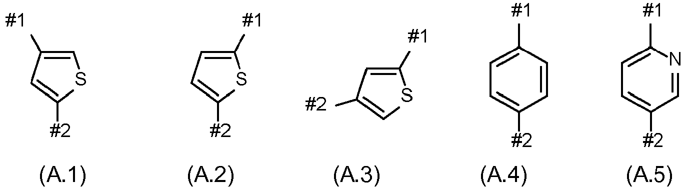 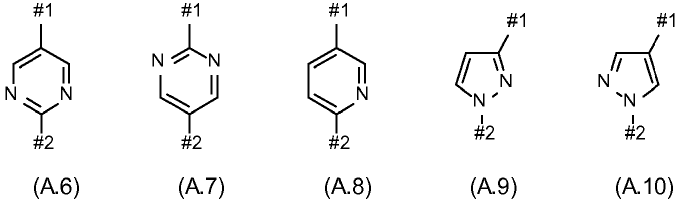 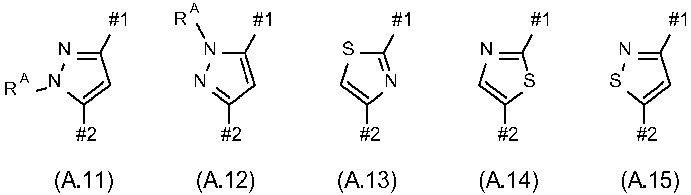 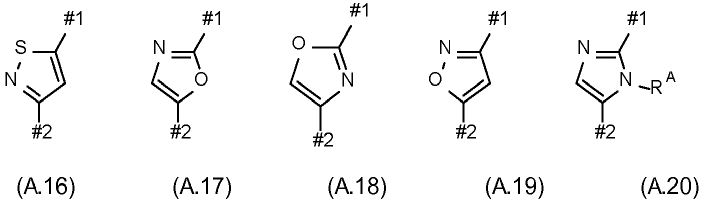 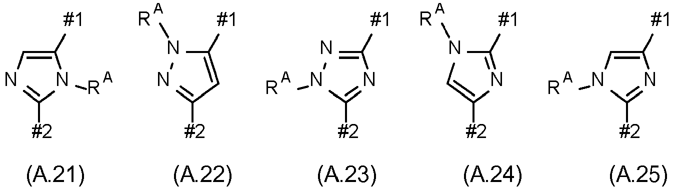 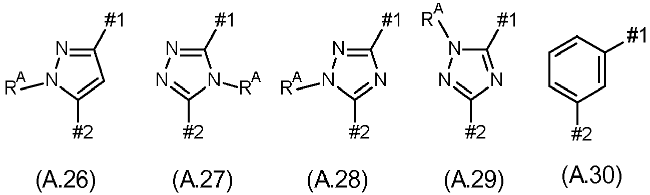 wherein #1 denotes the position which is bound to the isoxazole moiety and #2 denotes the position, which is connected to the group -CR³R⁴- or W of compounds of the formula I; and wherein the cyclic moiety A is unsubstituted or substituted with 1 or 2 identical or different groups R^{A} and wherein R^{A} is as defined or preferably defined herein. In another embodiment the cyclic moieties A as defined in any one of subformulae (A.1) to (A.30) is unsubstituted or substituted with 1 or 2 identical or different groups R^{A}; and wherein R^{A} is chlorine, fluorine or methyl. In a preferred embodiment the cyclic moiety A as defined in any one of subformulae (A.1) to (A.30) is unsubstituted.

In a preferred embodiment R^{A} is independently selected from the group consisting of halogen, C₁-C₆-alkyl or C₃-C₈-cycloalkyl. In another preferred embodiment R^{A} is independently selected from the group consisting of halogen, methyl or ethyl. More preferably R^{A} is independently selected from the group consisting of halogen, in particular R^{A} is fluorine.
Embodiment 1.1: R¹ is C₁-C₆-alkyl, C₂-C₆-alkenyl, C₂-C₆-alkynyl, C₃-C₈-cycloalkyl, C₃-C₈-cycloalkenyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms with the provision that the heterocycle cannot contain 2 contiguous atoms selected from O and S; and wherein any of the aliphatic or cyclic groups are unsubstituted or substituted with 1, 2, 3 or up to the maximum possible number of identical or different radicals R^{1a} as defined or preferably defined herein.
Embodiment 1.2: R¹ is phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms with the provision that the heterocycle cannot contain 2 contiguous atoms selected from O and S; and wherein any of the cyclic groups are unsubstituted or substituted with 1, 2, 3 or up to the maximum possible number of identical or different radicals R^{1a} as defined or preferably defined herein.
Embodiment 1.3: R¹ is C₃-C₈-cycloalkyl or C₃-C₈-cycloalkenyl; and wherein the cyclic group is unsubstituted or substituted with 1, 2, 3 or up to the maximum possible number of identical or different radicals R^{1a} as defined or preferably defined herein.
Embodiment 1.4: R¹ is C₁-C₆-alkyl; and wherein the alkyl group is unsubstituted or substituted with 1, 2, 3 or up to the maximum possible number of identical or different radicals R^{1a} as defined or preferably defined herein.
Embodiment 1.5: R¹ is difluoromethyl, trifluoromethyl, 2,2-difluoroethyl, 2,2,2-trifluoroethyl, 2-chloro-2-fluoroethyl, 2-chloro-2,2-difluoroethyl, 2,2,2-trichloroethyl and pentafluoroethyl, 3,3,3-trifluoropropyl, CH₂CF₂CF₃ or CF₂CF₂CF₅, CH(CH₃)CF₃, CH₂CF₂CH₃, CH₂C(CH₃)₂F, CH₂CH(CH₃)CF₃ or CH₂C(CH₃)₂CF₃.
Embodiment 1.6: R¹ is C₁-C₆-alkoxyimino-C₁-C₄-alkyl, C₂-C₆-alkenyloxyimino-C₁-C₄-alkyl or C₂-C₆-alkynyloxyimino-C₁-C₄-alkyl.
Embodiment 1.7: R¹ is a bicyclic carbocycle of the formula R^{a} 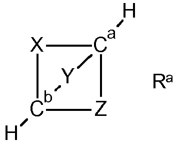 wherein
   - C^{a} and C^{b}: are bridgehead carbon atoms;
   - X: is a direct single bond or a divalent group selected from the group consisting of -CH₂- or -CH₂-CH₂-;
   - Y and Z: independently of each other are a divalent group selected from the group consisting of -CH₂- or -CH₂-CH₂-;
   and wherein R^{a} is connected to the remainder of the compounds of formula I through one of the ring carbon atoms; and wherein R^{a} is unsubstituted or substituted with 1 or 2 radicals selected from the group consisting of oxo, hydroxy, halogen, C₁-C₃-alkyl.
Embodiment 1.8: R¹ is a tricyclic carbocycle of the formula R^{b} 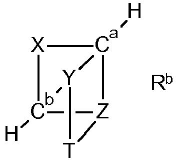 wherein
   - C^{a} and C^{b}: are bridgehead carbon atoms;
   - X: is a direct single bond or a divalent group selected from the group consisting of -CH₂- or -CH₂-CH₂-;
   - Y and Z: independently of each other are a divalent group selected from the group consisting of -CH₂- or -CH₂-CH₂-; and wherein groups Y and Z are attached to the bridgehead carbon atoms C^{a} and C^{b};
   - T: is a divalent group selected from the group consisting of -CH₂- or -CH₂-CH₂-; and wherein the group T is attached to one carbon atom in each of the groups Y and Z;
   and with the proviso that, if X is a direct single bond or a divalent group -CH₂-, the groups T and Z independently of each other are a divalent group -CH₂-CH₂-;
   and wherein R^{b} is connected to the remainder of the compounds of formula I through one of the ring carbon atoms; and wherein R^{b} is unsubstituted or substituted with 1 or 2 radicals selected from the group consisting of oxo, hydroxy, halogen, C₁-C₃-alkyl.
Embodiment 1.9: R¹ is a bicyclic or tricyclic carbocycle selected from the group consisting of radicals R¹.1 to R¹.31 below; wherein each radical may be connected to the remainder of the compounds of formula I through one of the ring carbon atoms by substitution of one hydrogen atom; and wherein R¹ is unsubstituted or substituted with 1 or 2 radicals selected from the group consisting of oxo, hydroxy, halogen and C₁-C₃-alkyl. 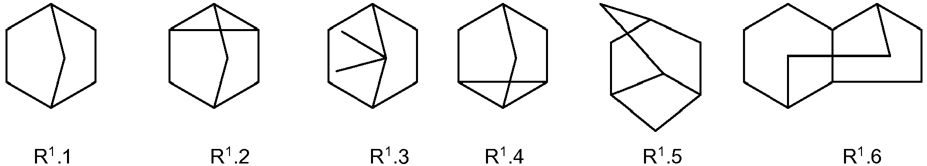 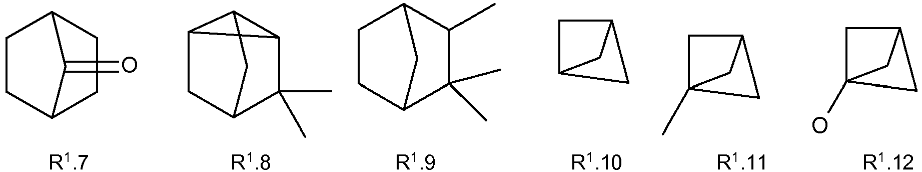 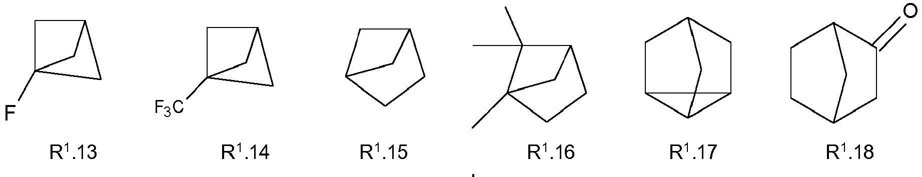 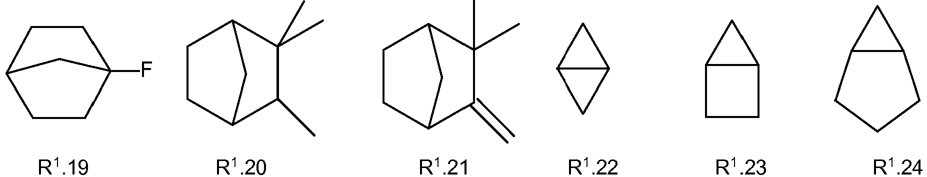 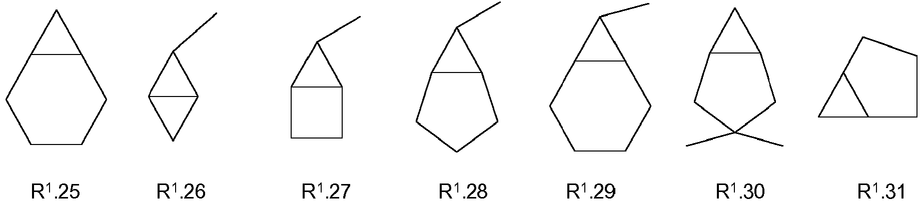
Embodiment 1.10: R¹ is selected from the group consisting of R¹.32 to R¹.57 below, particularly from R¹.32 to R¹.49, which are further unsubstituted, and wherein "#C" indicates the carbon atom, which is attached to the remainder of the compounds of formula I.

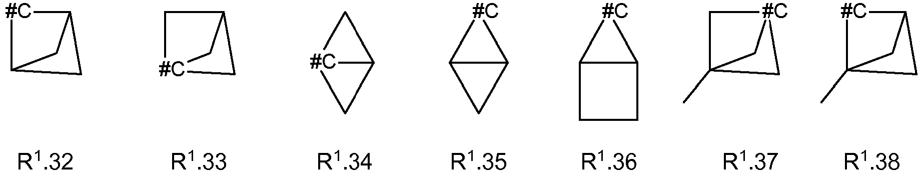

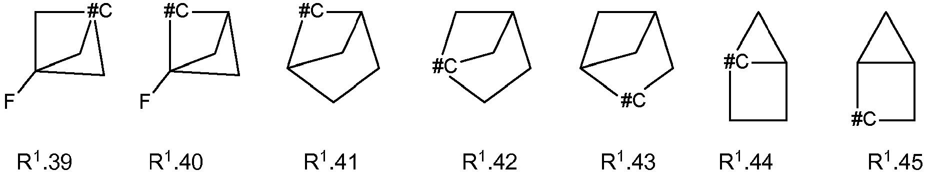

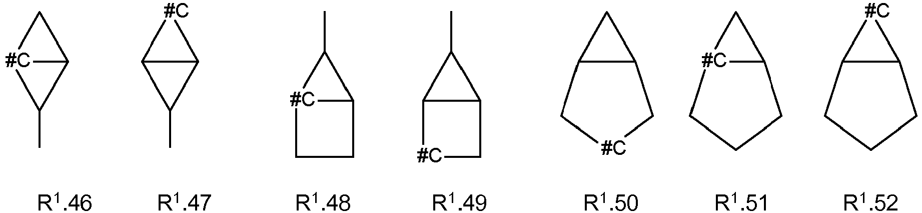

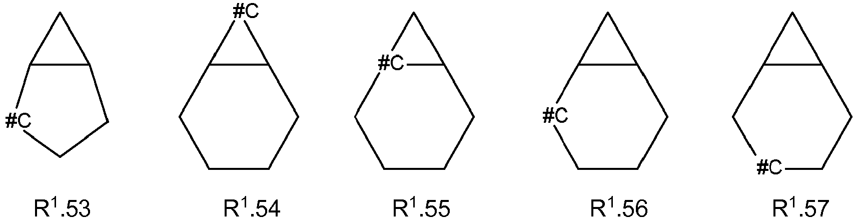

In one embodiment R^{1a} is selected from the group consisting of halogen, cyano, C₁-C₆-alkyl, C₁-C₆-haloalkyl, C₁-C₆-alkoxy, C₁-C₆-haloalkoxy and C₃-C₈-cycloalkyl.
In another aspect of the invention R^{1a} is selected from the group consisting of fluorine, chlorine, cyano, methyl, ethyl, methoxy, trifluoromethyl, trifluoromethoxy, difluoromethyl, difluoromethoxy or cyclopropyl.
In a preferred aspect of the invention R^{1a} is selected from the group consisting of halogen, C₁-C₆-alkyl and C₃-C₈-cycloalkyl; particularly from methyl, ethyl, fluorine and chlorine; more particularly from fluorine and chlorine.

In one aspect of the invention R² independently of each other are hydrogen, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, pyridinyl or -N(R^{2a})₂; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy; more preferably from halogen, in particular the radical is fluorine; and wherein R^{2a} is independently selected from the group consisting of hydrogen, OH, C₁-C₆-alkyl, C₂-C₆-alkenyl, C₂-C₆-alkynyl, C₃-C₈-cycloalkyl or C₁-C₆-alkoxy. Embodiment 2.1: R² independently of each other are hydrogen, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, C₁-C₆-alkylamino or diC₁-C₆-alkylamino; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy.
Embodiment 2.2: R² independently of each other are hydrogen, methyl, ethyl, *n*-propyl, *iso-*propyl, methoxy, ethyoxy, propyloxy, cyclopropyl, cyclopropyl-CH₂-, allyl, phenyl, 4-F-phenyl, 2-F-phenyl, C₁-C₆-alkylamino or diC₁-C₆-alkylamino.
Embodiment 2.3: R² independently of each other are hydrogen, C₁-C₆-alkyl, C₁-C₆-alkoxy, C₂-C₆-alkenyl, propargyl, C₃-C₈-cycloalkyl, C₁-C₆-alkylamino or diC₁-C₆-alkylamino. Embodiment 2.4: R² independently of each other are hydrogen, methyl, ethyl, *n*-propyl, *iso-*propyl, methoxy, ethyoxy, propyloxy, cyclopropyl, cyclopropyl-CH₂-, allyl, C₁-C₆-alkylamino or diC₁-C₆-alkylamino.
Embodiment 2.5: R² independently of each other are hydrogen, C₁-C₆-alkyl, C₁-C₆-alkoxy, C₂-C₆-alkenyl or propargyl, C₁-C₆-alkylamino or diC₁-C₆-alkylamino.
Embodiment 2.6: R² independently of each other are hydrogen, methy, ethyl, methoxy, ethyoxy, propyloxy, C₁-C₆-alkylamino or diC₁-C₆-alkylamino.
Embodiment 2.7: R² is hydrogen.

In one aspect R² independently of each other are hydrogen, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, pyridinyl or -N(R^{2a})₂; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy; more preferably from halogen, in particular the radical is fluorine; and wherein R^{2a} is independently selected from the group consisting of hydrogen, OH, C₁-C₆-alkyl, C₂-C₆-alkenyl, C₂-C₆-alkynyl, C₃-C₈-CyCloalkyl or C₁-C₆-alkoxy; and R¹ is C₁-C₆-alkyl, C₃-C₈-cycloalkyl, C₃-C₈-cycloalkenyl, C₂-C₆-alkenyl, C₂-C₆-alkynyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms with the provision that the heterocycle cannot contain 2 contiguous atoms selected from O and S; and wherein any of the aliphatic or cyclic groups in R¹ are unsubstituted or substituted with 1, 2, 3, 4 or up to the maximum possible number of identical or different radicals R^{1a} as defined or preferably defined herein.
In another aspect R² independently of each other are selected from the group consisting of hydrogen, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, C₁-C₆-alkylamino or diC₁-C₆-alkylamino; and R¹ is C₁-C₆-alkyl, C₃-C₈-cycloalkyl, C₃-C₈-Cycloalkenyl, C₂-C₆-alkenyl, C₂-C₆-alkynyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms with the provision that the heterocycle cannot contain 2 contiguous atoms selected from O and S; and wherein any of the aliphatic or cyclic groups in R¹ are unsubstituted or substituted with 1, 2, 3, 4 or up to the maximum possible number of identical or different radicals R^{1a} as defined or preferably defined herein.
In another aspect R² independently of each other are selected from the group consisting of hydrogen, methyl, ethyl, *n*-propyl, *iso*-propyl, methoxy, ethyoxy, propyloxy, cyclopropyl, cyclopropyl-CH₂-, allyl, C₁-C₆-alkylamino or diC₁-C₆-alkylamino; and R¹ is C₁-C₆-alkyl, C₃-C₈-cycloalkyl, C₃-C₈-cycloalkenyl, C₂-C₆-alkenyl or C₂-C₆-alkynyl; and wherein any of the aliphatic or cyclic groups in R¹ are unsubstituted or substituted with 1, 2, 3, 4 or up to the maximum possible number of identical or different radicals selected from the group consisting of halogen or C₁-C₆-alkyl.

In one aspect of the invention m is 0. In another aspect of the invention m is 1.
Embodiment 3.1: use of compounds of the formula I, wherein R³ and R⁴ independently of each other are hydrogen, halogen, C₁-C₆-alkyl or C₁-C₆-haloalkyl; or R³ and R⁴ together with the carbon atom to which they are bound form a cyclopropyl ring, wherein the cyclopropyl ring is unsubstituted.
Embodiment 3.2: use of compounds of the formula I, wherein R³ and R⁴ independently of each other are hydrogen or C₁-C₄-alkyl;
Embodiment 3.3: use of compounds of the formula I, wherein R³ and R⁴ independently of each other are hydrogen, methyl or ethyl.
Embodiment 3.4: use of compounds of the formula I, wherein R³ and R⁴ are independently of each other hydrogen, fluorine, chlorine, methyl or trifluoromethyl; or R³ and R⁴ together with the carbon atom to which they are bound form a cyclopropyl ring, wherein the cyclopropyl ring is unsubstituted.
Embodiment 3.5: use of compounds of the formula I, wherein R³ and R⁴ are both hydrogen.
Embodiment 3.6: use of compounds of the formula I, wherein R³ is hydrogen and R⁴ is methyl.
Embodiment 3.7: use of compounds of the formula I, wherein R³ and R⁴ are both methyl.
Embodiment 3.8: use of compounds of the formula I, wherein R³ and R⁴ are both fluorine.
Embodiment 3.9: use of compounds of the formula I, wherein R³ and R⁴ are both trifluoromethyl.
Embodiment 3.10: use of compounds of the formula I, wherein R³ and R⁴ together with the carbon atom to which they are bound a saturated monocyclic 3- to 5-membered saturated heterocycle or saturated carbocycle; and wherein the saturated heterocycle includes beside one or more carbon atoms no heteroatoms or 1 or 2 heteroatoms independently selected from N, O and S as ring member atoms with the provision that the heterocycle cannot contain 2 contiguous atoms selected from O and S; and wherein the heterocycle or the carbocycle is unsubstituted or substituted 1, 2, 3, 4 or up to the maximum possible number of identical or different radicals selected from the group consisting of halogen, cyano and C₁-C₂-alkyl.
Embodiment 3.11: use of compounds of the formula I, wherein R³ and R⁴ together with the carbon atom to which they are bound form a 3- or 4-membered carbocylic ring; and wherein the carbocylic ring is unsubstituted.
Embodiment 3.12: use of compounds of the formula I, wherein R³ and R⁴ together with the carbon atom to which they are bound form a cyclopropyl ring, wherein the cyclopropyl ring is unsubstituted.
Embodiment 3.13: use of compounds of the formula I, wherein R³ and R⁴ together with the carbon atom to which they are bound form a saturated 3-membered heterocycle; wherein the heterocycle includes beside two carbon atoms one heteroatom selected from N, O and S as ring member atoms with the provision that the heterocycle cannot contain 2 contiguous atoms selected from O and S; and wherein the heterocycle is unsubstituted or substituted 1, 2, 3, 4 or up to the maximum possible number of identical or different radicals selected from the group consisting of halogen, cyano and C₁-C₂-alkyl.
Embodiment 3.14: use of compounds of the formula I, wherein R³ is methyl and R⁴ is fluorine.
Embodiment 3.14: use of compounds of the formula I, wherein R³ is hydrogen and R⁴ is trifluoromethyl.

Further embodiments of the present invention relate to the use of compounds I, wherein group W is defined as follows:
Embodiment W.1: W is -(C=O)-NR²-#, -(C=S)-NR²-#, -S(=O)₂-NR²-#, -NR²-(C=O)-#, -NR²-(C=S)-# or -NR²-S(=O)₂-#.
Embodiment W.2: W is -NR²-(C=O)-NR²-#, -NR²-(C=S)-NR²-# or -NR²-S(=O)₂-NR²-#.
Emodiment W.3: W is -(C=O)-NR²-NR²-#, -(C=S)-NR²-NR²-#, -S(=O)₂-NR²-NR²-#, -NR²-NR²-(C=O)-#, -NR²-NR²-(C=S)-#, -NR²-NR²-S(=O)₂-#, -NR²-(C=O)-NR²-NR²-#, -NR²-(C=S)-NR²-NR²#2, -NR²-S(=O)₂-NR²-NR²#2, -NR²-NR²-(C=O)-NR²-#, -NR²-NR²-(C=S)-NR²-# or -NR²-NR²-S(=O)₂-NR²-#.
Embodiment W.4: W is -O-(C=O)-NR²-#, -O-(C=S)-NR²-#, -NR²-(C=O)-O-# or -NR²-(C=S)-O-#.

In all groups W # denotes the position, which is attached to the group R¹.

In further aspects of the present invention the embodiments E.1 to E.580 listed in Table E represent preferred combinations of the embodiments, which are defined above for each of the variables m, W, R¹, R², R³ and R⁴.

**Table E**

| **Embodiment** | **m** | **Embodiment W** | **Embodiment R¹** | **Embodiment R²** | **Embodiment R³, R⁴** |
|---|---|---|---|---|---|
| E.1 | 0 | W.1 | 1.1 | 2.1 | - |
| E.2 | 0 | W.1 | 1.2 | 2.1 | - |
| E.3 | 0 | W.1 | 1.3 | 2.1 | - |
| E.4 | 0 | W.1 | 1.4 | 2.1 | - |
| E.5 | 0 | W.1 | 1.5 | 2.1 | - |
| E.6 | 0 | W.1 | 1.6 | 2.1 | - |
| E.7 | 0 | W.1 | 1.7 | 2.1 | - |
| E.8 | 0 | W.1 | 1.8 | 2.1 | - |
| E.9 | 0 | W.1 | 1.9 | 2.1 | - |
| E.10 | 0 | W.1 | 1.10 | 2.1 | - |
| E.11 | 0 | W.1 | 1.1 | 2.4 | - |
| E.12 | 0 | W.1 | 1.2 | 2.4 | - |
| E.13 | 0 | W.1 | 1.3 | 2.4 | - |
| E.14 | 0 | W.1 | 1.4 | 2.4 | - |
| E.15 | 0 | W.1 | 1.5 | 2.4 | - |
| E.16 | 0 | W.1 | 1.6 | 2.4 | - |
| E.17 | 0 | W.1 | 1.7 | 2.4 | - |
| E.18 | 0 | W.1 | 1.8 | 2.4 | - |
| E.19 | 0 | W.1 | 1.9 | 2.4 | - |
| E.20 | 0 | W.1 | 1.10 | 2.4 | - |
| E.21 | 0 | W.2 | 1.1 | 2.1 | - |
| E.22 | 0 | W.2 | 1.2 | 2.1 | - |
| E.23 | 0 | W.2 | 1.3 | 2.1 | - |
| E.24 | 0 | W.2 | 1.4 | 2.1 | - |
| E.25 | 0 | W.2 | 1.5 | 2.1 | - |
| E.26 | 0 | W.2 | 1.6 | 2.1 | - |
| E.27 | 0 | W.2 | 1.7 | 2.1 | - |
| E.28 | 0 | W.2 | 1.8 | 2.1 | - |
| E.29 | 0 | W.2 | 1.9 | 2.1 | - |
| E.30 | 0 | W.2 | 1.10 | 2.1 | - |
| E.31 | 0 | W.2 | 1.1 | 2.4 | - |
| E.32 | 0 | W.2 | 1.2 | 2.4 | - |
| E.33 | 0 | W.2 | 1.3 | 2.4 | - |
| E.34 | 0 | W.2 | 1.4 | 2.4 | - |
| E.35 | 0 | W.2 | 1.5 | 2.4 | - |
| E.36 | 0 | W.2 | 1.6 | 2.4 | - |
| E.37 | 0 | W.2 | 1.7 | 2.4 | - |
| E.38 | 0 | W.2 | 1.8 | 2.4 | - |
| E.39 | 0 | W.2 | 1.9 | 2.4 | - |
| E.40 | 0 | W.2 | 1.10 | 2.4 | - |
| E.41 | 0 | W.3 | 1.1 | 2.1 | - |
| E.42 | 0 | W.3 | 1.2 | 2.1 | - |
| E.43 | 0 | W.3 | 1.3 | 2.1 | - |
| E.44 | 0 | W.3 | 1.4 | 2.1 | - |
| E.45 | 0 | W.3 | 1.5 | 2.1 | - |
| E.46 | 0 | W.3 | 1.6 | 2.1 | - |
| E.47 | 0 | W.3 | 1.7 | 2.1 | - |
| E.48 | 0 | W.3 | 1.8 | 2.1 | - |
| E.49 | 0 | W.3 | 1.9 | 2.1 | - |
| E.50 | 0 | W.3 | 1.10 | 2.1 | - |
| E.51 | 0 | W.3 | 1.1 | 2.4 | - |
| E.52 | 0 | W.3 | 1.2 | 2.4 | - |
| E.53 | 0 | W.3 | 1.3 | 2.4 | - |
| E.54 | 0 | W.3 | 1.4 | 2.4 | - |
| E.55 | 0 | W.3 | 1.5 | 2.4 | - |
| E.56 | 0 | W.3 | 1.6 | 2.4 | - |
| E.57 | 0 | W.3 | 1.7 | 2.4 | - |
| E.58 | 0 | W.3 | 1.8 | 2.4 | - |
| E.59 | 0 | W.3 | 1.9 | 2.4 | - |
| E.60 | 0 | W.3 | 1.10 | 2.4 | - |
| E.61 | 0 | W.4 | 1.1 | 2.1 | - |
| E.62 | 0 | W.4 | 1.2 | 2.1 | - |
| E.63 | 0 | W.4 | 1.3 | 2.1 | - |
| E.64 | 0 | W.4 | 1.4 | 2.1 | - |
| E.65 | 0 | W.4 | 1.5 | 2.1 | - |
| E.66 | 0 | W.4 | 1.6 | 2.1 | - |
| E.67 | 0 | W.4 | 1.7 | 2.1 | - |
| E.68 | 0 | W.4 | 1.8 | 2.1 | - |
| E.69 | 0 | W.4 | 1.9 | 2.1 | - |
| E.70 | 0 | W.4 | 1.10 | 2.1 | - |
| E.71 | 0 | W.4 | 1.1 | 2.4 | - |
| E.72 | 0 | W.4 | 1.2 | 2.4 | - |
| E.73 | 0 | W.4 | 1.3 | 2.4 | - |
| E.74 | 0 | W.4 | 1.4 | 2.4 | - |
| E.75 | 0 | W.4 | 1.5 | 2.4 | - |
| E.76 | 0 | W.4 | 1.6 | 2.4 | - |
| E.77 | 0 | W.4 | 1.7 | 2.4 | - |
| E.78 | 0 | W.4 | 1.8 | 2.4 | - |
| E.79 | 0 | W.4 | 1.9 | 2.4 | - |
| E.80 | 0 | W.4 | 1.10 | 2.4 | - |
| E.81 | 1 | W.1 | 1.1 | 2.1 | 3.1 |
| E.82 | 1 | W.1 | 1.2 | 2.1 | 3.1 |
| E.83 | 1 | W.1 | 1.3 | 2.1 | 3.1 |
| E.84 | 1 | W.1 | 1.4 | 2.1 | 3.1 |
| E.85 | 1 | W.1 | 1.5 | 2.1 | 3.1 |
| E.86 | 1 | W.1 | 1.6 | 2.1 | 3.1 |
| E.87 | 1 | W.1 | 1.7 | 2.1 | 3.1 |
| E.88 | 1 | W.1 | 1.8 | 2.1 | 3.1 |
| E.89 | 1 | W.1 | 1.9 | 2.1 | 3.1 |
| E.90 | 1 | W.1 | 1.10 | 2.1 | 3.1 |
| E.91 | 1 | W.1 | 1.1 | 2.4 | 3.1 |
| E.92 | 1 | W.1 | 1.2 | 2.4 | 3.1 |
| E.93 | 1 | W.1 | 1.3 | 2.4 | 3.1 |
| E.94 | 1 | W.1 | 1.4 | 2.4 | 3.1 |
| E.95 | 1 | W.1 | 1.5 | 2.4 | 3.1 |
| E.96 | 1 | W.1 | 1.6 | 2.4 | 3.1 |
| E.97 | 1 | W.1 | 1.7 | 2.4 | 3.1 |
| E.98 | 1 | W.1 | 1.8 | 2.4 | 3.1 |
| E.99 | 1 | W.1 | 1.9 | 2.4 | 3.1 |
| E.100 | 1 | W.1 | 1.10 | 2.4 | 3.1 |
| E.101 | 1 | W.2 | 1.1 | 2.1 | 3.1 |
| E.102 | 1 | W.2 | 1.2 | 2.1 | 3.1 |
| E.103 | 1 | W.2 | 1.3 | 2.1 | 3.1 |
| E.104 | 1 | W.2 | 1.4 | 2.1 | 3.1 |
| E.105 | 1 | W.2 | 1.5 | 2.1 | 3.1 |
| E.106 | 1 | W.2 | 1.6 | 2.1 | 3.1 |
| E.107 | 1 | W.2 | 1.7 | 2.1 | 3.1 |
| E.108 | 1 | W.2 | 1.8 | 2.1 | 3.1 |
| E.109 | 1 | W.2 | 1.9 | 2.1 | 3.1 |
| E.110 | 1 | W.2 | 1.10 | 2.1 | 3.1 |
| E.111 | 1 | W.2 | 1.1 | 2.4 | 3.1 |
| E.112 | 1 | W.2 | 1.2 | 2.4 | 3.1 |
| E.113 | 1 | W.2 | 1.3 | 2.4 | 3.1 |
| E.114 | 1 | W.2 | 1.4 | 2.4 | 3.1 |
| E.115 | 1 | W.2 | 1.5 | 2.4 | 3.1 |
| E.116 | 1 | W.2 | 1.6 | 2.4 | 3.1 |
| E.117 | 1 | W.2 | 1.7 | 2.4 | 3.1 |
| E.118 | 1 | W.2 | 1.8 | 2.4 | 3.1 |
| E.119 | 1 | W.2 | 1.9 | 2.4 | 3.1 |
| E.120 | 1 | W.2 | 1.10 | 2.4 | 3.1 |
| E.121 | 1 | W.3 | 1.1 | 2.1 | 3.1 |
| E.122 | 1 | W.3 | 1.2 | 2.1 | 3.1 |
| E.123 | 1 | W.3 | 1.3 | 2.1 | 3.1 |
| E.124 | 1 | W.3 | 1.4 | 2.1 | 3.1 |
| E.125 | 1 | W.3 | 1.5 | 2.1 | 3.1 |
| E.126 | 1 | W.3 | 1.6 | 2.1 | 3.1 |
| E.127 | 1 | W.3 | 1.7 | 2.1 | 3.1 |
| E.128 | 1 | W.3 | 1.8 | 2.1 | 3.1 |
| E.129 | 1 | W.3 | 1.9 | 2.1 | 3.1 |
| E.130 | 1 | W.3 | 1.10 | 2.1 | 3.1 |
| E.131 | 1 | W.3 | 1.1 | 2.4 | 3.1 |
| E.132 | 1 | W.3 | 1.2 | 2.4 | 3.1 |
| E.133 | 1 | W.3 | 1.3 | 2.4 | 3.1 |
| E.134 | 1 | W.3 | 1.4 | 2.4 | 3.1 |
| E.135 | 1 | W.3 | 1.5 | 2.4 | 3.1 |
| E.136 | 1 | W.3 | 1.6 | 2.4 | 3.1 |
| E.137 | 1 | W.3 | 1.7 | 2.4 | 3.1 |
| E.138 | 1 | W.3 | 1.8 | 2.4 | 3.1 |
| E.139 | 1 | W.3 | 1.9 | 2.4 | 3.1 |
| E.140 | 1 | W.3 | 1.10 | 2.4 | 3.1 |
| E.141 | 1 | W.4 | 1.1 | 2.1 | 3.1 |
| E.142 | 1 | W.4 | 1.2 | 2.1 | 3.1 |
| E.143 | 1 | W.4 | 1.3 | 2.1 | 3.1 |
| E.144 | 1 | W.4 | 1.4 | 2.1 | 3.1 |
| E.145 | 1 | W.4 | 1.5 | 2.1 | 3.1 |
| E.146 | 1 | W.4 | 1.6 | 2.1 | 3.1 |
| E.147 | 1 | W.4 | 1.7 | 2.1 | 3.1 |
| E.148 | 1 | W.4 | 1.8 | 2.1 | 3.1 |
| E.149 | 1 | W.4 | 1.9 | 2.1 | 3.1 |
| E.150 | 1 | W.4 | 1.10 | 2.1 | 3.1 |
| E.151 | 1 | W.4 | 1.1 | 2.4 | 3.1 |
| E.152 | 1 | W.4 | 1.2 | 2.4 | 3.1 |
| E.153 | 1 | W.4 | 1.3 | 2.4 | 3.1 |
| E.154 | 1 | W.4 | 1.4 | 2.4 | 3.1 |
| E.155 | 1 | W.4 | 1.5 | 2.4 | 3.1 |
| E.156 | 1 | W.4 | 1.6 | 2.4 | 3.1 |
| E.157 | 1 | W.4 | 1.7 | 2.4 | 3.1 |
| E.158 | 1 | W.4 | 1.8 | 2.4 | 3.1 |
| E.159 | 1 | W.4 | 1.9 | 2.4 | 3.1 |
| E.160 | 1 | W.4 | 1.10 | 2.4 | 3.1 |
| E.161 | 1 | W.1 | 1.1 | 2.1 | 3.4 |
| E.162 | 1 | W.1 | 1.2 | 2.1 | 3.4 |
| E.163 | 1 | W.1 | 1.3 | 2.1 | 3.4 |
| E.164 | 1 | W.1 | 1.4 | 2.1 | 3.4 |
| E.165 | 1 | W.1 | 1.5 | 2.1 | 3.4 |
| E.166 | 1 | W.1 | 1.6 | 2.1 | 3.4 |
| E.167 | 1 | W.1 | 1.7 | 2.1 | 3.4 |
| E.168 | 1 | W.1 | 1.8 | 2.1 | 3.4 |
| E.169 | 1 | W.1 | 1.9 | 2.1 | 3.4 |
| E.170 | 1 | W.1 | 1.10 | 2.1 | 3.4 |
| E.171 | 1 | W.1 | 1.1 | 2.4 | 3.4 |
| E.172 | 1 | W.1 | 1.2 | 2.4 | 3.4 |
| E.173 | 1 | W.1 | 1.3 | 2.4 | 3.4 |
| E.174 | 1 | W.1 | 1.4 | 2.4 | 3.4 |
| E.175 | 1 | W.1 | 1.5 | 2.4 | 3.4 |
| E.176 | 1 | W.1 | 1.6 | 2.4 | 3.4 |
| E.177 | 1 | W.1 | 1.7 | 2.4 | 3.4 |
| E.178 | 1 | W.1 | 1.8 | 2.4 | 3.4 |
| E.179 | 1 | W.1 | 1.9 | 2.4 | 3.4 |
| E.180 | 1 | W.1 | 1.10 | 2.4 | 3.4 |
| E.181 | 1 | W.2 | 1.1 | 2.1 | 3.4 |
| E.182 | 1 | W.2 | 1.2 | 2.1 | 3.4 |
| E.183 | 1 | W.2 | 1.3 | 2.1 | 3.4 |
| E.184 | 1 | W.2 | 1.4 | 2.1 | 3.4 |
| E.185 | 1 | W.2 | 1.5 | 2.1 | 3.4 |
| E.186 | 1 | W.2 | 1.6 | 2.1 | 3.4 |
| E.187 | 1 | W.2 | 1.7 | 2.1 | 3.4 |
| E.188 | 1 | W.2 | 1.8 | 2.1 | 3.4 |
| E.189 | 1 | W.2 | 1.9 | 2.1 | 3.4 |
| E.190 | 1 | W.2 | 1.10 | 2.1 | 3.4 |
| E.191 | 1 | W.2 | 1.1 | 2.4 | 3.4 |
| E.192 | 1 | W.2 | 1.2 | 2.4 | 3.4 |
| E.193 | 1 | W.2 | 1.3 | 2.4 | 3.4 |
| E.194 | 1 | W.2 | 1.4 | 2.4 | 3.4 |
| E.195 | 1 | W.2 | 1.5 | 2.4 | 3.4 |
| E.196 | 1 | W.2 | 1.6 | 2.4 | 3.4 |
| E.197 | 1 | W.2 | 1.7 | 2.4 | 3.4 |
| E.198 | 1 | W.2 | 1.8 | 2.4 | 3.4 |
| E.199 | 1 | W.2 | 1.9 | 2.4 | 3.4 |
| E.200 | 1 | W.2 | 1.10 | 2.4 | 3.4 |
| E.201 | 1 | W.3 | 1.1 | 2.1 | 3.4 |
| E.202 | 1 | W.3 | 1.2 | 2.1 | 3.4 |
| E.203 | 1 | W.3 | 1.3 | 2.1 | 3.4 |
| E.204 | 1 | W.3 | 1.4 | 2.1 | 3.4 |
| E.205 | 1 | W.3 | 1.5 | 2.1 | 3.4 |
| E.206 | 1 | W.3 | 1.6 | 2.1 | 3.4 |
| E.207 | 1 | W.3 | 1.7 | 2.1 | 3.4 |
| E.208 | 1 | W.3 | 1.8 | 2.1 | 3.4 |
| E.209 | 1 | W.3 | 1.9 | 2.1 | 3.4 |
| E.210 | 1 | W.3 | 1.10 | 2.1 | 3.4 |
| E.211 | 1 | W.3 | 1.1 | 2.4 | 3.4 |
| E.212 | 1 | W.3 | 1.2 | 2.4 | 3.4 |
| E.213 | 1 | W.3 | 1.3 | 2.4 | 3.4 |
| E.214 | 1 | W.3 | 1.4 | 2.4 | 3.4 |
| E.215 | 1 | W.3 | 1.5 | 2.4 | 3.4 |
| E.216 | 1 | W.3 | 1.6 | 2.4 | 3.4 |
| E.217 | 1 | W.3 | 1.7 | 2.4 | 3.4 |
| E.218 | 1 | W.3 | 1.8 | 2.4 | 3.4 |
| E.219 | 1 | W.3 | 1.9 | 2.4 | 3.4 |
| E.220 | 1 | W.3 | 1.10 | 2.4 | 3.4 |
| E.221 | 1 | W.4 | 1.1 | 2.1 | 3.4 |
| E.222 | 1 | W.4 | 1.2 | 2.1 | 3.4 |
| E.223 | 1 | W.4 | 1.3 | 2.1 | 3.4 |
| E.224 | 1 | W.4 | 1.4 | 2.1 | 3.4 |
| E.225 | 1 | W.4 | 1.5 | 2.1 | 3.4 |
| E.226 | 1 | W.4 | 1.6 | 2.1 | 3.4 |
| E.227 | 1 | W.4 | 1.7 | 2.1 | 3.4 |
| E.228 | 1 | W.4 | 1.8 | 2.1 | 3.4 |
| E.229 | 1 | W.4 | 1.9 | 2.1 | 3.4 |
| E.230 | 1 | W.4 | 1.10 | 2.1 | 3.4 |
| E.231 | 1 | W.4 | 1.1 | 2.4 | 3.4 |
| E.232 | 1 | W.4 | 1.2 | 2.4 | 3.4 |
| E.233 | 1 | W.4 | 1.3 | 2.4 | 3.4 |
| E.234 | 1 | W.4 | 1.4 | 2.4 | 3.4 |
| E.235 | 1 | W.4 | 1.5 | 2.4 | 3.4 |
| E.236 | 1 | W.4 | 1.6 | 2.4 | 3.4 |
| E.237 | 1 | W.4 | 1.7 | 2.4 | 3.4 |
| E.238 | 1 | W.4 | 1.8 | 2.4 | 3.4 |
| E.239 | 1 | W.4 | 1.9 | 2.4 | 3.4 |
| E.240 | 1 | W.4 | 1.10 | 2.4 | 3.4 |
| E.241 | 1 | W.1 | 1.1 | 2.1 | 3.5 |
| E.242 | 1 | W.1 | 1.2 | 2.1 | 3.5 |
| E.243 | 1 | W.1 | 1.3 | 2.1 | 3.5 |
| E.244 | 1 | W.1 | 1.4 | 2.1 | 3.5 |
| E.245 | 1 | W.1 | 1.5 | 2.1 | 3.5 |
| E.246 | 1 | W.1 | 1.6 | 2.1 | 3.5 |
| E.247 | 1 | W.1 | 1.7 | 2.1 | 3.5 |
| E.248 | 1 | W.1 | 1.8 | 2.1 | 3.5 |
| E.249 | 1 | W.1 | 1.9 | 2.1 | 3.5 |
| E.250 | 1 | W.1 | 1.10 | 2.1 | 3.5 |
| E.251 | 1 | W.1 | 1.1 | 2.4 | 3.5 |
| E.252 | 1 | W.1 | 1.2 | 2.4 | 3.5 |
| E.253 | 1 | W.1 | 1.3 | 2.4 | 3.5 |
| E.254 | 1 | W.1 | 1.4 | 2.4 | 3.5 |
| E.255 | 1 | W.1 | 1.5 | 2.4 | 3.5 |
| E.256 | 1 | W.1 | 1.6 | 2.4 | 3.5 |
| E.257 | 1 | W.1 | 1.7 | 2.4 | 3.5 |
| E.258 | 1 | W.1 | 1.8 | 2.4 | 3.5 |
| E.259 | 1 | W.1 | 1.9 | 2.4 | 3.5 |
| E.260 | 1 | W.1 | 1.10 | 2.4 | 3.5 |
| E.261 | 1 | W.2 | 1.1 | 2.1 | 3.5 |
| E.262 | 1 | W.2 | 1.2 | 2.1 | 3.5 |
| E.263 | 1 | W.2 | 1.3 | 2.1 | 3.5 |
| E.264 | 1 | W.2 | 1.4 | 2.1 | 3.5 |
| E.265 | 1 | W.2 | 1.5 | 2.1 | 3.5 |
| E.266 | 1 | W.2 | 1.6 | 2.1 | 3.5 |
| E.267 | 1 | W.2 | 1.7 | 2.1 | 3.5 |
| E.268 | 1 | W.2 | 1.8 | 2.1 | 3.5 |
| E.269 | 1 | W.2 | 1.9 | 2.1 | 3.5 |
| E.270 | 1 | W.2 | 1.10 | 2.1 | 3.5 |
| E.271 | 1 | W.2 | 1.1 | 2.4 | 3.5 |
| E.272 | 1 | W.2 | 1.2 | 2.4 | 3.5 |
| E.273 | 1 | W.2 | 1.3 | 2.4 | 3.5 |
| E.274 | 1 | W.2 | 1.4 | 2.4 | 3.5 |
| E.275 | 1 | W.2 | 1.5 | 2.4 | 3.5 |
| E.276 | 1 | W.2 | 1.6 | 2.4 | 3.5 |
| E.277 | 1 | W.2 | 1.7 | 2.4 | 3.5 |
| E.278 | 1 | W.2 | 1.8 | 2.4 | 3.5 |
| E.279 | 1 | W.2 | 1.9 | 2.4 | 3.5 |
| E.280 | 1 | W.2 | 1.10 | 2.4 | 3.5 |
| E.281 | 1 | W.3 | 1.1 | 2.1 | 3.5 |
| E.282 | 1 | W.3 | 1.2 | 2.1 | 3.5 |
| E.283 | 1 | W.3 | 1.3 | 2.1 | 3.5 |
| E.284 | 1 | W.3 | 1.4 | 2.1 | 3.5 |
| E.285 | 1 | W.3 | 1.5 | 2.1 | 3.5 |
| E.286 | 1 | W.3 | 1.6 | 2.1 | 3.5 |
| E.287 | 1 | W.3 | 1.7 | 2.1 | 3.5 |
| E.288 | 1 | W.3 | 1.8 | 2.1 | 3.5 |
| E.289 | 1 | W.3 | 1.9 | 2.1 | 3.5 |
| E.290 | 1 | W.3 | 1.10 | 2.1 | 3.5 |
| E.291 | 1 | W.3 | 1.1 | 2.4 | 3.5 |
| E.292 | 1 | W.3 | 1.2 | 2.4 | 3.5 |
| E.293 | 1 | W.3 | 1.3 | 2.4 | 3.5 |
| E.294 | 1 | W.3 | 1.4 | 2.4 | 3.5 |
| E.295 | 1 | W.3 | 1.5 | 2.4 | 3.5 |
| E.296 | 1 | W.3 | 1.6 | 2.4 | 3.5 |
| E.297 | 1 | W.3 | 1.7 | 2.4 | 3.5 |
| E.298 | 1 | W.3 | 1.8 | 2.4 | 3.5 |
| E.299 | 1 | W.3 | 1.9 | 2.4 | 3.5 |
| E.300 | 1 | W.3 | 1.10 | 2.4 | 3.5 |
| E.301 | 1 | W.4 | 1.1 | 2.1 | 3.5 |
| E.302 | 1 | W.4 | 1.2 | 2.1 | 3.5 |
| E.303 | 1 | W.4 | 1.3 | 2.1 | 3.5 |
| E.304 | 1 | W.4 | 1.4 | 2.1 | 3.5 |
| E.305 | 1 | W.4 | 1.5 | 2.1 | 3.5 |
| E.306 | 1 | W.4 | 1.6 | 2.1 | 3.5 |
| E.307 | 1 | W.4 | 1.7 | 2.1 | 3.5 |
| E.308 | 1 | W.4 | 1.8 | 2.1 | 3.5 |
| E.309 | 1 | W.4 | 1.9 | 2.1 | 3.5 |
| E.310 | 1 | W.4 | 1.10 | 2.1 | 3.5 |
| E.311 | 1 | W.4 | 1.1 | 2.4 | 3.5 |
| E.312 | 1 | W.4 | 1.2 | 2.4 | 3.5 |
| E.313 | 1 | W.4 | 1.3 | 2.4 | 3.5 |
| E.314 | 1 | W.4 | 1.4 | 2.4 | 3.5 |
| E.315 | 1 | W.4 | 1.5 | 2.4 | 3.5 |
| E.316 | 1 | W.4 | 1.6 | 2.4 | 3.5 |
| E.317 | 1 | W.4 | 1.7 | 2.4 | 3.5 |
| E.318 | 1 | W.4 | 1.8 | 2.4 | 3.5 |
| E.319 | 1 | W.4 | 1.9 | 2.4 | 3.5 |
| E.320 | 1 | W.4 | 1.10 | 2.4 | 3.5 |
| E.321 | 1 | W.1 | 1.1 | 2.1 | 3.6 |
| E.322 | 1 | W.1 | 1.2 | 2.1 | 3.6 |
| E.323 | 1 | W.1 | 1.3 | 2.1 | 3.6 |
| E.324 | 1 | W.1 | 1.4 | 2.1 | 3.6 |
| E.325 | 1 | W.1 | 1.5 | 2.1 | 3.6 |
| E.326 | 1 | W.1 | 1.6 | 2.1 | 3.6 |
| E.327 | 1 | W.1 | 1.7 | 2.1 | 3.6 |
| E.328 | 1 | W.1 | 1.8 | 2.1 | 3.6 |
| E.329 | 1 | W.1 | 1.9 | 2.1 | 3.6 |
| E.330 | 1 | W.1 | 1.10 | 2.1 | 3.6 |
| E.331 | 1 | W.1 | 1.1 | 2.4 | 3.6 |
| E.332 | 1 | W.1 | 1.2 | 2.4 | 3.6 |
| E.333 | 1 | W.1 | 1.3 | 2.4 | 3.6 |
| E.334 | 1 | W.1 | 1.4 | 2.4 | 3.6 |
| E.335 | 1 | W.1 | 1.5 | 2.4 | 3.6 |
| E.336 | 1 | W.1 | 1.6 | 2.4 | 3.6 |
| E.337 | 1 | W.1 | 1.7 | 2.4 | 3.6 |
| E.338 | 1 | W.1 | 1.8 | 2.4 | 3.6 |
| E.339 | 1 | W.1 | 1.9 | 2.4 | 3.6 |
| E.340 | 1 | W.1 | 1.10 | 2.4 | 3.6 |
| E.341 | 1 | W.2 | 1.1 | 2.1 | 3.6 |
| E.342 | 1 | W.2 | 1.2 | 2.1 | 3.6 |
| E.343 | 1 | W.2 | 1.3 | 2.1 | 3.6 |
| E.344 | 1 | W.2 | 1.4 | 2.1 | 3.6 |
| E.345 | 1 | W.2 | 1.5 | 2.1 | 3.6 |
| E.346 | 1 | W.2 | 1.6 | 2.1 | 3.6 |
| E.347 | 1 | W.2 | 1.7 | 2.1 | 3.6 |
| E.348 | 1 | W.2 | 1.8 | 2.1 | 3.6 |
| E.349 | 1 | W.2 | 1.9 | 2.1 | 3.6 |
| E.350 | 1 | W.2 | 1.10 | 2.1 | 3.6 |
| E.351 | 1 | W.2 | 1.1 | 2.4 | 3.6 |
| E.352 | 1 | W.2 | 1.2 | 2.4 | 3.6 |
| E.353 | 1 | W.2 | 1.3 | 2.4 | 3.6 |
| E.354 | 1 | W.2 | 1.4 | 2.4 | 3.6 |
| E.355 | 1 | W.2 | 1.5 | 2.4 | 3.6 |
| E.356 | 1 | W.2 | 1.6 | 2.4 | 3.6 |
| E.357 | 1 | W.2 | 1.7 | 2.4 | 3.6 |
| E.358 | 1 | W.2 | 1.8 | 2.4 | 3.6 |
| E.359 | 1 | W.2 | 1.9 | 2.4 | 3.6 |
| E.360 | 1 | W.2 | 1.10 | 2.4 | 3.6 |
| E.361 | 1 | W.3 | 1.1 | 2.1 | 3.6 |
| E.362 | 1 | W.3 | 1.2 | 2.1 | 3.6 |
| E.363 | 1 | W.3 | 1.3 | 2.1 | 3.6 |
| E.364 | 1 | W.3 | 1.4 | 2.1 | 3.6 |
| E.365 | 1 | W.3 | 1.5 | 2.1 | 3.6 |
| E.366 | 1 | W.3 | 1.6 | 2.1 | 3.6 |
| E.367 | 1 | W.3 | 1.7 | 2.1 | 3.6 |
| E.368 | 1 | W.3 | 1.8 | 2.1 | 3.6 |
| E.369 | 1 | W.3 | 1.9 | 2.1 | 3.6 |
| E.370 | 1 | W.3 | 1.10 | 2.1 | 3.6 |
| E.371 | 1 | W.3 | 1.1 | 2.4 | 3.6 |
| E.372 | 1 | W.3 | 1.2 | 2.4 | 3.6 |
| E.373 | 1 | W.3 | 1.3 | 2.4 | 3.6 |
| E.374 | 1 | W.3 | 1.4 | 2.4 | 3.6 |
| E.375 | 1 | W.3 | 1.5 | 2.4 | 3.6 |
| E.376 | 1 | W.3 | 1.6 | 2.4 | 3.6 |
| E.377 | 1 | W.3 | 1.7 | 2.4 | 3.6 |
| E.378 | 1 | W.3 | 1.8 | 2.4 | 3.6 |
| E.379 | 1 | W.3 | 1.9 | 2.4 | 3.6 |
| E.380 | 1 | W.3 | 1.10 | 2.4 | 3.6 |
| E.381 | 1 | W.4 | 1.1 | 2.1 | 3.6 |
| E.382 | 1 | W.4 | 1.2 | 2.1 | 3.6 |
| E.383 | 1 | W.4 | 1.3 | 2.1 | 3.6 |
| E.384 | 1 | W.4 | 1.4 | 2.1 | 3.6 |
| E.385 | 1 | W.4 | 1.5 | 2.1 | 3.6 |
| E.386 | 1 | W.4 | 1.6 | 2.1 | 3.6 |
| E.387 | 1 | W.4 | 1.7 | 2.1 | 3.6 |
| E.388 | 1 | W.4 | 1.8 | 2.1 | 3.6 |
| E.389 | 1 | W.4 | 1.9 | 2.1 | 3.6 |
| E.390 | 1 | W.4 | 1.10 | 2.1 | 3.6 |
| E.391 | 1 | W.4 | 1.1 | 2.4 | 3.6 |
| E.392 | 1 | W.4 | 1.2 | 2.4 | 3.6 |
| E.393 | 1 | W.4 | 1.3 | 2.4 | 3.6 |
| E.394 | 1 | W.4 | 1.4 | 2.4 | 3.6 |
| E.395 | 1 | W.4 | 1.5 | 2.4 | 3.6 |
| E.396 | 1 | W.4 | 1.6 | 2.4 | 3.6 |
| E.397 | 1 | W.4 | 1.7 | 2.4 | 3.6 |
| E.398 | 1 | W.4 | 1.8 | 2.4 | 3.6 |
| E.399 | 1 | W.4 | 1.9 | 2.4 | 3.6 |
| E.400 | 1 | W.4 | 1.10 | 2.4 | 3.6 |
| E.401 | 1 | W.1 | 1.1 | 2.1 | 3.8 |
| E.402 | 1 | W.1 | 1.2 | 2.1 | 3.8 |
| E.403 | 1 | W.1 | 1.3 | 2.1 | 3.8 |
| E.404 | 1 | W.1 | 1.4 | 2.1 | 3.8 |
| E.405 | 1 | W.1 | 1.5 | 2.1 | 3.8 |
| E.406 | 1 | W.1 | 1.6 | 2.1 | 3.8 |
| E.407 | 1 | W.1 | 1.7 | 2.1 | 3.8 |
| E.408 | 1 | W.1 | 1.8 | 2.1 | 3.8 |
| E.409 | 1 | W.1 | 1.9 | 2.1 | 3.8 |
| E.410 | 1 | W.1 | 1.10 | 2.1 | 3.8 |
| E.411 | 1 | W.1 | 1.1 | 2.4 | 3.8 |
| E.412 | 1 | W.1 | 1.2 | 2.4 | 3.8 |
| E.413 | 1 | W.1 | 1.3 | 2.4 | 3.8 |
| E.414 | 1 | W.1 | 1.4 | 2.4 | 3.8 |
| E.415 | 1 | W.1 | 1.5 | 2.4 | 3.8 |
| E.416 | 1 | W.1 | 1.6 | 2.4 | 3.8 |
| E.417 | 1 | W.1 | 1.7 | 2.4 | 3.8 |
| E.418 | 1 | W.1 | 1.8 | 2.4 | 3.8 |
| E.419 | 1 | W.1 | 1.9 | 2.4 | 3.8 |
| E.420 | 1 | W.1 | 1.10 | 2.4 | 3.8 |
| E.421 | 1 | W.2 | 1.1 | 2.1 | 3.8 |
| E.422 | 1 | W.2 | 1.2 | 2.1 | 3.8 |
| E.423 | 1 | W.2 | 1.3 | 2.1 | 3.8 |
| E.424 | 1 | W.2 | 1.4 | 2.1 | 3.8 |
| E.425 | 1 | W.2 | 1.5 | 2.1 | 3.8 |
| E.426 | 1 | W.2 | 1.6 | 2.1 | 3.8 |
| E.427 | 1 | W.2 | 1.7 | 2.1 | 3.8 |
| E.428 | 1 | W.2 | 1.8 | 2.1 | 3.8 |
| E.429 | 1 | W.2 | 1.9 | 2.1 | 3.8 |
| E.430 | 1 | W.2 | 1.10 | 2.1 | 3.8 |
| E.431 | 1 | W.2 | 1.1 | 2.4 | 3.8 |
| E.432 | 1 | W.2 | 1.2 | 2.4 | 3.8 |
| E.433 | 1 | W.2 | 1.3 | 2.4 | 3.8 |
| E.434 | 1 | W.2 | 1.4 | 2.4 | 3.8 |
| E.435 | 1 | W.2 | 1.5 | 2.4 | 3.8 |
| E.436 | 1 | W.2 | 1.6 | 2.4 | 3.8 |
| E.437 | 1 | W.2 | 1.7 | 2.4 | 3.8 |
| E.438 | 1 | W.2 | 1.8 | 2.4 | 3.8 |
| E.439 | 1 | W.2 | 1.9 | 2.4 | 3.8 |
| E.440 | 1 | W.2 | 1.10 | 2.4 | 3.8 |
| E.441 | 1 | W.3 | 1.1 | 2.1 | 3.8 |
| E.442 | 1 | W.3 | 1.2 | 2.1 | 3.8 |
| E.443 | 1 | W.3 | 1.3 | 2.1 | 3.8 |
| E.444 | 1 | W.3 | 1.4 | 2.1 | 3.8 |
| E.445 | 1 | W.3 | 1.5 | 2.1 | 3.8 |
| E.446 | 1 | W.3 | 1.6 | 2.1 | 3.8 |
| E.447 | 1 | W.3 | 1.7 | 2.1 | 3.8 |
| E.448 | 1 | W.3 | 1.8 | 2.1 | 3.8 |
| E.449 | 1 | W.3 | 1.9 | 2.1 | 3.8 |
| E.450 | 1 | W.3 | 1.10 | 2.1 | 3.8 |
| E.451 | 1 | W.3 | 1.1 | 2.4 | 3.8 |
| E.452 | 1 | W.3 | 1.2 | 2.4 | 3.8 |
| E.453 | 1 | W.3 | 1.3 | 2.4 | 3.8 |
| E.454 | 1 | W.3 | 1.4 | 2.4 | 3.8 |
| E.455 | 1 | W.3 | 1.5 | 2.4 | 3.8 |
| E.456 | 1 | W.3 | 1.6 | 2.4 | 3.8 |
| E.457 | 1 | W.3 | 1.7 | 2.4 | 3.8 |
| E.458 | 1 | W.3 | 1.8 | 2.4 | 3.8 |
| E.459 | 1 | W.3 | 1.9 | 2.4 | 3.8 |
| E.460 | 1 | W.3 | 1.10 | 2.4 | 3.8 |
| E.461 | 1 | W.4 | 1.1 | 2.1 | 3.8 |
| E.462 | 1 | W.4 | 1.2 | 2.1 | 3.8 |
| E.463 | 1 | W.4 | 1.3 | 2.1 | 3.8 |
| E.464 | 1 | W.4 | 1.4 | 2.1 | 3.8 |
| E.465 | 1 | W.4 | 1.5 | 2.1 | 3.8 |
| E.466 | 1 | W.4 | 1.6 | 2.1 | 3.8 |
| E.467 | 1 | W.4 | 1.7 | 2.1 | 3.8 |
| E.468 | 1 | W.4 | 1.8 | 2.1 | 3.8 |
| E.469 | 1 | W.4 | 1.9 | 2.1 | 3.8 |
| E.470 | 1 | W.4 | 1.10 | 2.1 | 3.8 |
| E.471 | 1 | W.4 | 1.1 | 2.4 | 3.8 |
| E.472 | 1 | W.4 | 1.2 | 2.4 | 3.8 |
| E.473 | 1 | W.4 | 1.3 | 2.4 | 3.8 |
| E.474 | 1 | W.4 | 1.4 | 2.4 | 3.8 |
| E.475 | 1 | W.4 | 1.5 | 2.4 | 3.8 |
| E.476 | 1 | W.4 | 1.6 | 2.4 | 3.8 |
| E.477 | 1 | W.4 | 1.7 | 2.4 | 3.8 |
| E.478 | 1 | W.4 | 1.8 | 2.4 | 3.8 |
| E.479 | 1 | W.4 | 1.9 | 2.4 | 3.8 |
| E.480 | 1 | W.4 | 1.10 | 2.4 | 3.8 |
| E.481 | 1 | W.1 | 1.1 | 2.1 | 3.12 |
| E.482 | 1 | W.1 | 1.2 | 2.1 | 3.12 |
| E.483 | 1 | W.1 | 1.3 | 2.1 | 3.12 |
| E.484 | 1 | W.1 | 1.4 | 2.1 | 3.12 |
| E.485 | 1 | W.1 | 1.5 | 2.1 | 3.12 |
| E.486 | 1 | W.1 | 1.6 | 2.1 | 3.12 |
| E.487 | 1 | W.1 | 1.7 | 2.1 | 3.12 |
| E.488 | 1 | W.1 | 1.8 | 2.1 | 3.12 |
| E.489 | 1 | W.1 | 1.9 | 2.1 | 3.12 |
| E.490 | 1 | W.1 | 1.10 | 2.1 | 3.12 |
| E.491 | 1 | W.1 | 1.1 | 2.4 | 3.12 |
| E.492 | 1 | W.1 | 1.2 | 2.4 | 3.12 |
| E.493 | 1 | W.1 | 1.3 | 2.4 | 3.12 |
| E.494 | 1 | W.1 | 1.4 | 2.4 | 3.12 |
| E.495 | 1 | W.1 | 1.5 | 2.4 | 3.12 |
| E.496 | 1 | W.1 | 1.6 | 2.4 | 3.12 |
| E.497 | 1 | W.1 | 1.7 | 2.4 | 3.12 |
| E.498 | 1 | W.1 | 1.8 | 2.4 | 3.12 |
| E.499 | 1 | W.1 | 1.9 | 2.4 | 3.12 |
| E.500 | 1 | W.1 | 1.10 | 2.4 | 3.12 |
| E.501 | 1 | W.2 | 1.1 | 2.1 | 3.12 |
| E.502 | 1 | W.2 | 1.2 | 2.1 | 3.12 |
| E.503 | 1 | W.2 | 1.3 | 2.1 | 3.12 |
| E.504 | 1 | W.2 | 1.4 | 2.1 | 3.12 |
| E.505 | 1 | W.2 | 1.5 | 2.1 | 3.12 |
| E.506 | 1 | W.2 | 1.6 | 2.1 | 3.12 |
| E.507 | 1 | W.2 | 1.7 | 2.1 | 3.12 |
| E.508 | 1 | W.2 | 1.8 | 2.1 | 3.12 |
| E.509 | 1 | W.2 | 1.9 | 2.1 | 3.12 |
| E.510 | 1 | W.2 | 1.10 | 2.1 | 3.12 |
| E.511 | 1 | W.2 | 1.1 | 2.4 | 3.12 |
| E.512 | 1 | W.2 | 1.2 | 2.4 | 3.12 |
| E.513 | 1 | W.2 | 1.3 | 2.4 | 3.12 |
| E.514 | 1 | W.2 | 1.4 | 2.4 | 3.12 |
| E.515 | 1 | W.2 | 1.5 | 2.4 | 3.12 |
| E.516 | 1 | W.2 | 1.6 | 2.4 | 3.12 |
| E.517 | 1 | W.2 | 1.7 | 2.4 | 3.12 |
| E.518 | 1 | W.2 | 1.8 | 2.4 | 3.12 |
| E.519 | 1 | W.2 | 1.9 | 2.4 | 3.12 |
| E.520 | 1 | W.2 | 1.10 | 2.4 | 3.12 |
| E.521 | 1 | W.3 | 1.1 | 2.1 | 3.12 |
| E.522 | 1 | W.3 | 1.2 | 2.1 | 3.12 |
| E.523 | 1 | W.3 | 1.3 | 2.1 | 3.12 |
| E.524 | 1 | W.3 | 1.4 | 2.1 | 3.12 |
| E.525 | 1 | W.3 | 1.5 | 2.1 | 3.12 |
| E.526 | 1 | W.3 | 1.6 | 2.1 | 3.12 |
| E.527 | 1 | W.3 | 1.7 | 2.1 | 3.12 |
| E.528 | 1 | W.3 | 1.8 | 2.1 | 3.12 |
| E.529 | 1 | W.3 | 1.9 | 2.1 | 3.12 |
| E.530 | 1 | W.3 | 1.10 | 2.1 | 3.12 |
| E.531 | 1 | W.3 | 1.1 | 2.4 | 3.12 |
| E.532 | 1 | W.3 | 1.2 | 2.4 | 3.12 |
| E.533 | 1 | W.3 | 1.3 | 2.4 | 3.12 |
| E.534 | 1 | W.3 | 1.4 | 2.4 | 3.12 |
| E.535 | 1 | W.3 | 1.5 | 2.4 | 3.12 |
| E.536 | 1 | W.3 | 1.6 | 2.4 | 3.12 |
| E.537 | 1 | W.3 | 1.7 | 2.4 | 3.12 |
| E.538 | 1 | W.3 | 1.8 | 2.4 | 3.12 |
| E.539 | 1 | W.3 | 1.9 | 2.4 | 3.12 |
| E.540 | 1 | W.3 | 1.10 | 2.4 | 3.12 |
| E.541 | 1 | W.4 | 1.1 | 2.1 | 3.12 |
| E.542 | 1 | W.4 | 1.2 | 2.1 | 3.12 |
| E.543 | 1 | W.4 | 1.3 | 2.1 | 3.12 |
| E.544 | 1 | W.4 | 1.4 | 2.1 | 3.12 |
| E.545 | 1 | W.4 | 1.5 | 2.1 | 3.12 |
| E.546 | 1 | W.4 | 1.6 | 2.1 | 3.12 |
| E.547 | 1 | W.4 | 1.7 | 2.1 | 3.12 |
| E.548 | 1 | W.4 | 1.8 | 2.1 | 3.12 |
| E.549 | 1 | W.4 | 1.9 | 2.1 | 3.12 |
| E.550 | 1 | W.4 | 1.10 | 2.1 | 3.12 |
| E.551 | 1 | W.4 | 1.1 | 2.4 | 3.12 |
| E.552 | 1 | W.4 | 1.2 | 2.4 | 3.12 |
| E.553 | 1 | W.4 | 1.3 | 2.4 | 3.12 |
| E.554 | 1 | W.4 | 1.4 | 2.4 | 3.12 |
| E.555 | 1 | W.4 | 1.5 | 2.4 | 3.12 |
| E.556 | 1 | W.4 | 1.6 | 2.4 | 3.12 |
| E.557 | 1 | W.4 | 1.7 | 2.4 | 3.12 |
| E.558 | 1 | W.4 | 1.8 | 2.4 | 3.12 |
| E.559 | 1 | W.4 | 1.9 | 2.4 | 3.12 |
| E.560 | 1 | W.4 | 1.10 | 2.4 | 3.12 |
| E.561 | 1 | W.1 | 1.1 | 2.1 | 3.14 |
| E.562 | 1 | W.1 | 1.2 | 2.1 | 3.14 |
| E.563 | 1 | W.1 | 1.3 | 2.1 | 3.14 |
| E.564 | 1 | W.1 | 1.4 | 2.1 | 3.14 |
| E.565 | 1 | W.1 | 1.5 | 2.1 | 3.14 |
| E.566 | 1 | W.1 | 1.6 | 2.1 | 3.14 |
| E.567 | 1 | W.1 | 1.7 | 2.1 | 3.14 |
| E.568 | 1 | W.1 | 1.8 | 2.1 | 3.14 |
| E.569 | 1 | W.1 | 1.9 | 2.1 | 3.14 |
| E.570 | 1 | W.1 | 1.10 | 2.1 | 3.14 |
| E.571 | 1 | W.1 | 1.1 | 2.4 | 3.14 |
| E.572 | 1 | W.1 | 1.2 | 2.4 | 3.14 |
| E.573 | 1 | W.1 | 1.3 | 2.4 | 3.14 |
| E.574 | 1 | W.1 | 1.4 | 2.4 | 3.14 |
| E.575 | 1 | W.1 | 1.5 | 2.4 | 3.14 |
| E.576 | 1 | W.1 | 1.6 | 2.4 | 3.14 |
| E.577 | 1 | W.1 | 1.7 | 2.4 | 3.14 |
| E.578 | 1 | W.1 | 1.8 | 2.4 | 3.14 |
| E.579 | 1 | W.1 | 1.9 | 2.4 | 3.14 |
| E.580 | 1 | W.1 | 1.10 | 2.4 | 3.14 |

In one embodiment the invention relates to the use of compounds of the formula I.1 or of compounds of the formula 1.2, or the N-oxides, or the agriculturally acceptable salts thereof, 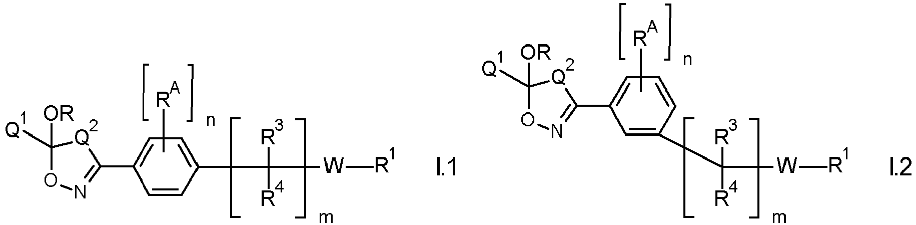 wherein n is 0, 1 or 2; and wherein the meaning of the variables W, p, R², R³, R⁴, m and R¹ are as defined herein for compounds of the formula I or as preferably defined in embodiments E.1 to E.580 in Table E; and wherein Q¹, Q², R and R^{A} are as defined or preferably defined herein for compounds of the formula I.

A preferred embodiment relates to compounds of the formula I.1 or to compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CF₃, Q² is -CF₂-, R is as defined in Embodiment R.1 above, n is 0 or 1 and R^{A} is halogen, C₁-C₆-alkyl or C₃-C₈-cycloalkyl.

A preferred embodiment relates to compounds of the formula I.1 or to compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CF₃, Q² is -CF₂-, R is hydrogen, n is 0 or 1 and R^{A} is halogen, C₁-C₆-alkyl or C₃-C₈-cycloalkyl.

Another preferred embodiment relates to compounds of the formula I.1 or to compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CHF₂, Q² is -CF₂-, R is hydrogen, n is 0 or 1 and R^{A} is halogen, C₁-C₆-alkyl or C₃-C₈-cycloalkyl.

In one preferred embodiment relates to the use of compounds of the formula I.1 or of compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CF₃, Q² is -CH₂-, R is as defined in Embodiment R.1 above, n is 0 or 1 and R^{A} is halogen, C₁-C₆-alkyl or C₃-C₈-cycloalkyl.

In one preferred embodiment relates to compounds of the formula I.1 or to compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CF₃, Q² is -CH₂-, R is hydrogen, n is 0 or 1 and R^{A} is halogen, C₁-C₆-alkyl or C₃-C₈-cycloalkyl.

Another preferred embodiment relates to the use of compounds of the formula I.1 or of compounds of the formula I.2 as defined above, wherein the meaning of the variables W, p, R², R³, R⁴, m and R¹ are as defined herein for compounds of the formula I or as preferably defined in embodiments E.1 to E.580 in Table E; and wherein Q¹ is CHF₂, Q² is -CH₂-, R is hydrogen and R^{A} is halogen, C₁-C₆-alkyl or C₃-C₈-cycloalkyl.

In a further embodiment the invention relates to the use of the group of compounds I.1a of formula I.1, or the N-oxides, or the agriculturally acceptable salts thereof, wherein:
- R^{A}: is halogen, C₁-C₆-alkyl or C₃-C₈-cycloalkyl;
- n: is 0 or 1;
- Q¹: is CF₃;
- Q²: is -CH₂-;
- R: is hydrogen, C₁-C₄-alkyl, C₃-C₈-cycloalkyl -Si(C₁-C₄-alkyl) or -(C=O)-R^{X}, wherein R^{X} is (C=O)-C₁-C₄-alkyl, C(=O)-C₁-C₄-alkoxy -N(R^{xa})₂, C₁-C₆-alkyl-C(=O)-O-CH₂-, C₃-C₆-cycloalkyl-C(=O)-O-CH₂-, a 5- or 6-membered saturated, partially unsaturated or aromatic mono- or bicyclic heterocycle, wherein the ring member atoms of said mono- or bicyclic heterocycle include besides carbon atoms further 1 or 2 heteroatoms selected from N, O and S as ring member atoms with the provision that the heterocycle cannot contain 2 contiguous atoms selected from O and S; and wherein R^{xa} is independently selected from hydrogen and C₁-C₄-alkyl;
- W: is -(C=O)-NR²-#, -(C=S)-NR²-#, -S(=O)₂-NR²-#, -NR²-(C=O)-#, -NR²-(C=S)-# or -NR²-S(=O)₂-#, wherein # denotes the position which is attached to the group R¹;
- R¹: is C₁-C₆-alkyl, C₂-C₆-alkenyl, C₂-C₆-alkynyl, C₃-C₈-cycloalkyl, C₃-C₈-cycloalkenyl, phenyl or pyridinyl; and wherein the aliphatic and cyclic groups are unsubstituted or substituted with 1, 2, 3 or up to the maximum possible number of identical or different radials selected from the group consisting of halogen, C₁-C₆-alkyl and C₃-C₈-cycloalkyl;
- R²: is hydrogen, C₁-C₆-alkyl, C₁-C₆-alkoxy, C₂-C₆-alkenyl or propargyl, C₁-C₆-alkylamino or diC₁-C₆-alkylamino;
- m: is 0 or 1;
- R³ and R⁴: are independently selected from the group consisting of hydrogen, fluorine, chlorine, methyl or trifluoromethyl.

In a further embodiment the invention relates to the use of the group of compounds I.1a, wherein n is 0; m is 0; W is -(C=O)-NR²-#, -(C=S)-NR²-# or -S(=O)ₚ-NR²-#, wherein # denotes the position which is attached to the group R¹.

In a further embodiment the invention relates to the use of the group of compounds 1.1a, wherein n is 0; m is 1; W is -(C=O)-NR²-#, -(C=S)-NR²-# or -S(=O)ₚ-NR²-#, wherein # denotes the position which is attached to the group R¹; and wherein R³ and R⁴ are fluorine; or R³ and R⁴ together with the carbon atom to which they are bound form a cyclopropyl ring.

In a further embodiment the invention relates to the use of the group of compounds 1.1a, wherein n is 0; m is 1; W is -NR²-(C=O)-#, -NR²-(C=S)-# or -NR²-S(=O)ₚ-#, wherein # denotes the position which is attached to the group R¹; and wherein R³ and R⁴ are hydrogen.

In a further embodiment the invention relates to the use of the group of compounds I.1a, wherein n is 0; m is 1; W is -NR²-(C=O)-#, -NR²-(C=S)-# or -NR²-S(=O)ₚ-#, wherein # denotes the position which is attached to the group R¹; and wherein R³ is hydrogen and R⁴ is trifluoromethyl.

In a further embodiment the invention relates to the use of the group of compounds I.1a of formula I.1, or the N-oxides, or the agriculturally acceptable salts thereof, wherein:
- R^{A}: is halogen, C₁-C₆-alkyl or C₃-C₈-cycloalkyl;
- n: is 0 or 1;
- Q¹: is CHF₂ or CF₃;
- Q²: is -CH₂-;
- R: is hydrogen, methyl, ethyl or trimethylsilyl;
- W: is -(C=O)-NR²-#, -(C=S)-NR²-#, -S(=O)₂-NR²-#, -NR²-(C=O)-#, -NR²-(C=S)-# or -NR²-S(=O)₂-#, wherein # denotes the position which is attached to the group R¹;
- R¹: is C₁-C₆-alkyl, C₂-C₆-alkenyl, C₂-C₆-alkynyl, C₃-C₈-cycloalkyl, C₃-C₈-cycloalkenyl, phenyl or pyridinyl; and wherein the aliphatic and cyclic groups are unsubstituted or substituted with 1, 2, 3 or up to the maximum possible number of identical or different radials selected from the group consisting of halogen, C₁-C₆-alkyl and C₃-C₈-cycloalkyl;
- R²: is hydrogen, C₁-C₆-alkyl, C₁-C₆-alkoxy, C₂-C₆-alkenyl or propargyl, C₁-C₆-alkylamino or diC₁-C₆-alkylamino;
- m: is 0 or 1;
- R³ and R⁴: are independently selected from the group consisting of hydrogen, fluorine, chlorine, methyl or trifluoromethyl;
- or R³ and R⁴: together with the carbon atom to which they are bound form a cyclopropyl ring.

In a further embodiment the invention relates to the use of the group of compounds I.1a, wherein n is 0; m is 1; W is -(C=O)-NR²-#, -(C=S)-NR²-# or -S(=O)ₚ-NR²-#, wherein # denotes the position which is attached to the group R¹; and wherein R³ and R⁴ are fluorine; or R³ and R⁴ together with the carbon atom to which they are bound form a cyclopropyl ring.

In a further embodiment the invention relates to the use of the group of compounds I.1a, wherein n is 0; m is 1; W is -NR²-(C=O)-#, -NR²-(C=S)-# or -NR²-S(=O)ₚ-#, wherein # denotes the position which is attached to the group R¹; and wherein R³ and R⁴ are hydrogen; or R³ and R⁴ together with the carbon atom to which they are bound form a cyclopropyl ring.

In one embodiment, the present invention relates to the use of compounds of the formulae I.A, I.B, I.C, I.D, I.E, I.F, I.G, I.H, I.J, I.K, I.L, I.M, I.N, I.O, I.P, I.Q, I.R, I.S, I.T, I.U, I.V, I.W, I.X, I.Y, I.B2, I.H2 and I.M2 and to their use for controlling phytopathogenic fungi, wherein the variables R¹ and R² are as defined or preferably defined herein.

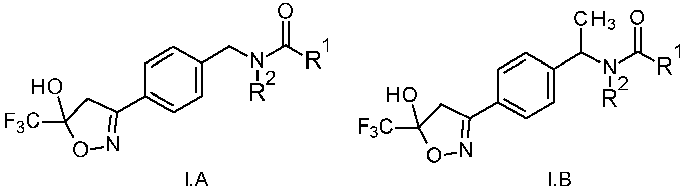

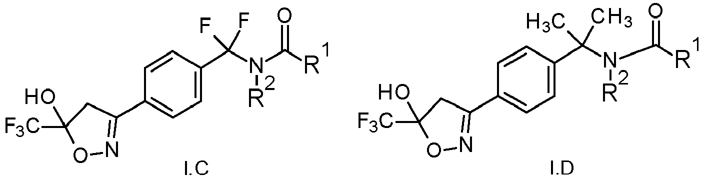

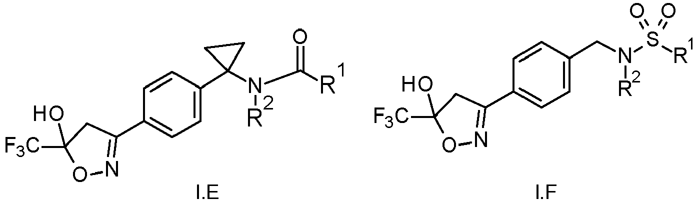

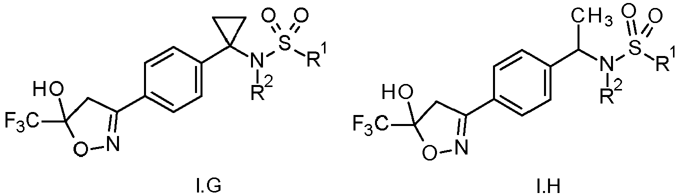

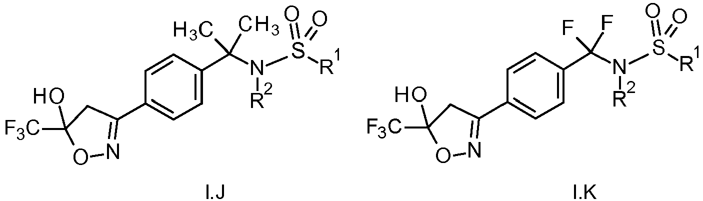

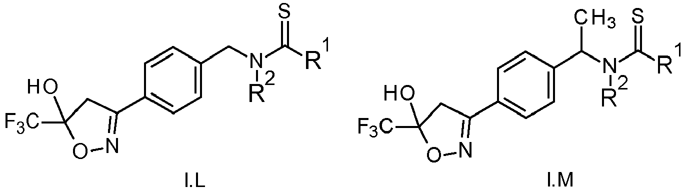

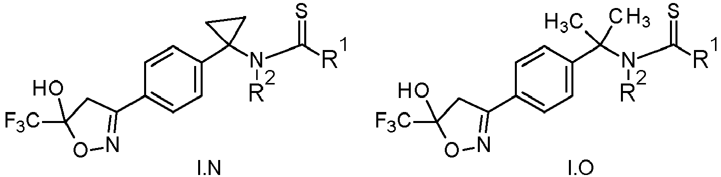

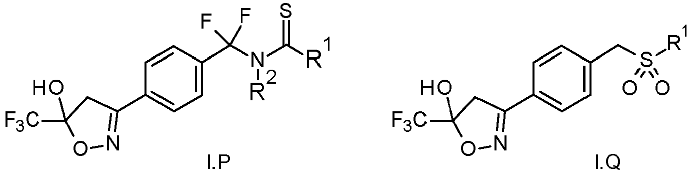

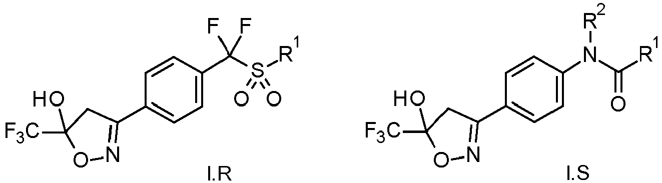

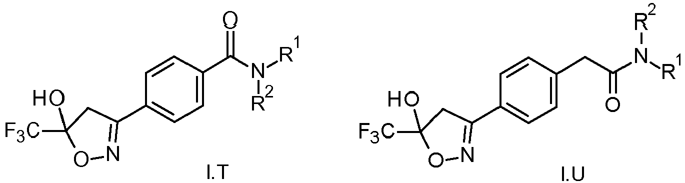

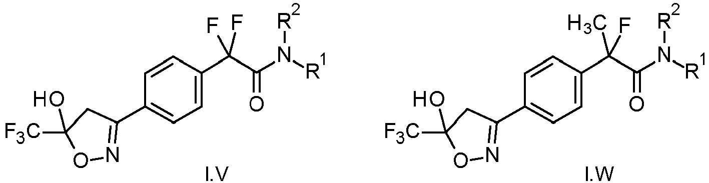

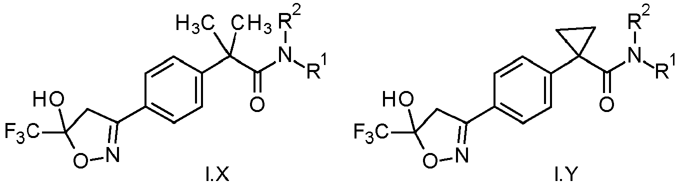

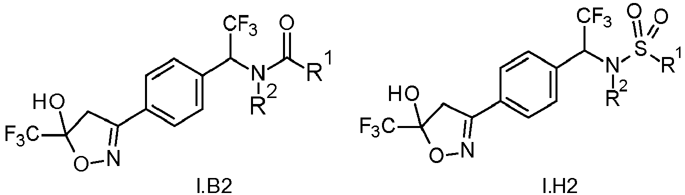

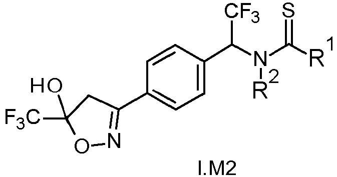

Preference is given to the use of compounds of the formula I, which are compiled in Tables 1 to 24 below, and which may be used according to the invention.
Table 1: Compounds of the formula I.A, in which R¹ and R² for each individual compound corresponds in each case to one line A-1 to A-1700 of Table A1 (compounds I.A.A-1 to I.A.A-1700). This means, for example, that a compound of formula I.A, wherein R¹ is *iso*-propyl and R² is hydrogen (corresponding to the definition A-4 in Table A1) is named I.A.A-4.
Table 2: Compounds of the formula I.B, in which R¹ and R² for each individual compound corresponds in each case to one line A-1 to A-1700 of Table A1 (compounds I.B.A-1 to I.B.A-1700). This means, for example, that a compound of formula I.B, wherein R¹ is *iso*-propyl and R² is ethyl (corresponding to the definition A-684 in Table A1) is named I.B.A-684.
Table 3: Compounds of the formula I.C, in which R¹ and R² for each individual compound corresponds in each case to one line A-1 to A-1700 of Table A1 (compounds I.C.A-1 to I.C.A-1700)
Table 4: Compounds of the formula I.D, in which R¹ and R² for each individual compound corresponds in each case to one line A-1 to A-1700 of Table A1 (compounds I.D.A-1 to I.D.A-1700).
Table 5: Compounds of the formula I.E, in which R¹ and R² for each individual compound corresponds in each case to one line A-1 to A-1700 of Table A1 (compounds I.E.A-1 to I.E.A-1700).
Table 6: Compounds of the formula I.F, in which R¹ and R² for each individual compound corresponds in each case to one line A-1 to A-1700 of Table A1 (compounds I.F.A-1 to I.F.A-1700).
Table 7: Compounds of the formula I.G, in which R¹ and R² for each individual compound corresponds in each case toone line A-1 to A-1700 of Table A1 (compounds I.G.A-1 to I.G.A-1700).
Table 8: Compounds of the formula I.H, in which R¹ and R² for each individual compound corresponds in each case to one line A-1 to A-1700 of Table A1 (compounds I.H.A-1 to I.H.A-1700)
Table 9: Compounds of the formula I.J, in which R¹ and R² for each individual compound corresponds in each case to one line A-1 to A-1700 of Table A1 (compounds I.J.A-1 to I.J.A-1700).
Table 10: Compounds of the formula I.K, in which R¹ and R² for each individual compound corresponds in each case to one line A-1 to A-1700 of Table A1 (compounds I.K.A-1 to I.K.A-1700).
Table 11: Compounds of the formula I.L, in which R¹ and R² for each individual compound corresponds in each case to one line A-1 to A-1700 of Table A1 (compounds I.L.A-1 to I.L.A-1700).
Table 12: Compounds of the formula I.M, in which R¹ and R² for each individual compound corresponds in each case to one line A-1 to A-1700 of Table A1 (compounds I.M.A-1 to I.M.A-1700).
Table 13: Compounds of the formula I.N, in which R¹ and R² for each individual compound corresponds in each case to one line A-1 to A-1700 of Table A1 (compounds I.N.A-1 to I.N.A-1700).
Table 14: Compounds of the formula I.O, in which R¹ and R² for each individual compound corresponds in each case to one line A-1 to A-1700 of Table A1 (compounds I.O.A-1 to I.O.A-1700).
Table 15: Compounds of the formula I.P, in which R¹ and R² for each individual compound corresponds in each case to one line A-1 to A-1700 of Table A1 (compounds I.P.A-1 to I.P.A-1700).
Table 16: Compounds of the formula I.Q, in which R¹ for each individual compound corresponds in each case to one line A-1 to A-1700 of Table A1 (compounds I.Q.A-1 to I.Q.A-1700).
Table 17: Compounds of the formula I.R, in which R¹ for each individual compound corresponds in each case to one line A-1 to A-1700 of Table A1 (compounds I.R.A-1 to I.R.A-1700).
Table 18: Compounds of the formula I.S, in which R¹ and R² for each individual compound corresponds in each case to one line A-1 to A-1700 of Table A1 (compounds I.S.A-1 to I.S.A-1700).
Table 19: Compounds of the formula I.T, in which R¹ and R² for each individual compound corresponds in each case to one line A-1 to A-1700 of Table A1 (compounds I.T.A-1 to I.T.A-1700).
Table 20: Compounds of the formula I.U, in which R¹ and R² for each individual compound corresponds in each case to one line A-1 to A-1700 of Table A1 (compounds I.U.A-1 to I.U.A-1700).
Table 21: Compounds of the formula I.V, in which R¹ and R² for each individual compound corresponds in each case to one line A-1 to A-1700 of Table A1 (compounds I.V.A-1 to I.V.A-1700).
Table 22: Compounds of the formula I.W, in which R¹ and R² for each individual compound corresponds in each case to one line A-1 to A-1700 of Table A1 (compounds I.W.A-1 to I.W.A-1700).
Table 23: Compounds of the formula I.X, in which R¹ and R² for each individual compound corresponds in each case to one line A-1 to A-1700 of Table A1 (compounds I.X.A-1 to I.X.A-1700).
Table 24: Compounds of the formula I.Y, in which R¹ and R² for each individual compound corresponds in each case to one line A-1 to A-1700 of Table A1 (compounds I.Y.A-1 to I.Y.A-1700).
Table 25: Compounds of the formula I.B2, in which R¹ and R² for each individual compound corresponds in each case to one line A-1 to A-1700 of Table A1 (compounds I.B2.A-1 to I.B2.A-1700).
Table 26: Compounds of the formula I.H2, in which R¹ and R² for each individual compound corresponds in each case to one line A-1 to A-1700 of Table A1 (compounds I.H2.A-1 to I.H2.A-1700).
Table 27: Compounds of the formula I.M2, in which R¹ and R² for each individual compound corresponds in each case to one line A-1 to A-1700 of Table A1 (compounds I.M2.A-1 to I.M2.A-1700).

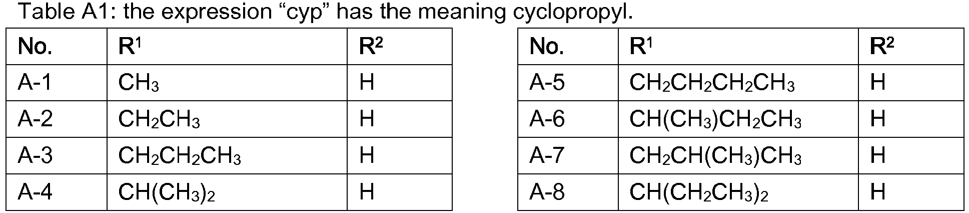

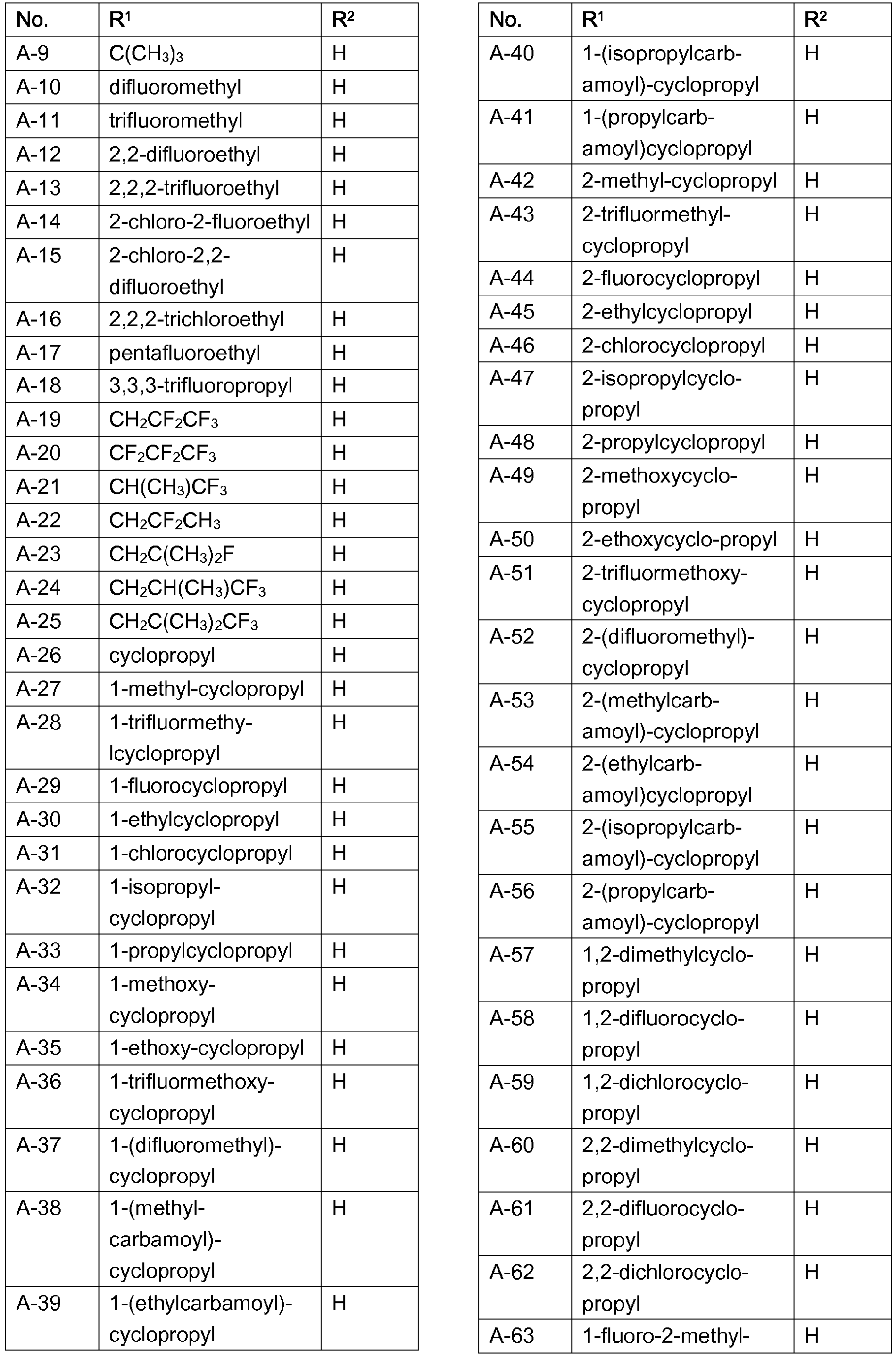

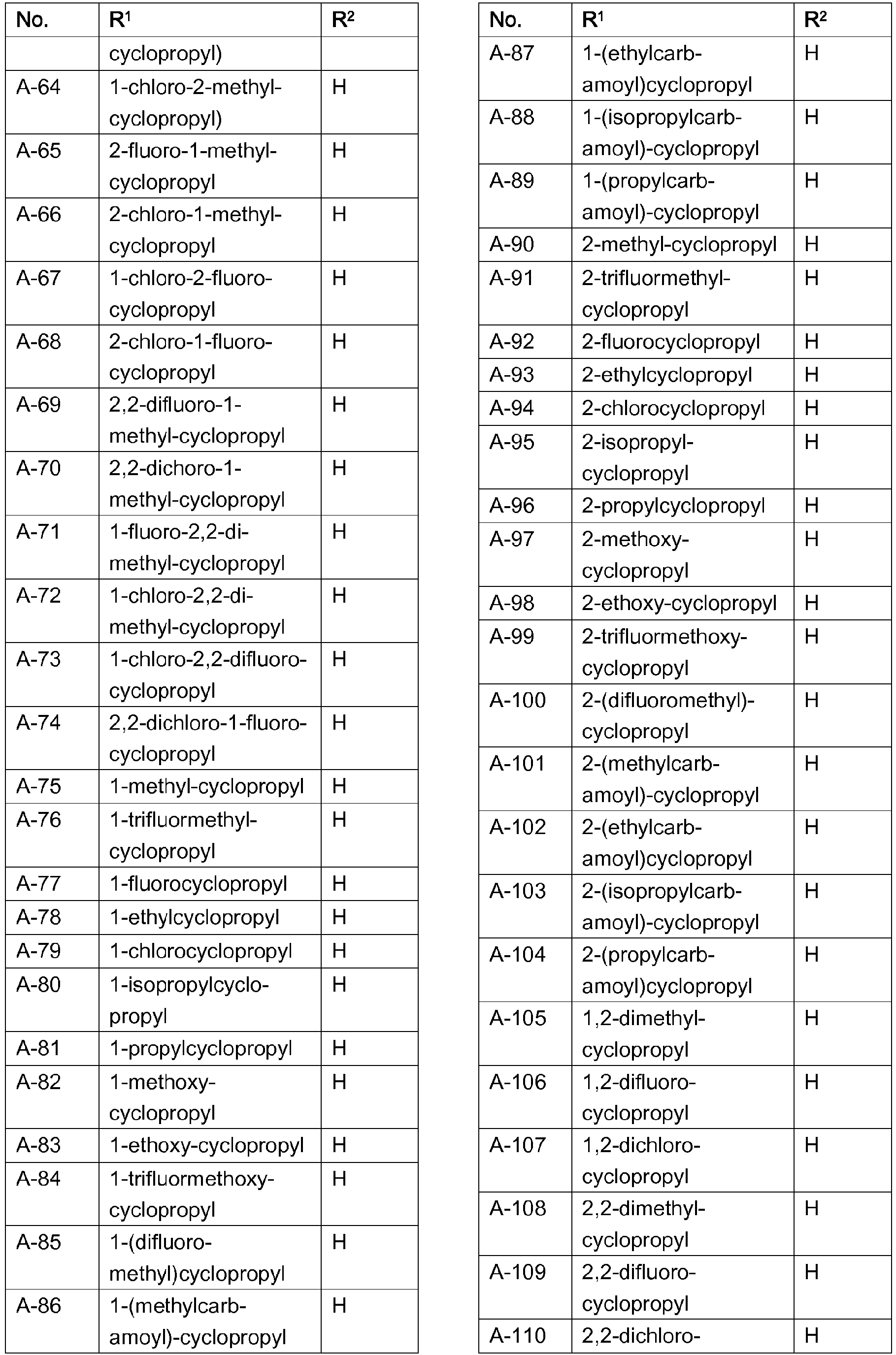

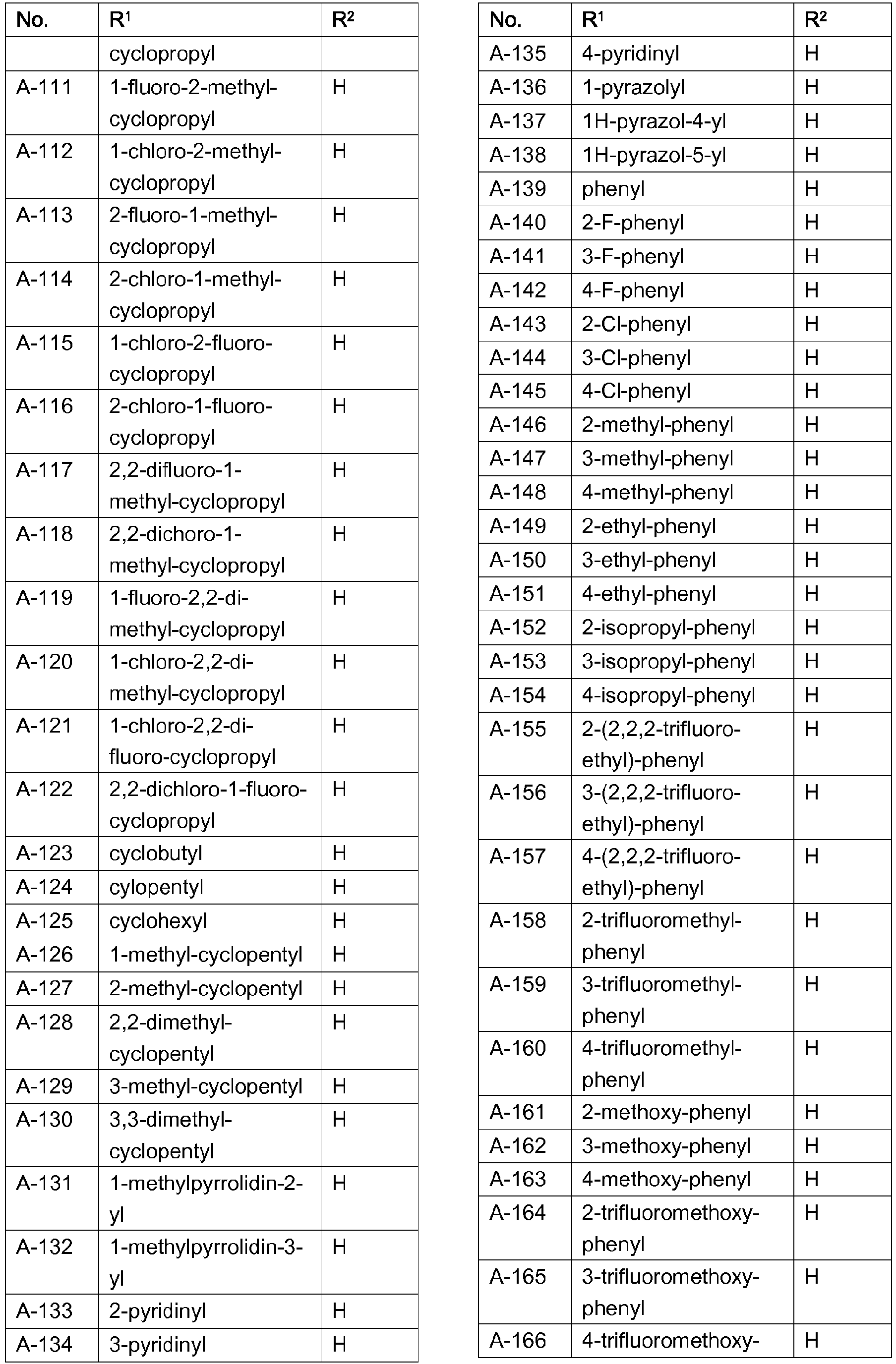

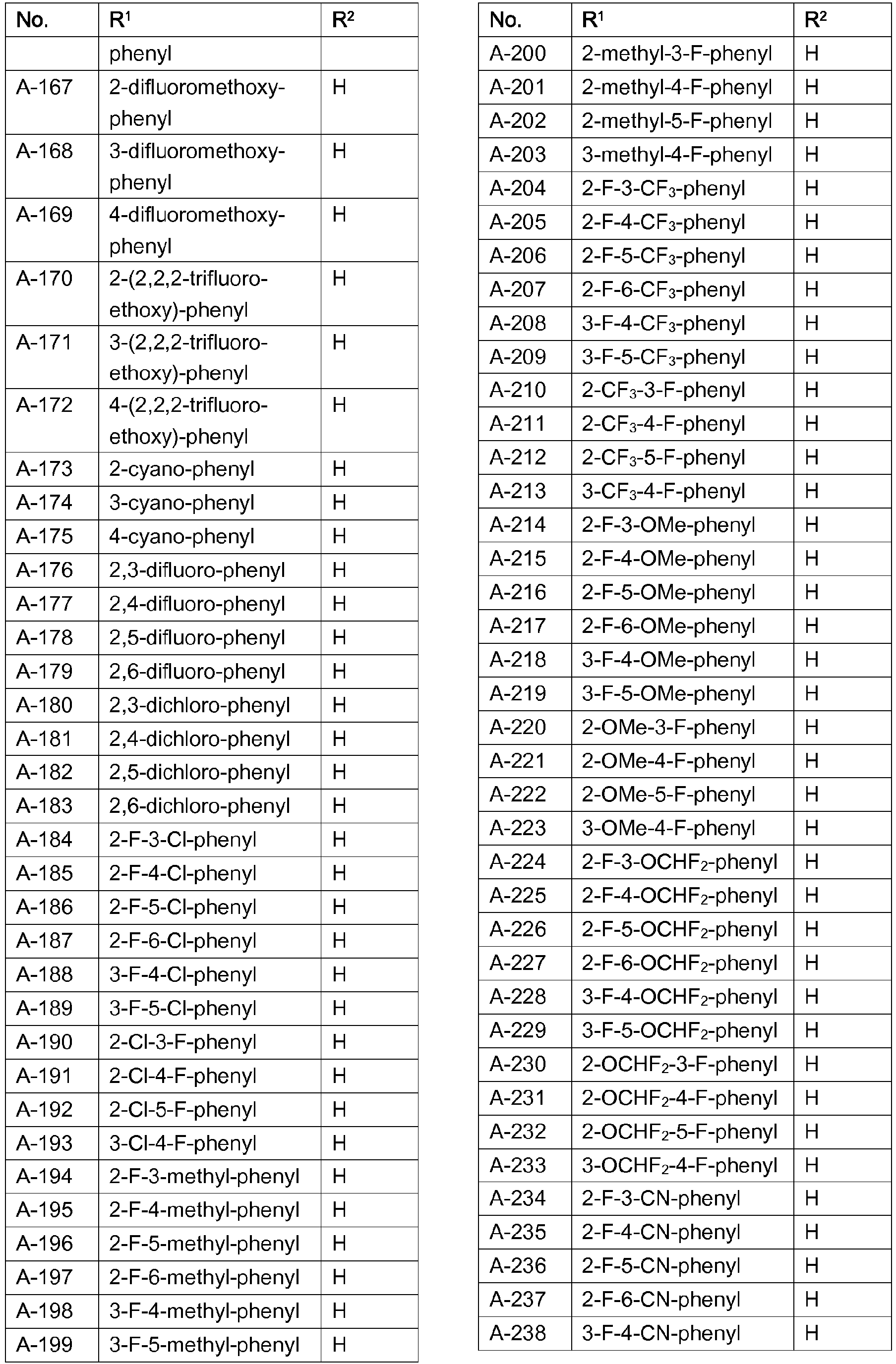

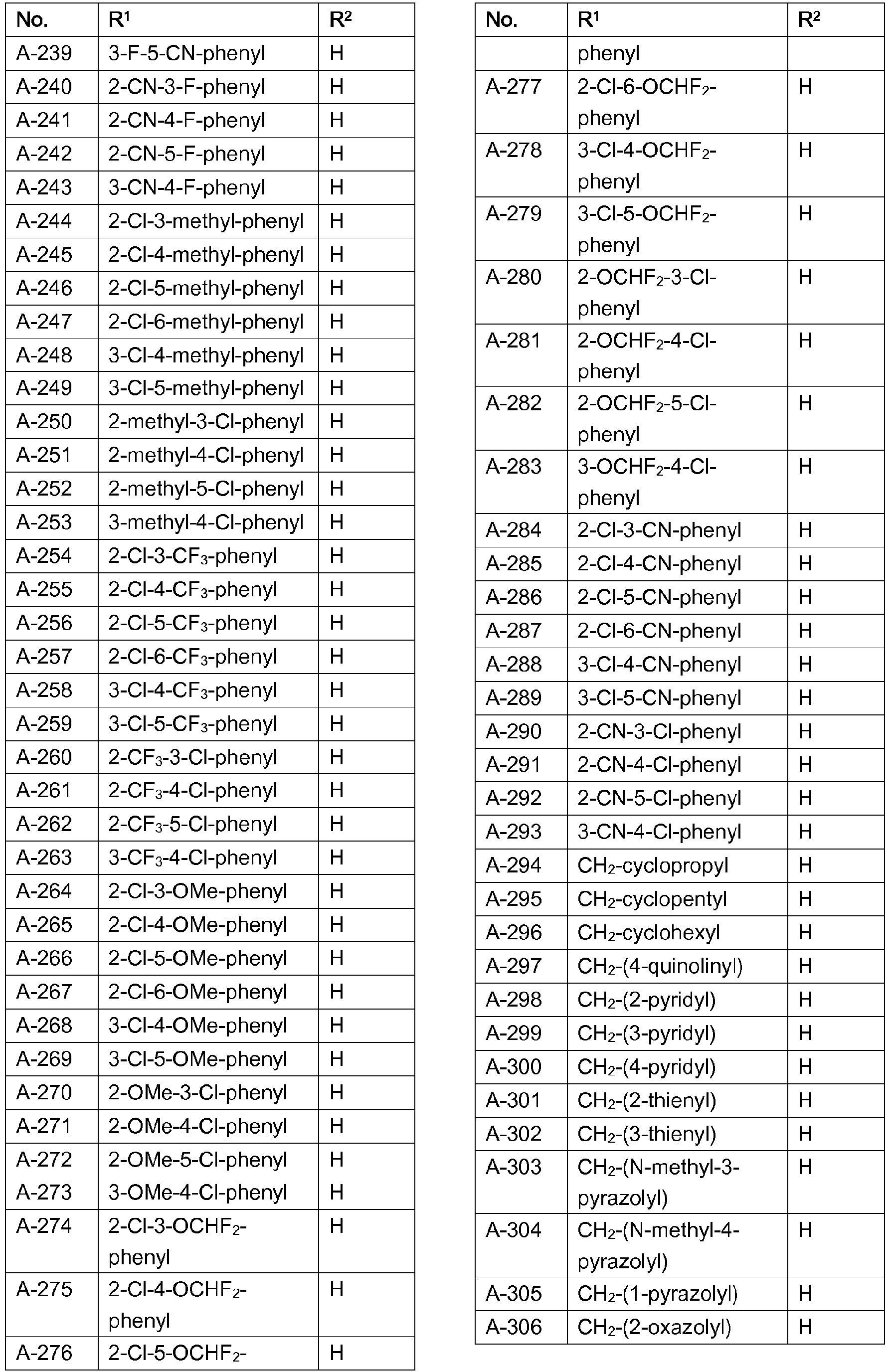

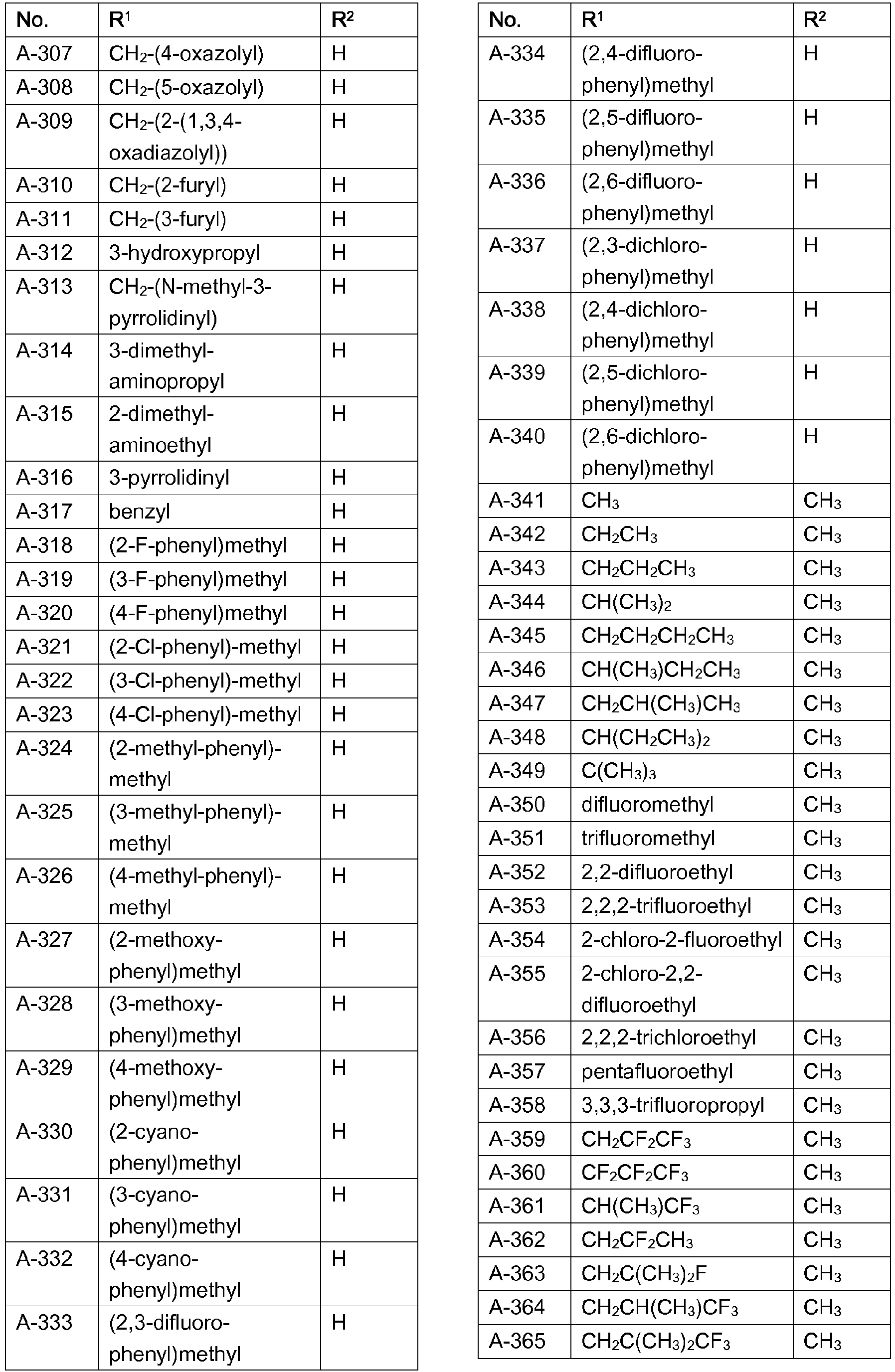

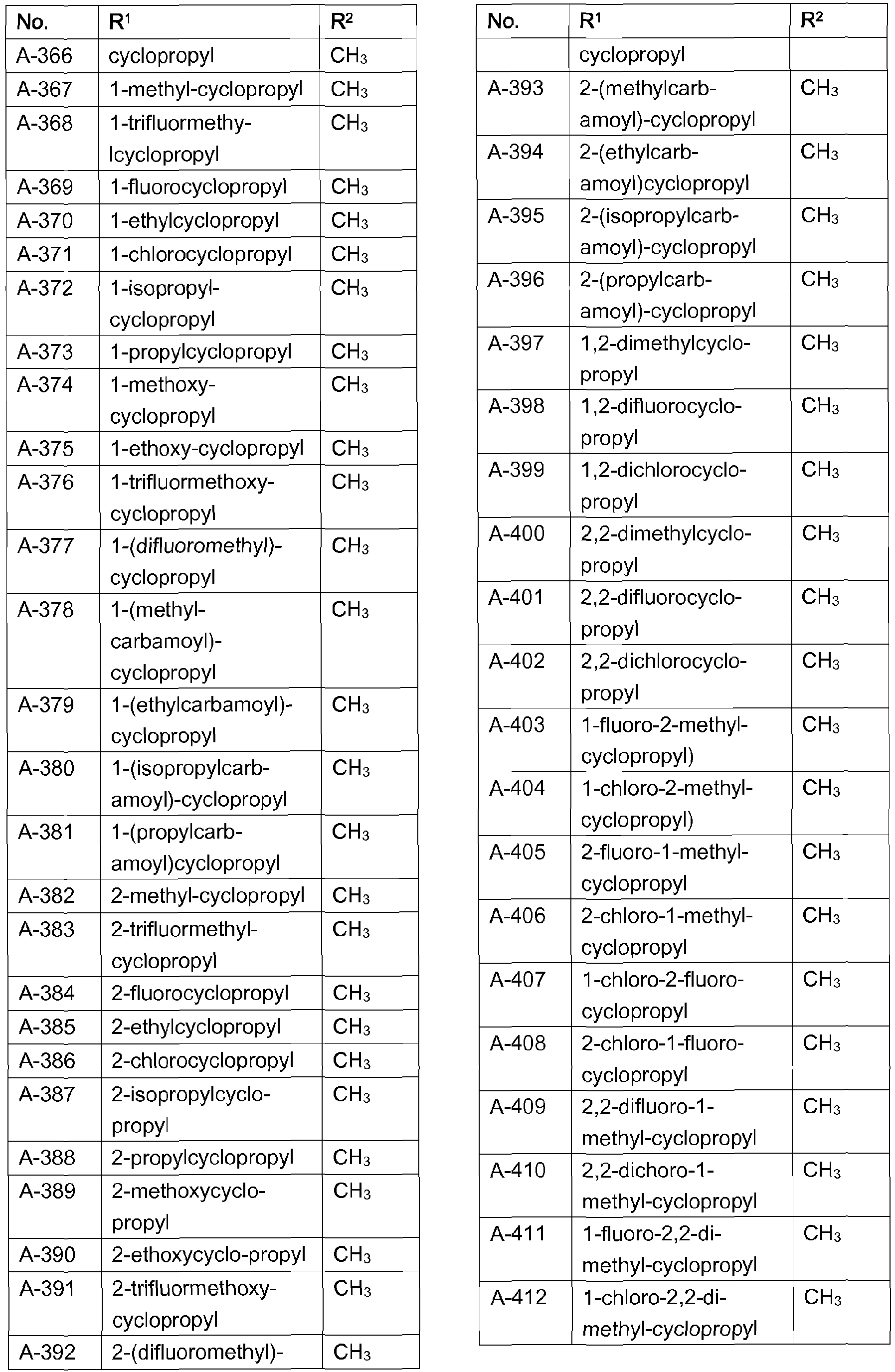

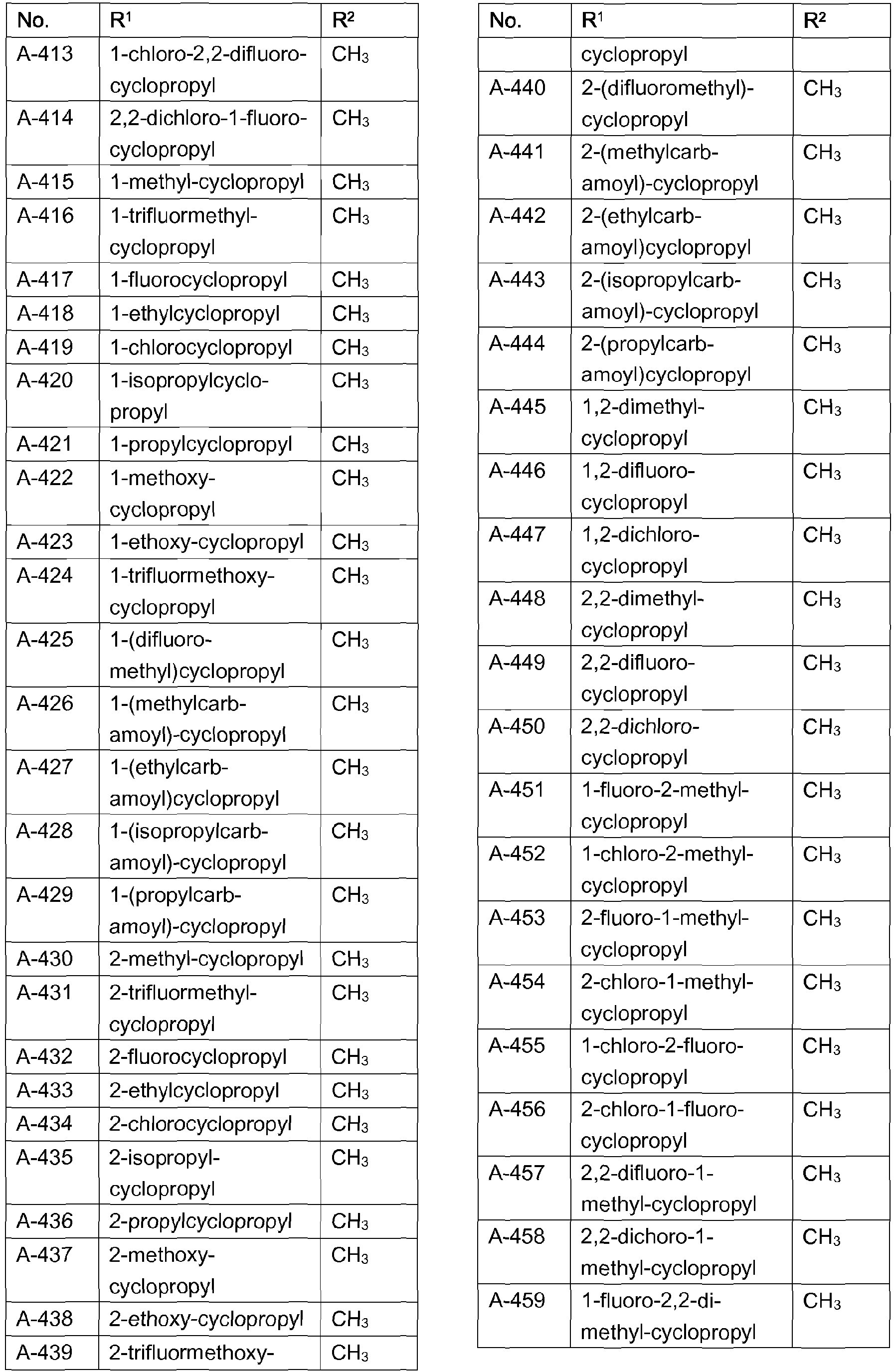

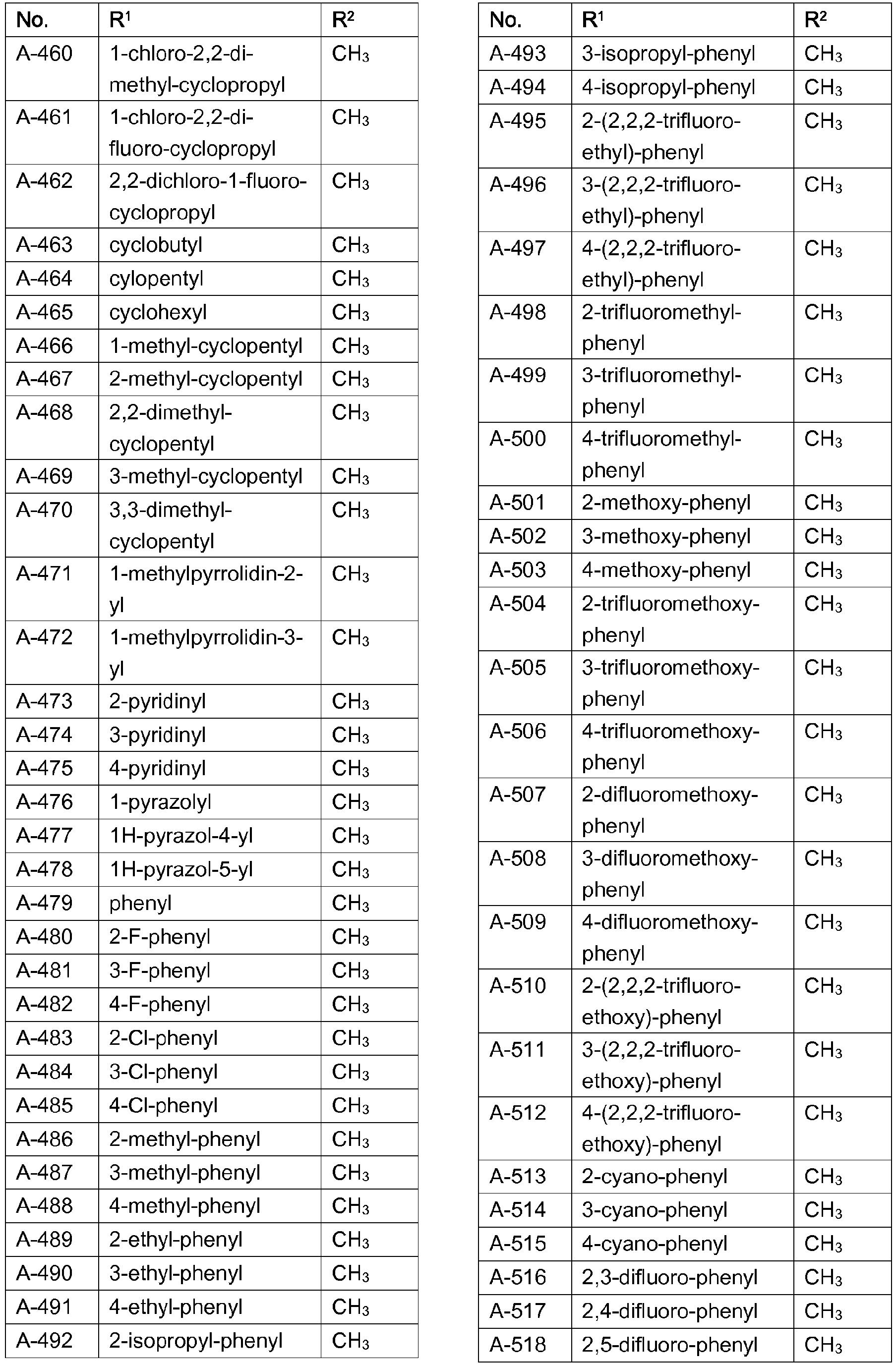

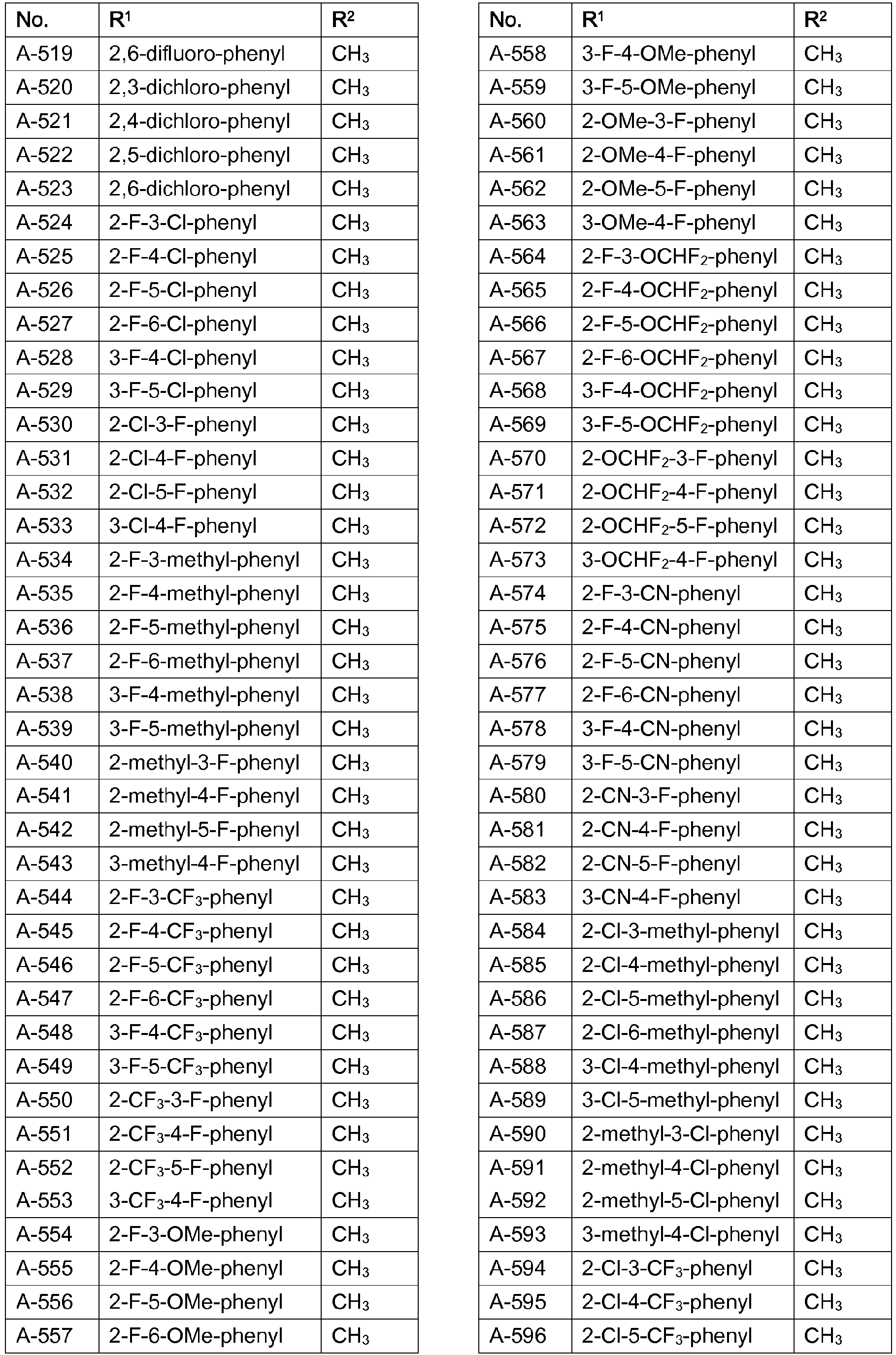

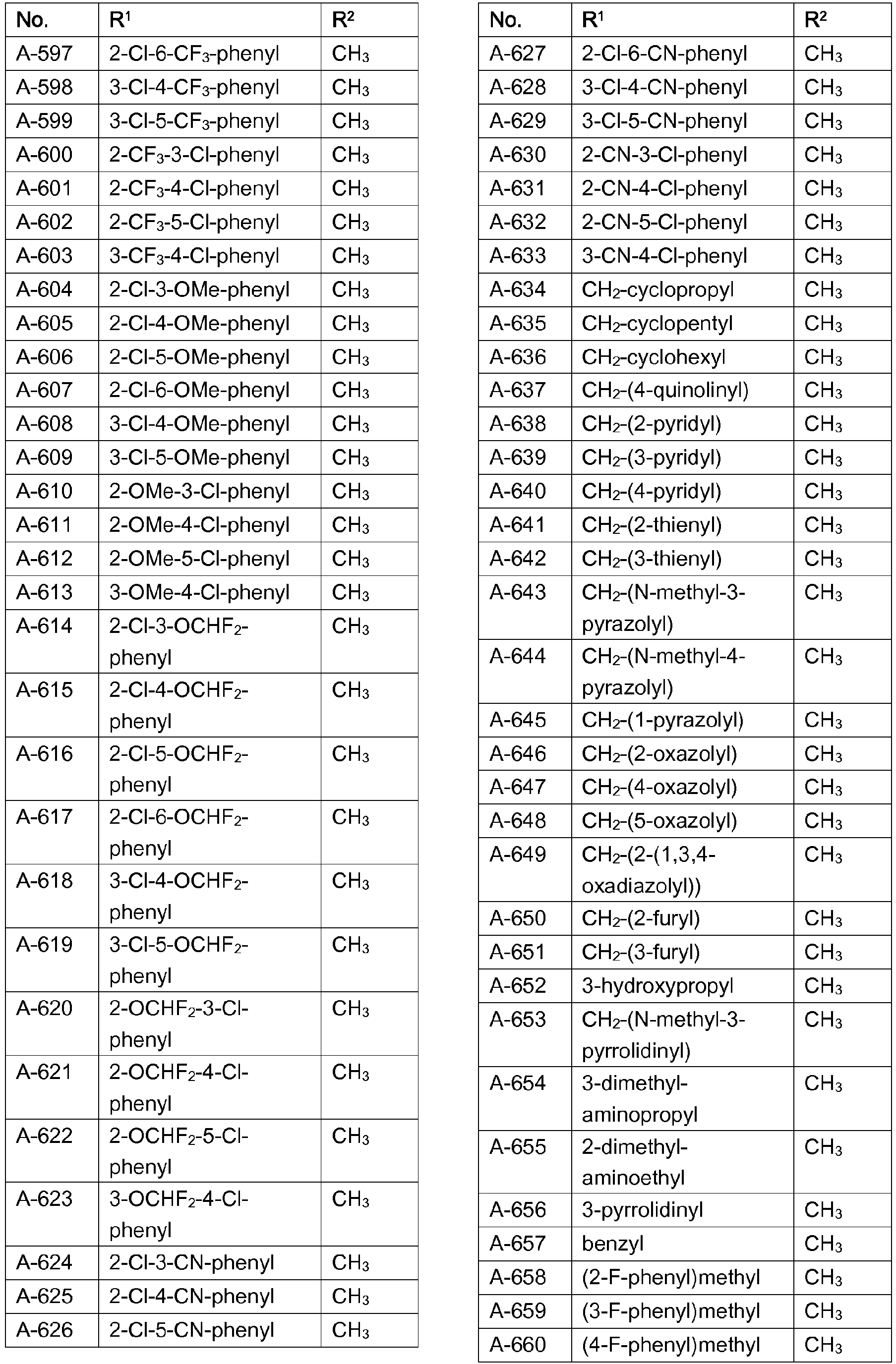

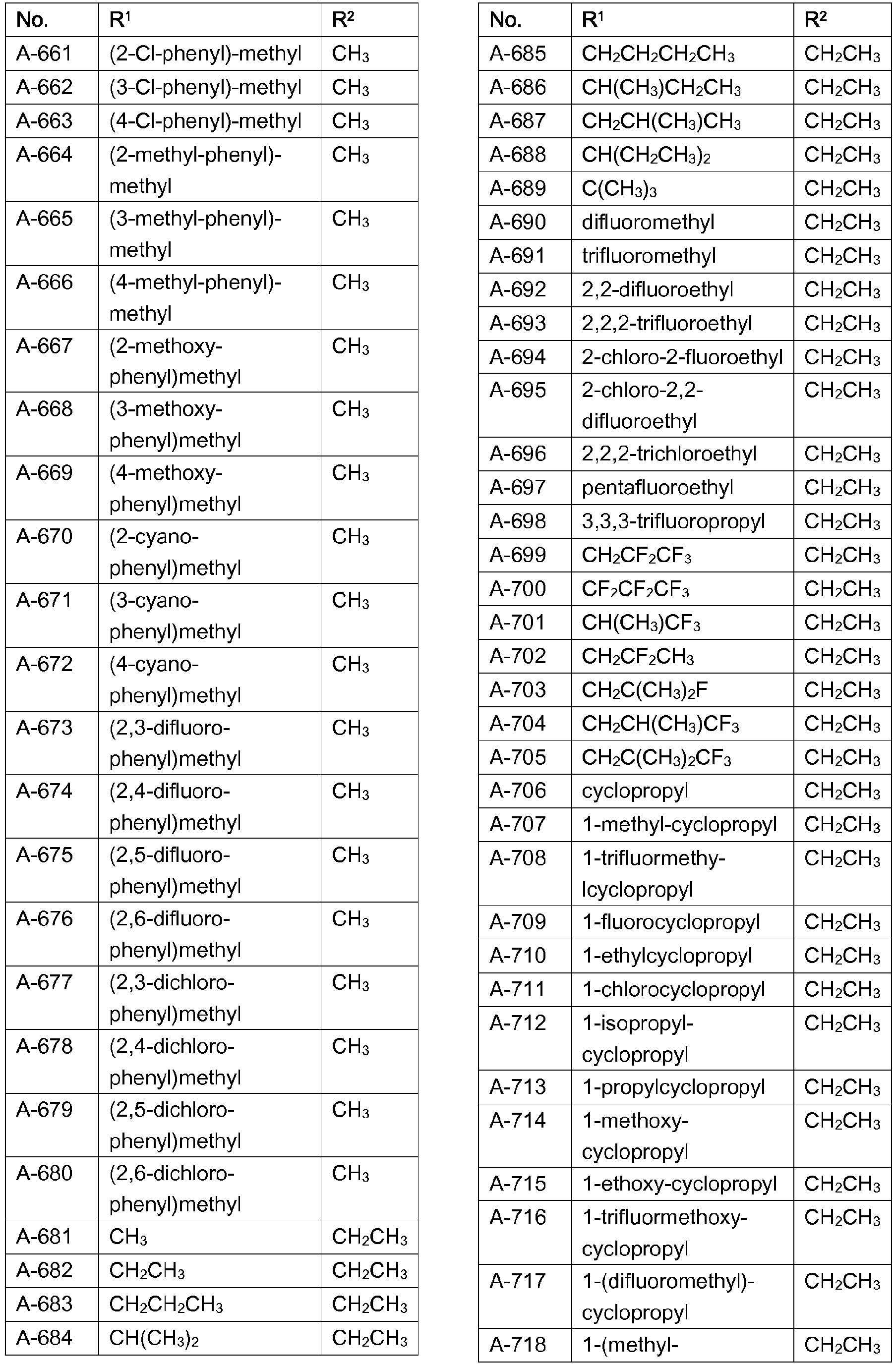

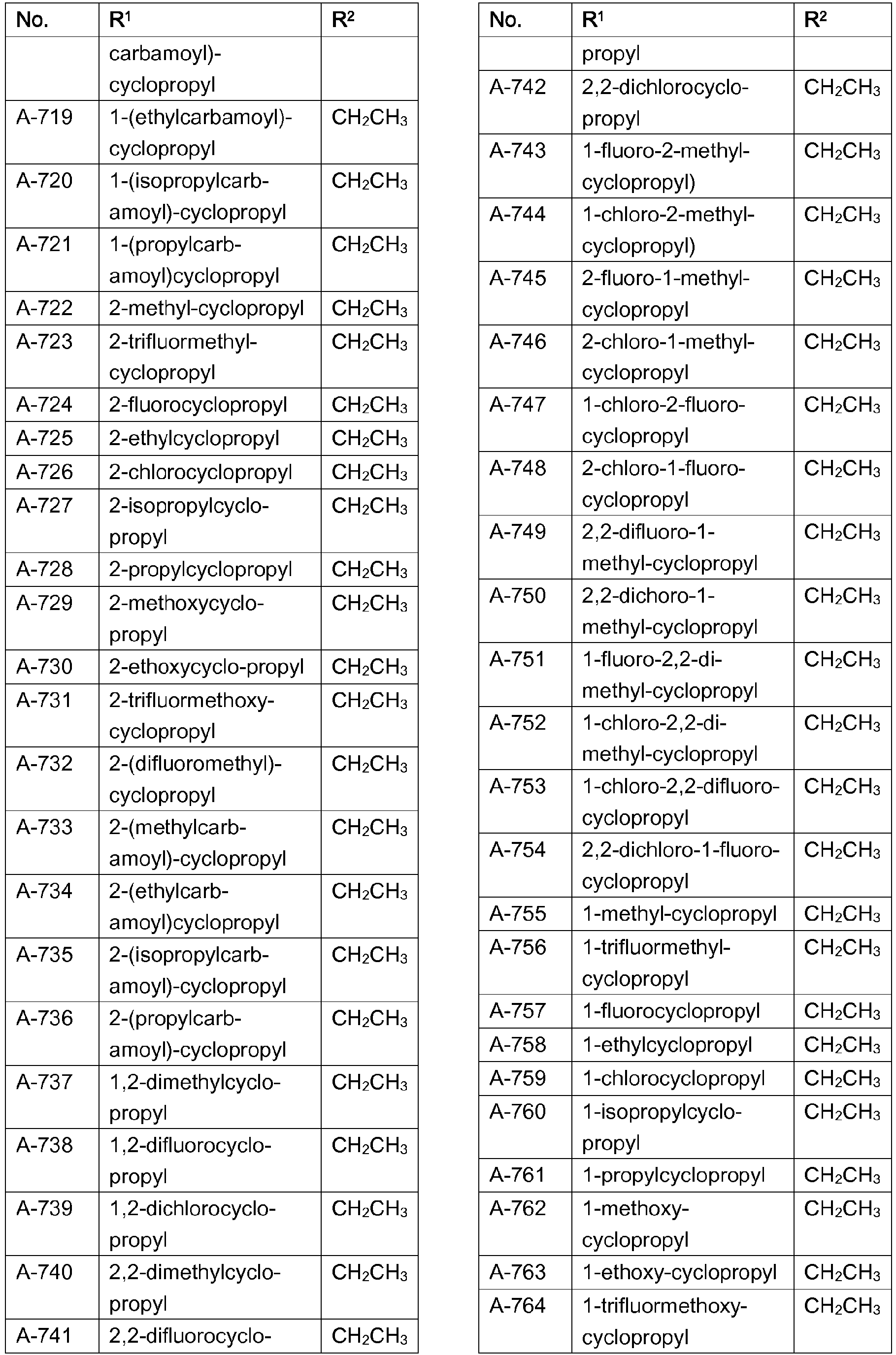

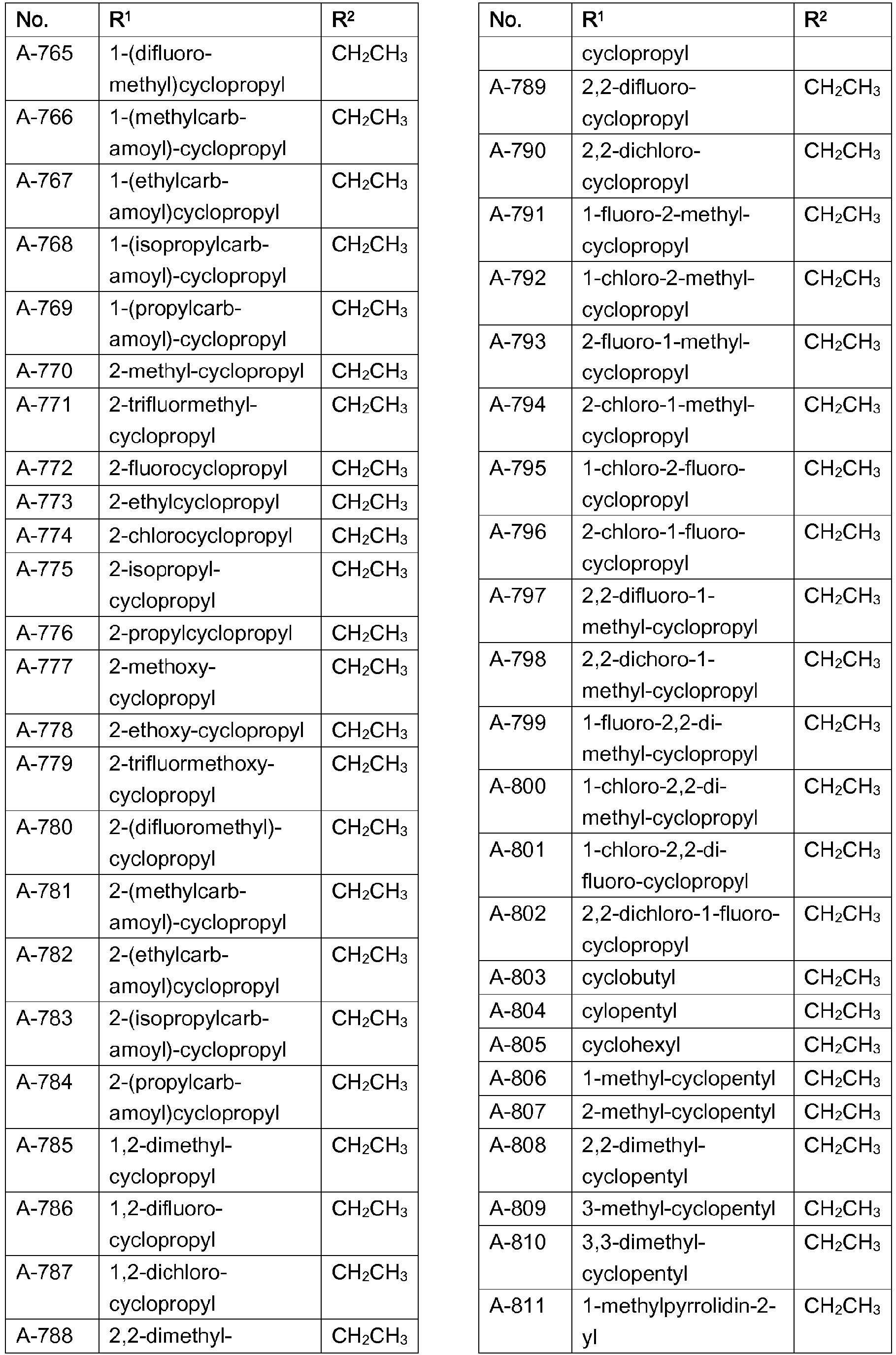

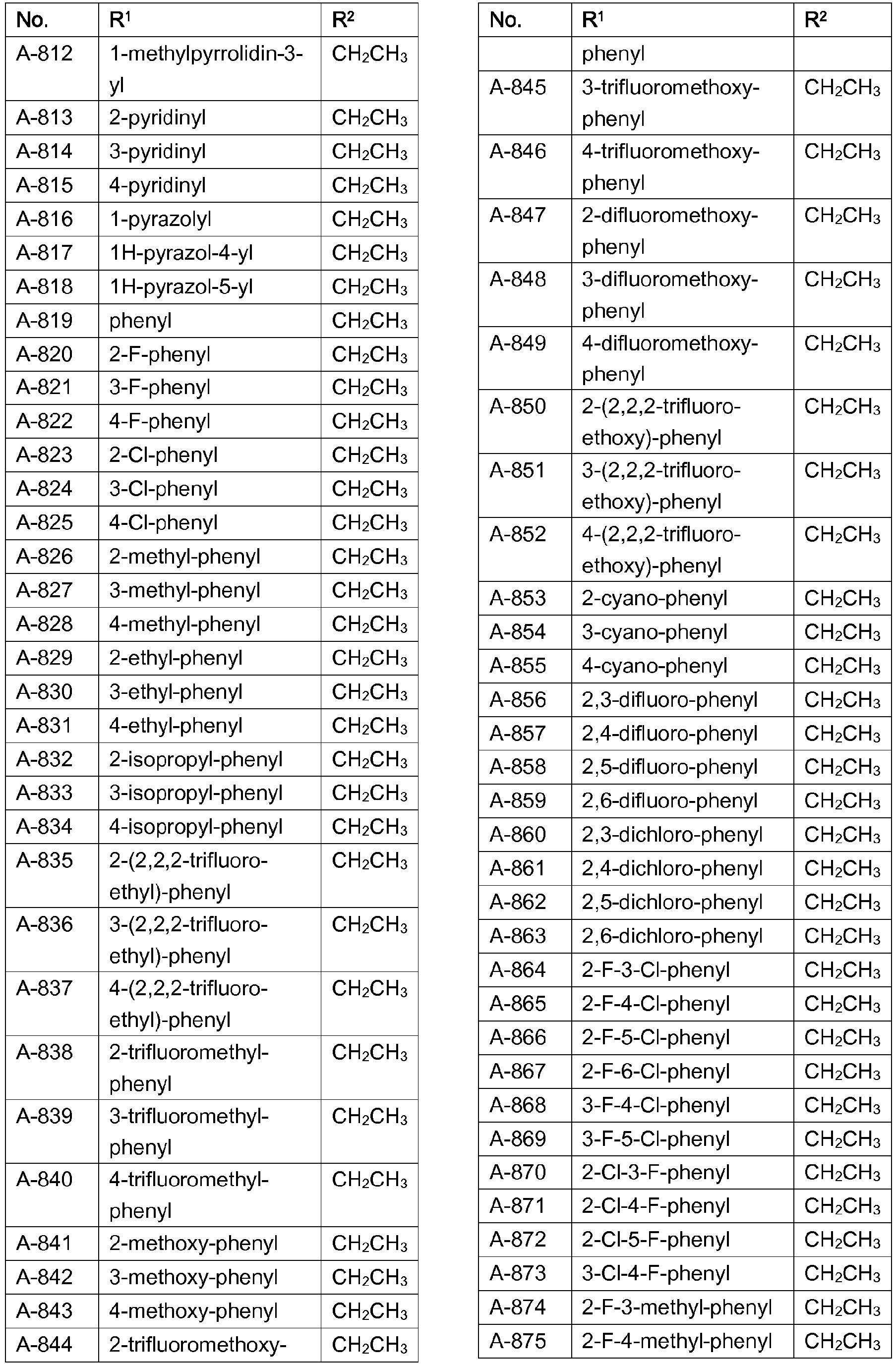

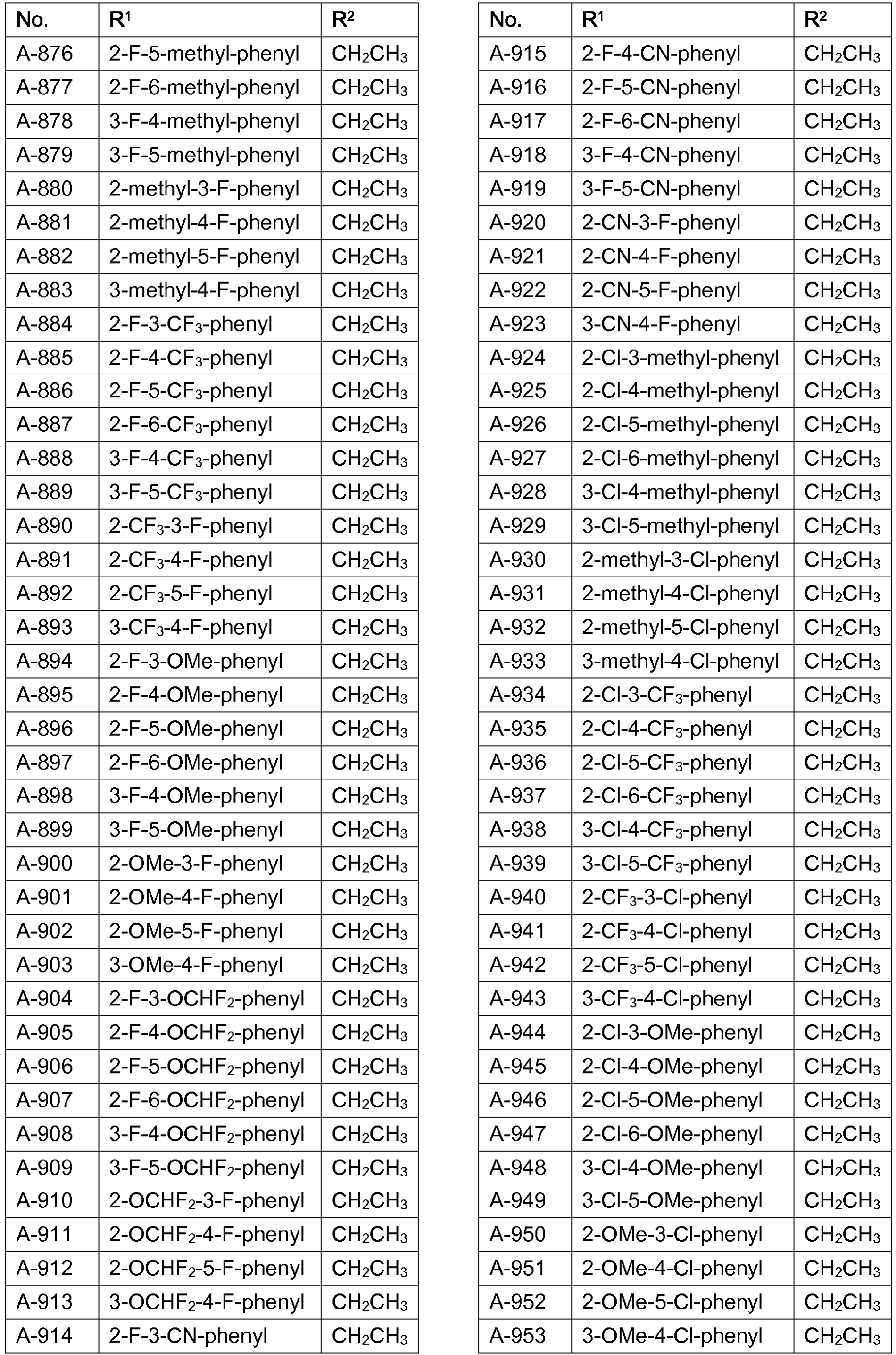

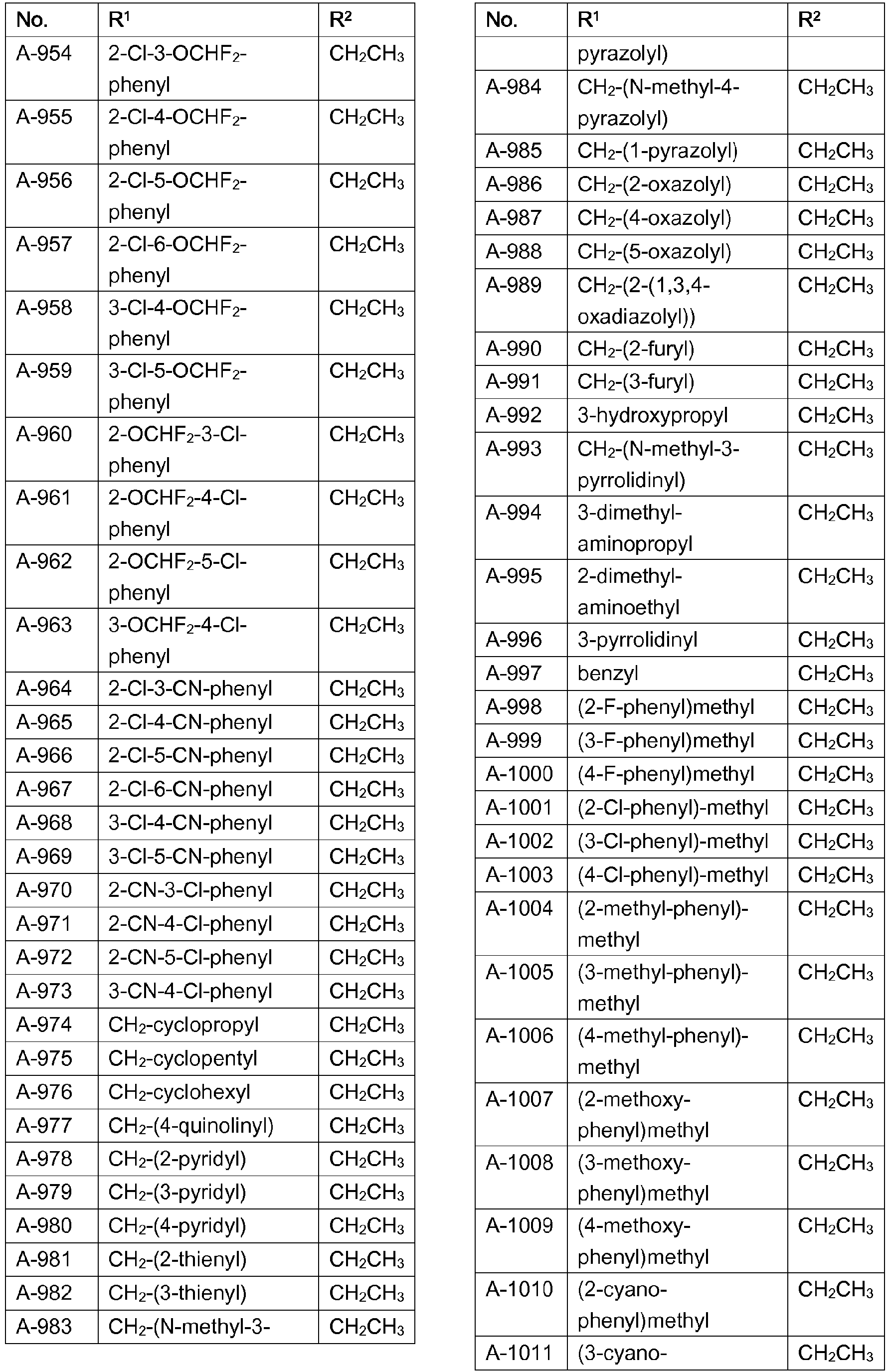

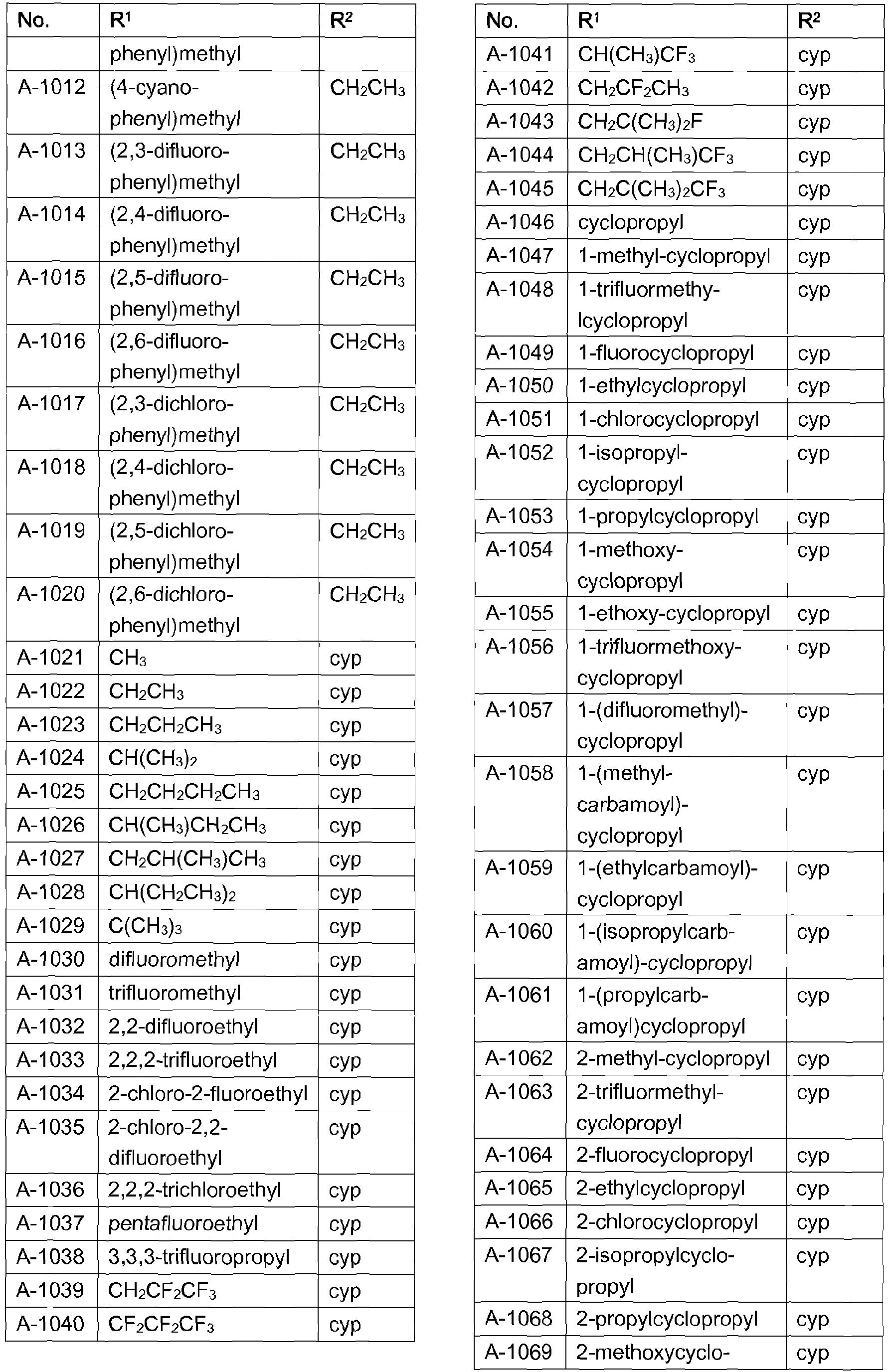

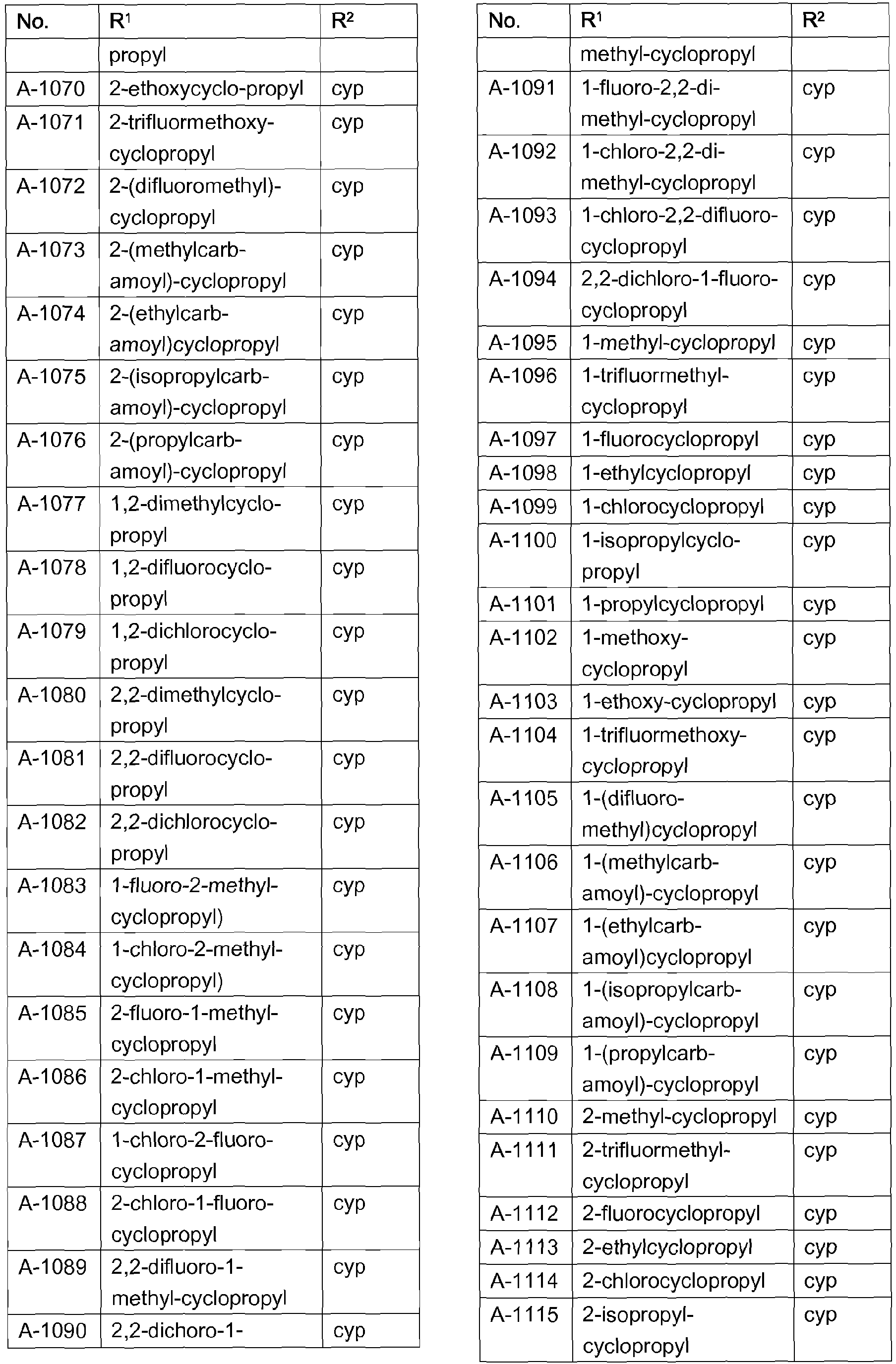

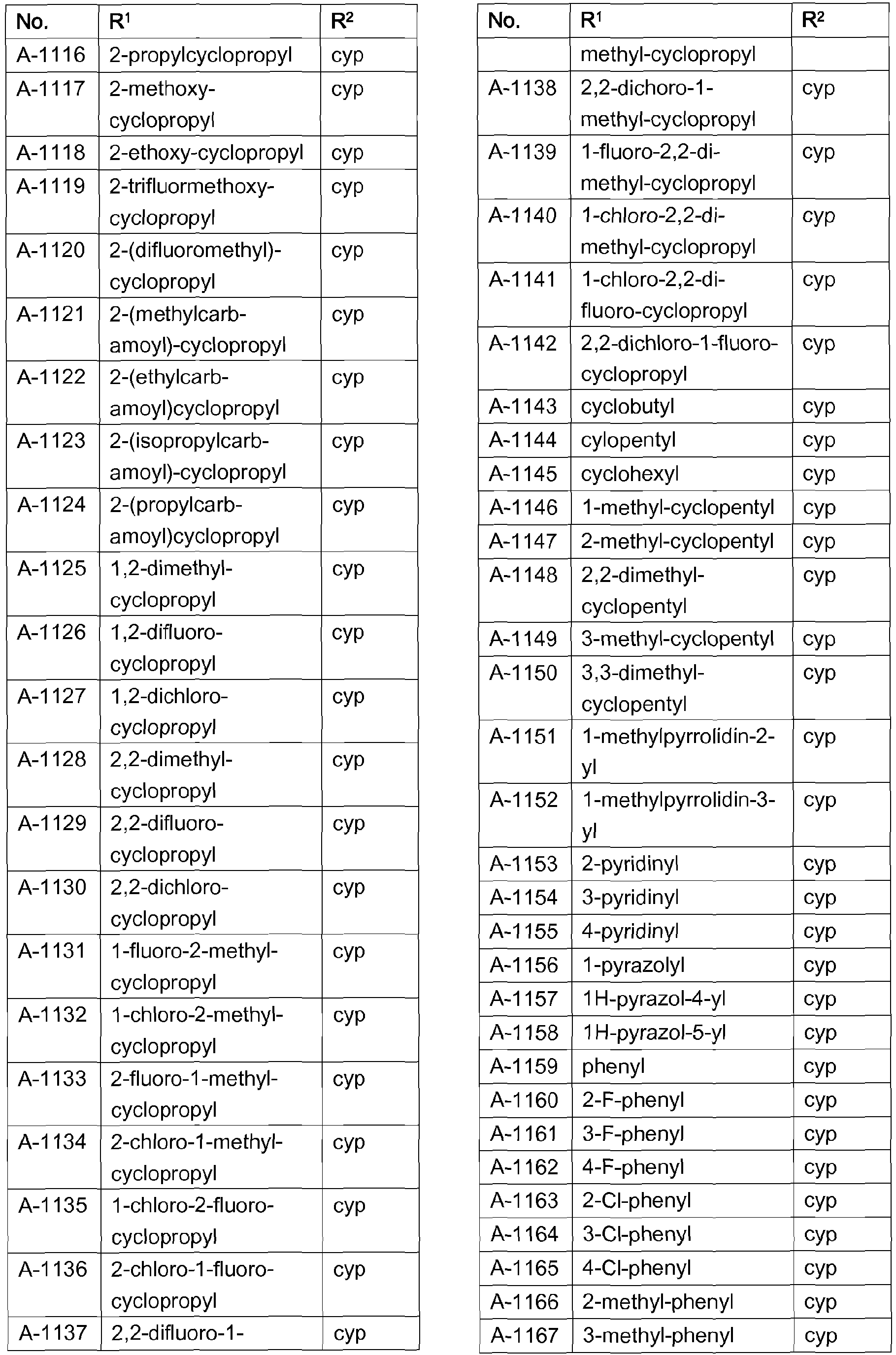

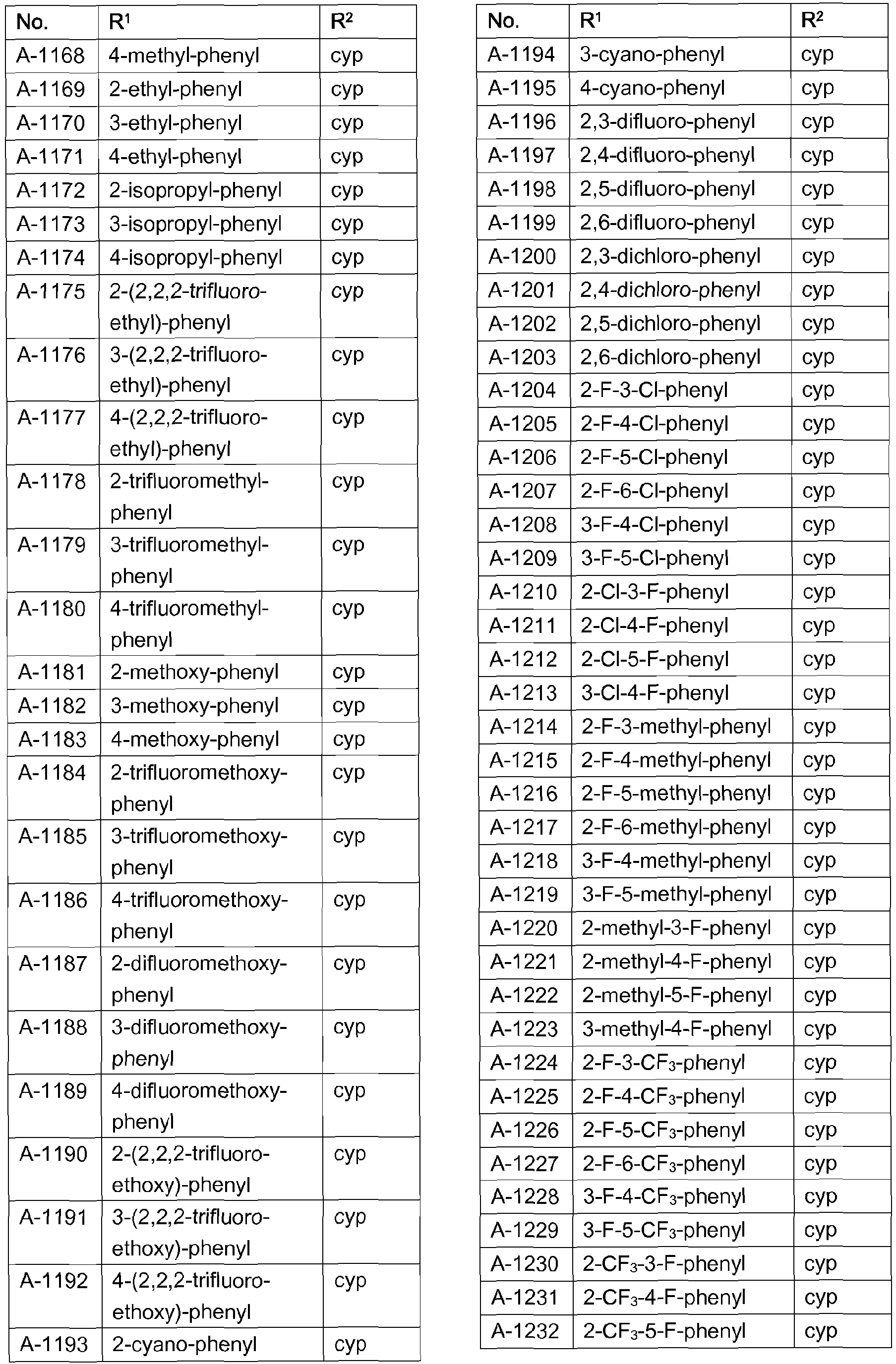

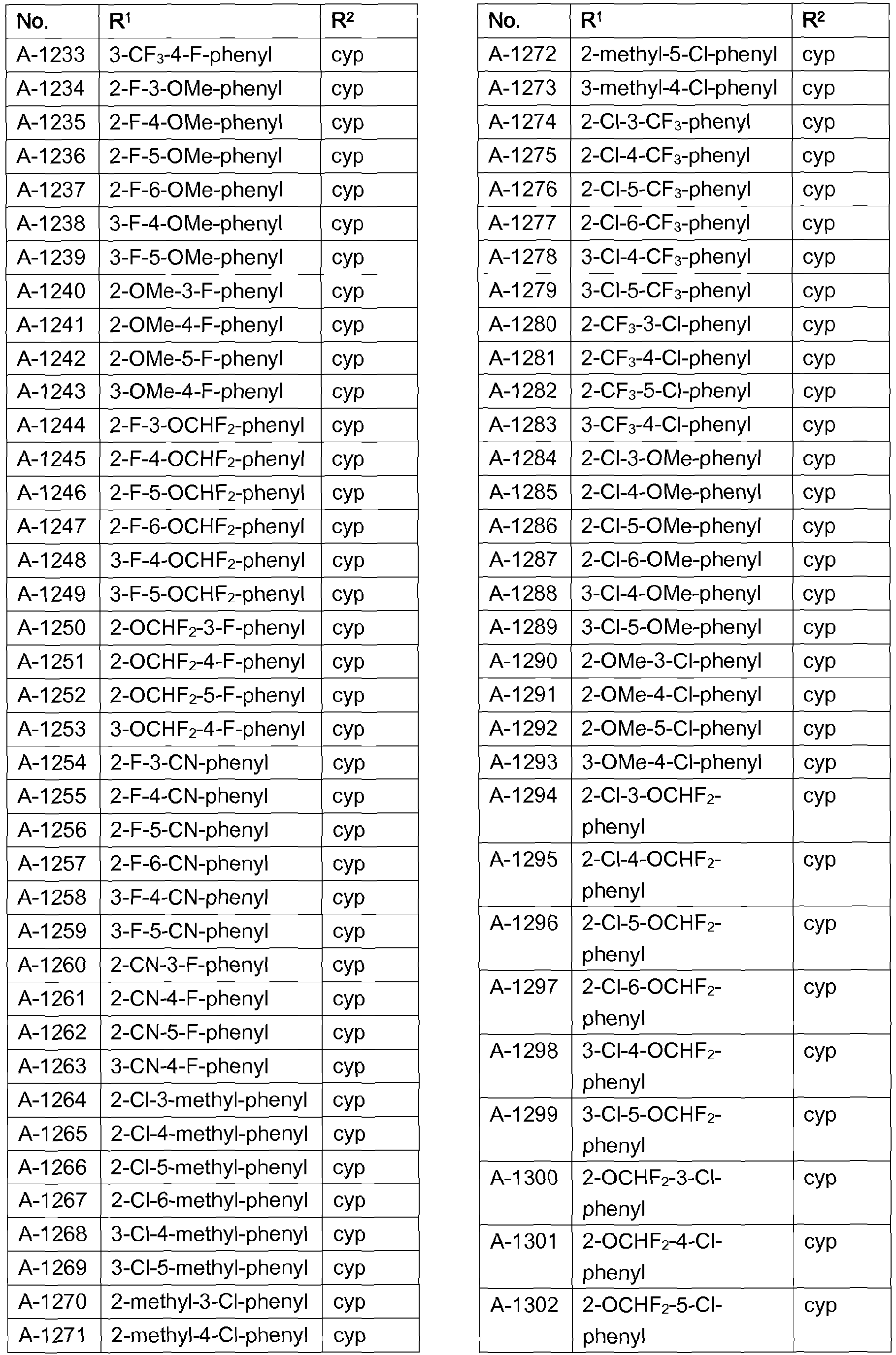

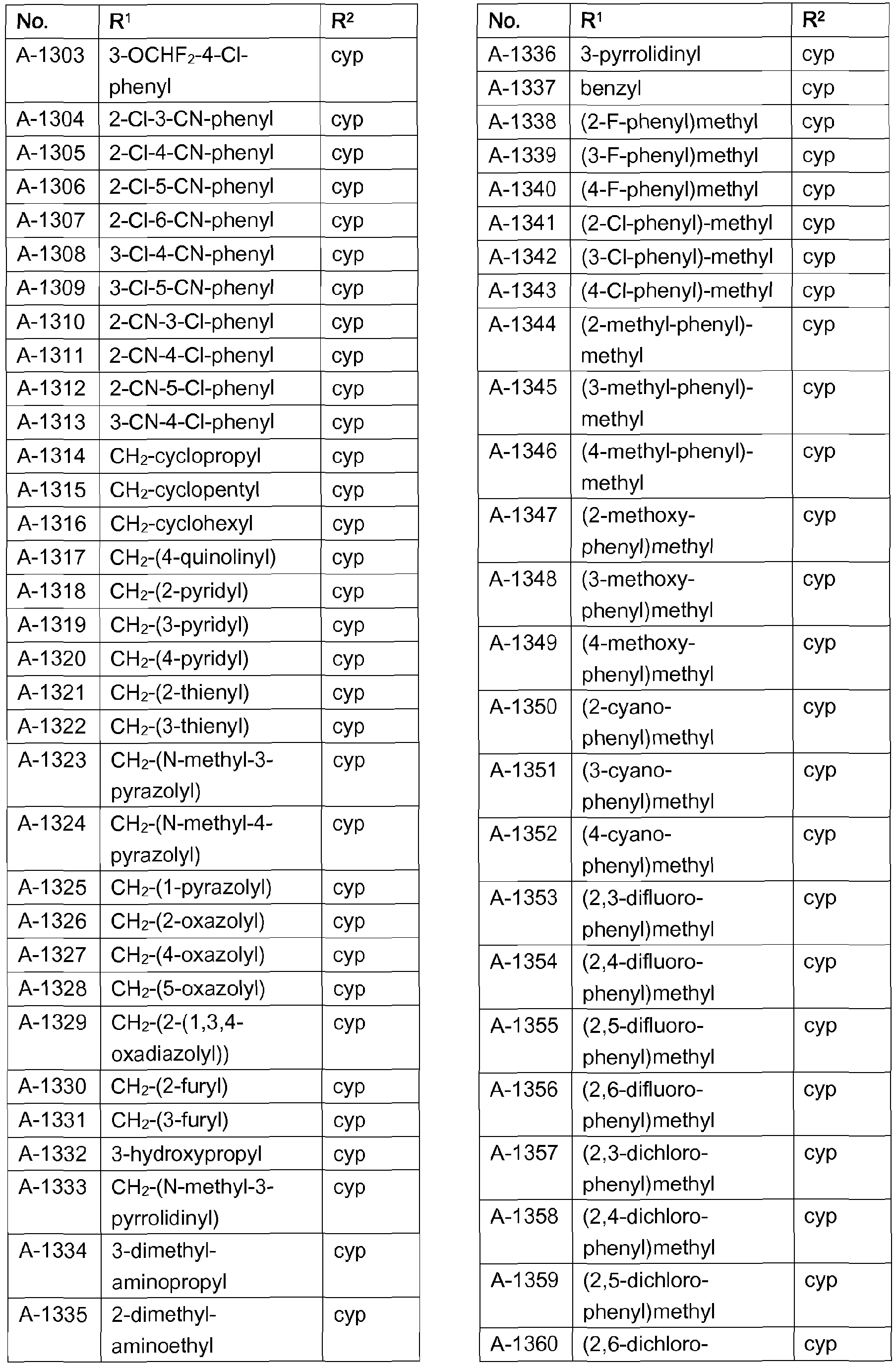

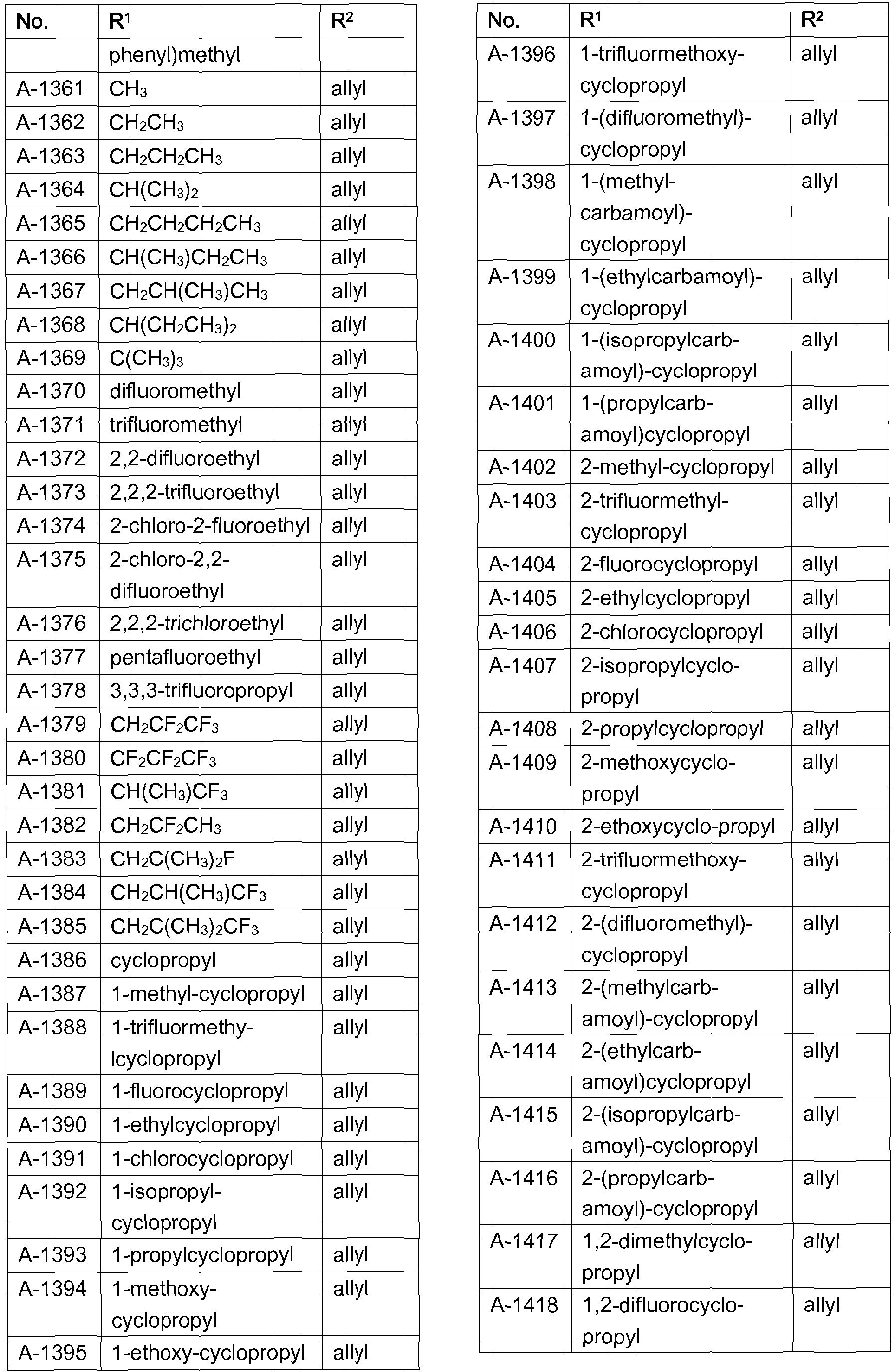

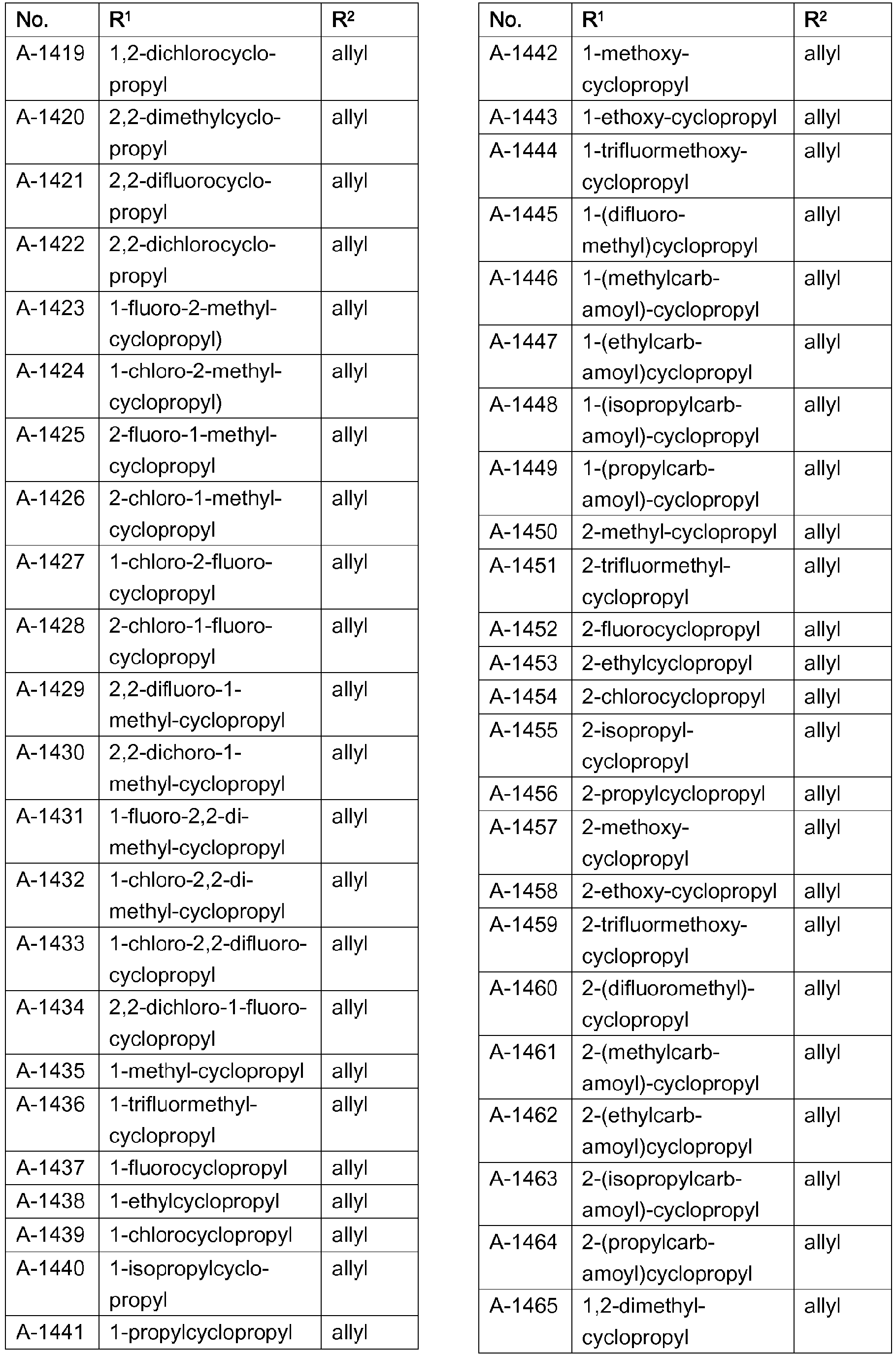

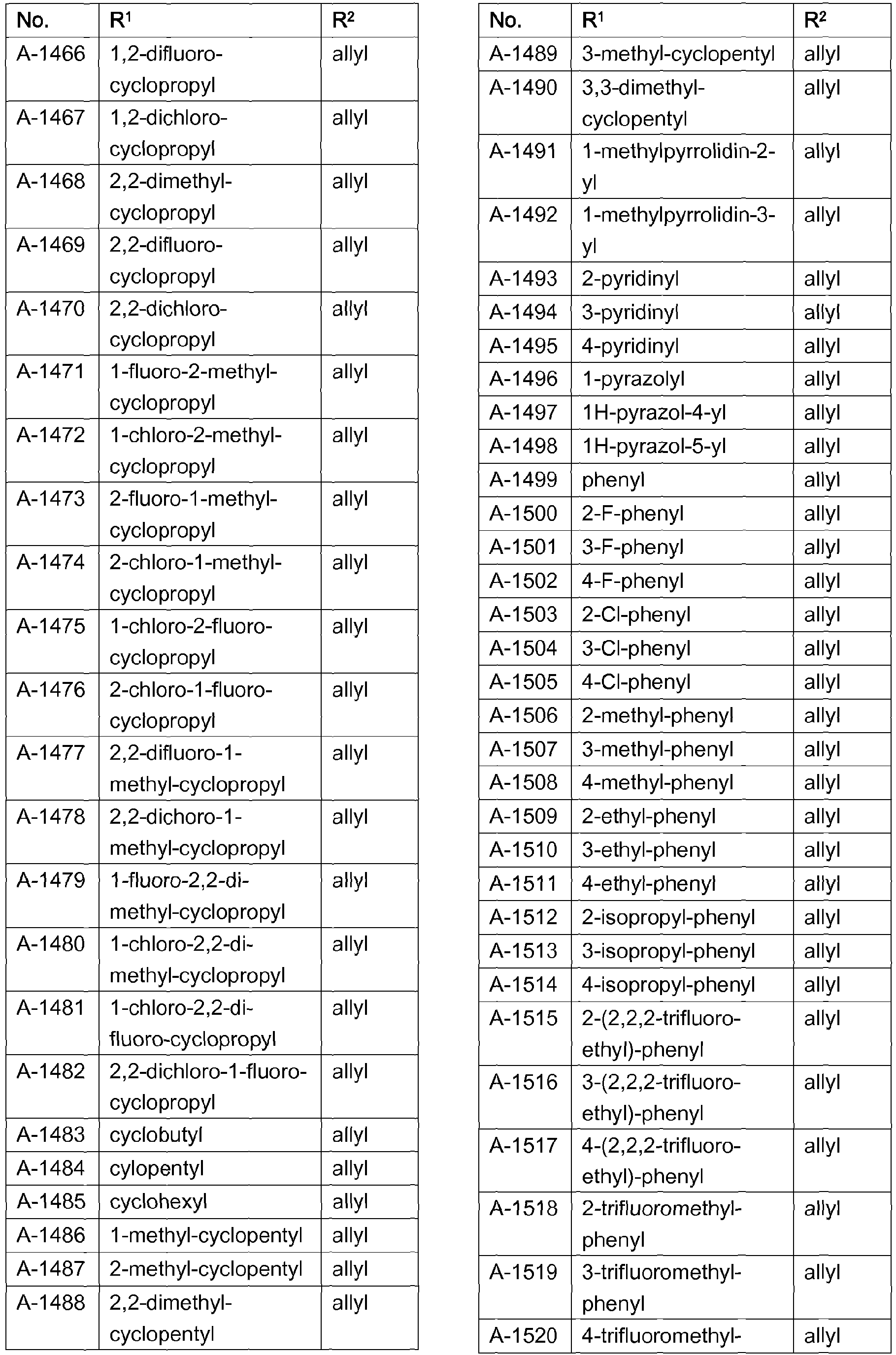

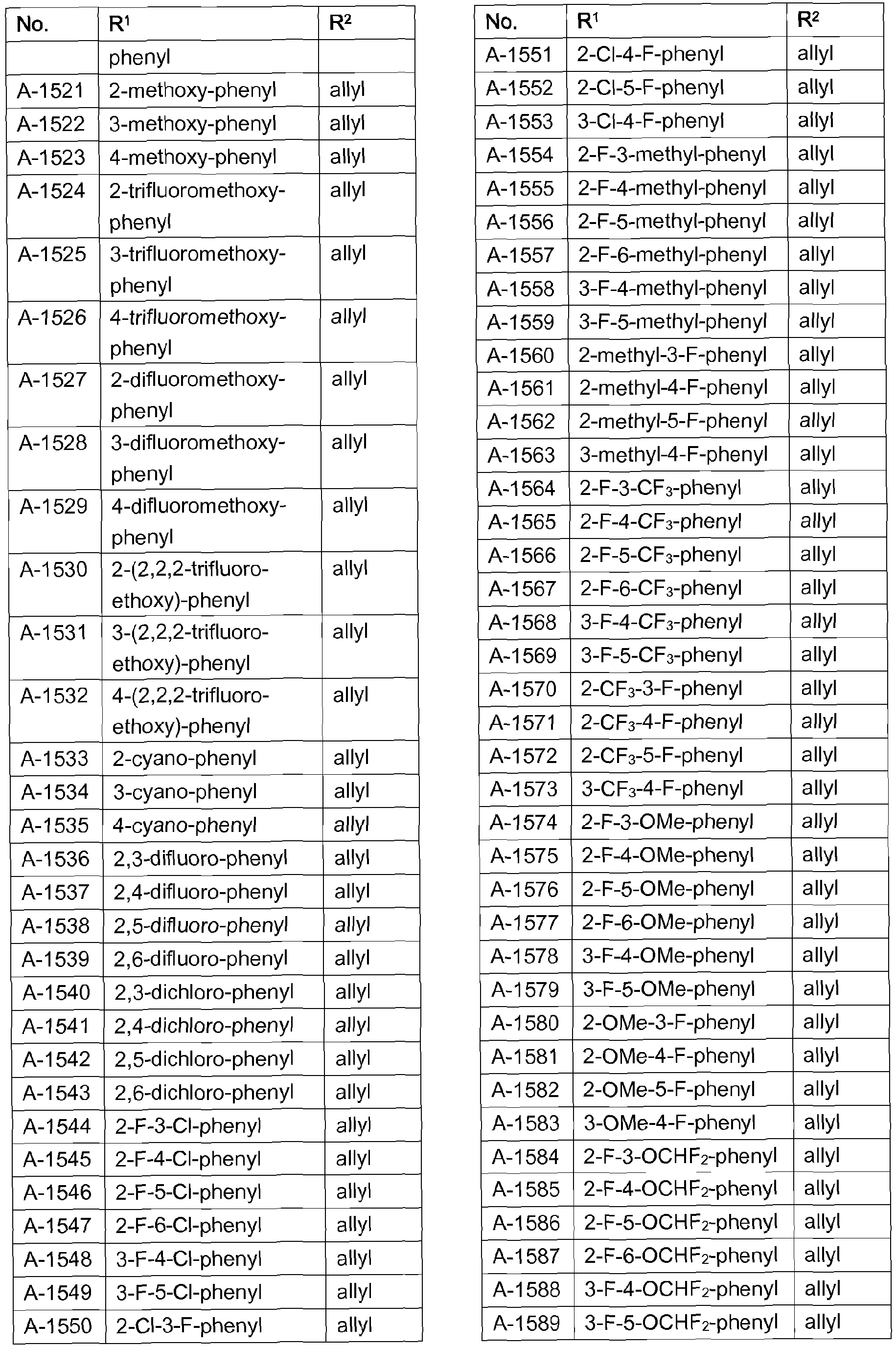

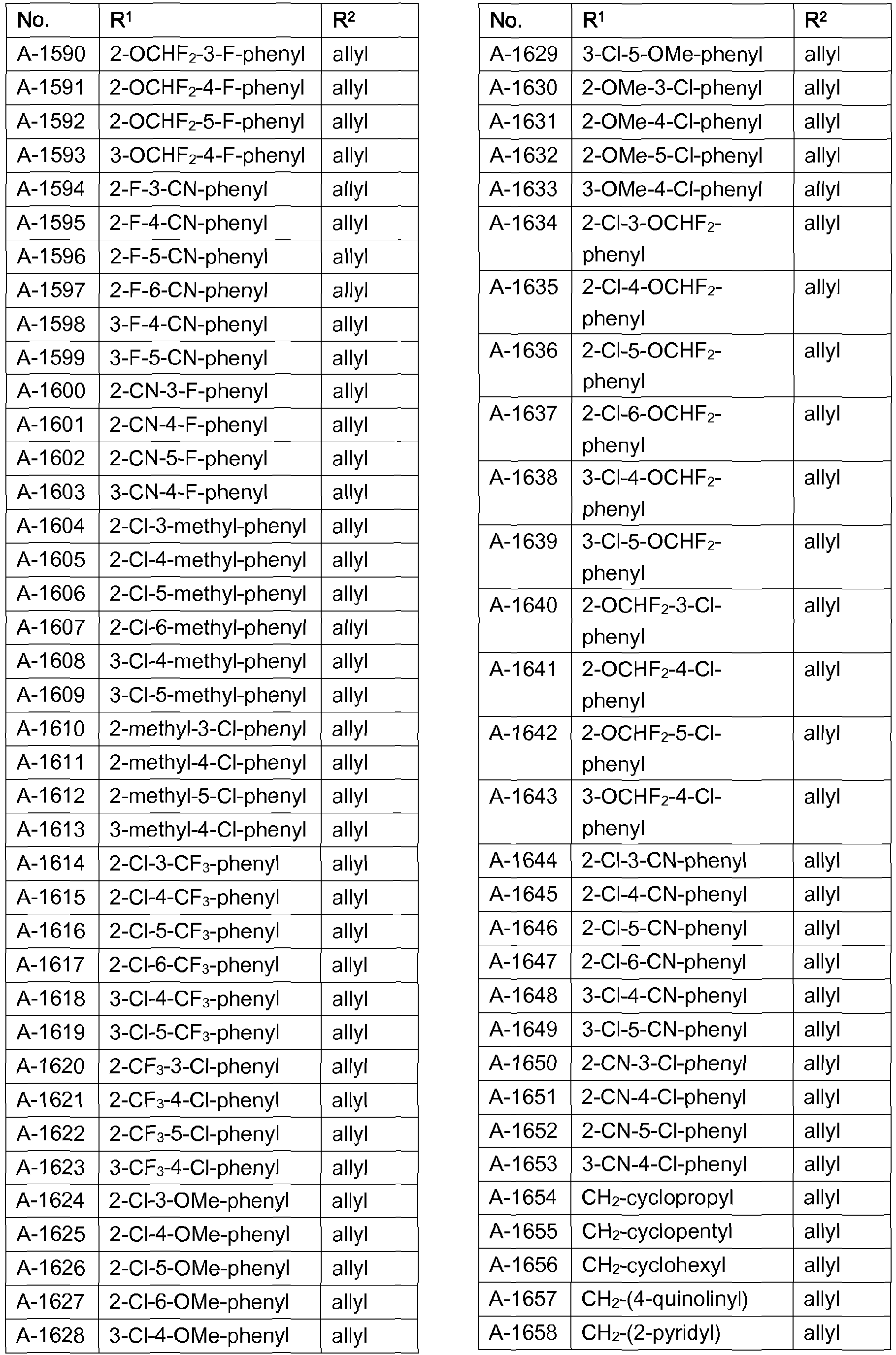

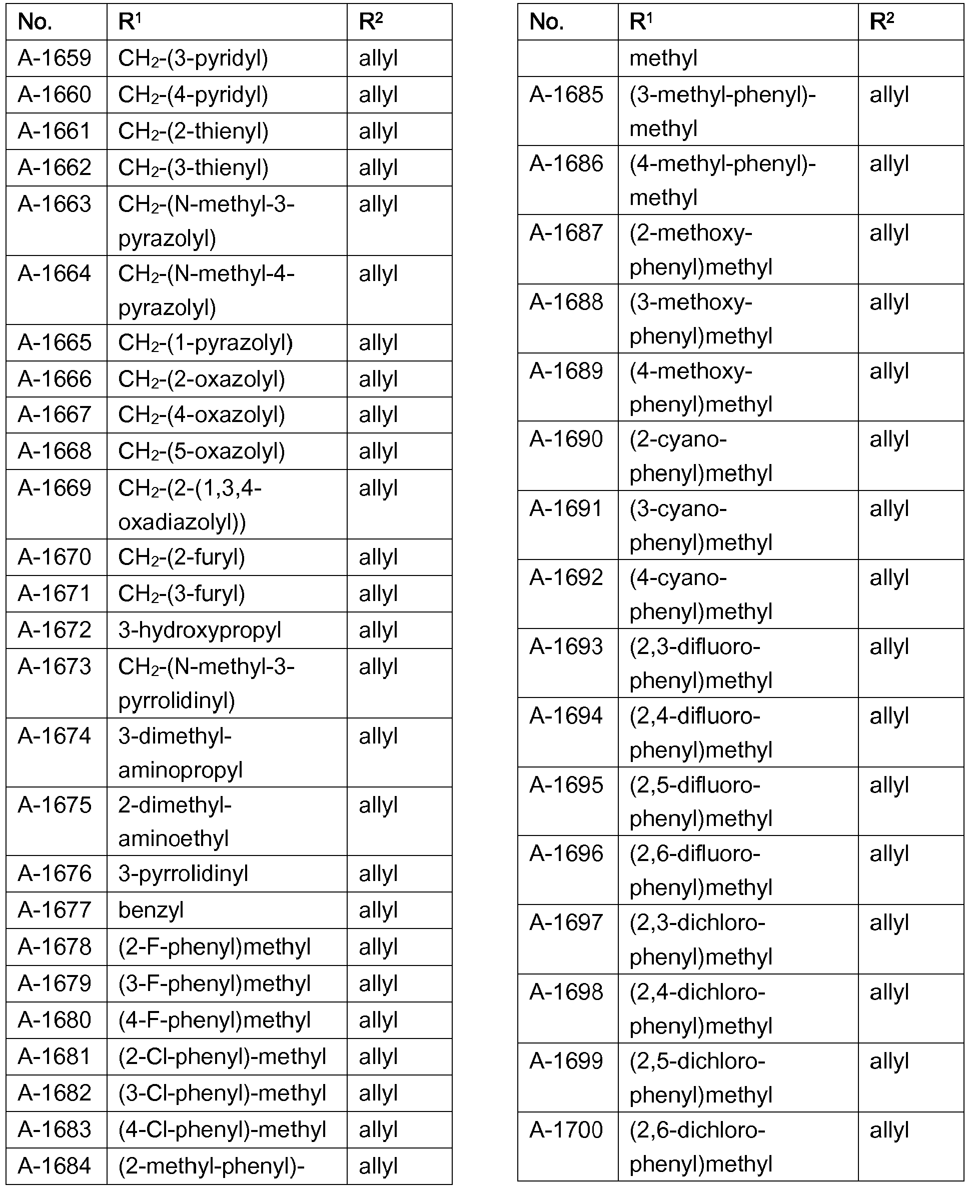

Compounds of the formula I can be prepared as described in the literature starting from diketones of type I-1 by reaction with hydroxylamine (see, for example, WO 2009000662 or Journal of Organic Chemistry 1995, 60(12), 3907-3909). Typically, water or an organic solvent is used, wheras the temperature is in the range between 0 °C and 120 °C, and in the presence of a base. Suitable bases are selected from triethylamine, pyridine, sodium hydroxide, sodium acetate, potassium carbonate or sodium carbonate. Suitable solvents are tertahydrofurane, *N,N*-dimethylformamide, ethanol, methanol, water or mixtures of these solvents. Elevated temperatures between 60 °C and 120 °C are preferred (see for example, Journal of Fluorine Chemistry, 2006, 127(7), 880-888; WO 2008006561).

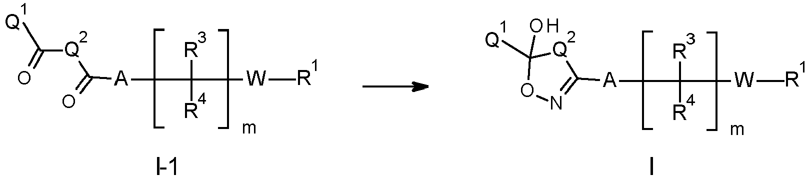

For the preparation of compounds I-2a, wherein Q² is -CH₂-, masked ketones of type I-3 can be used instead of the diketone I-1, whereas P is, for example, alkyl (Journal of Heterocyclic Chemistry, 1996, 33(6), 1619-1622; Ultrasonics Sonochemistry, 2006, 13(4), 364-370).

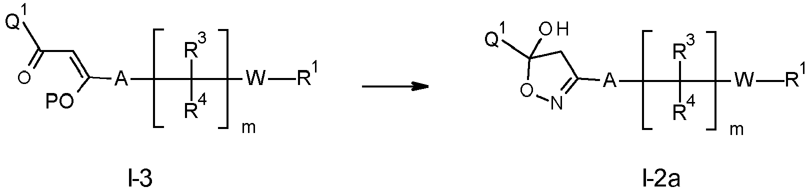

Acylation of the hydroxyl group in compounds of the formula I, provides access to compounds, wherein R is (C=O)-C₁-C₄-alkyl or C(=O)-C₁-C₄-alkoxy as defined herein. The transformation can be achieved as described in the literature (Journal of Heterocyclic Chemistry 2005, 42(7), 1253-1255) by using acid chlorides or acid anhydrides in the presence of a base. Preferably the reaction is carried out between 0 °C and room temperature in tetrahydrofurane or dioxane in the presence of triethylamine, pyridine or diisopropylethylamine. Alkylation or silylation of the OH-group can be accomplished by employing alkylating agents under conditions known to skilled persons and chloro silanes in the presence of a base.

Fluorinated diketones I-1a, wherein Q¹ is CHF₂ or CF₃ and Q² is -CH₂-, can be obtained from methyl ketones I-4 through reaction with a suitable acylating agent in an organic solvent at temperatures in the range between 0 °C and 100 °C. Preferably the methyl ketone I-4 is reacted with a trifluoro acetate in the presence of sodium hydride in tetrahydrofurane or in the presence of a sodium alkoxide in methanol or ethanol at 0 °C to room temperature (see, for example, Tetrahedron 2014, 70(31), 4668-4674; Bioorg. Med. Chem. Lett. 2014, 24(6), 1581-1588). The corresponding difluoromethyl ketone can be prepared in a similar way (Journal of Fluorine Chemistry 2015, 178, 6-13).

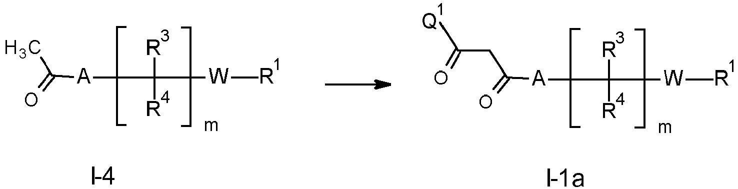

Compounds I-1b, wherein Q² is -CF₂-, can be prepared by reacting compounds I-1a with a fluorinating agent. Typically Selectfluor® (1-chloromethyl-4-fluor-1,4-diazoniabicyclo-[2.2.2]octanbis(tetrafluorborat)) is used as a fluorinating agent in MeCN as a solvent as described in WO 2012129384.

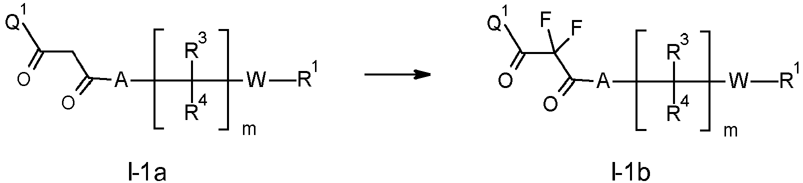

Treatment of compounds I-1b with hydroxylamine in the present of base leads to the formation of the hydroxyisoxazoline I-2b, wherein Q² is -CF₂-, in analogy with the synthesis of I-2a above.

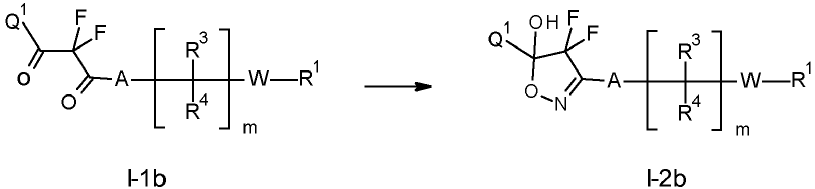

The preparation of compounds I, wherein m is 0, proceeds through diketones of the type I-1 c. Carboxylic acid derivatives of type I-6 can be used for the synthesis of diketones of the type I-1c. This transformation can be carried out in the presence of esters (X is -O-; as described, for example, in WO 2009/029632) or amides (X is -NR²-; see, for example, Bioorg. Med. Chem. 2006, 14(15), 5370-5383). The reaction can be carried out in water or in an organic solvent in the presence of a suitable base between 0 °C and 100 °C. In a typical procedure, an alkoxide is used in alcohol, or sodium hydride or sodium bis(trimethylsilyl)amide in tetrahydrofurane at 0 °C to room temperature.

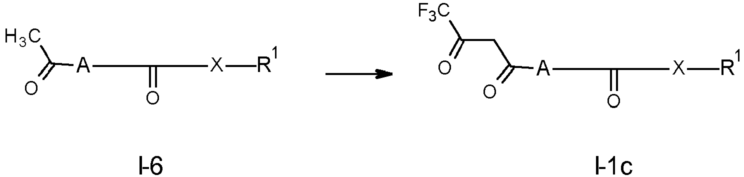

Starting from compounds I-1c the isoxazoles of formula I can be prepared as described above for compounds I-1a and I-1b. The skilled person will appreciate that also other carboxylic acid derivatives besides esters and amides can be used for this type of transfomation. The required acetophenones I-6 are either commercially available or they are readily accessible for a skilled person from commercially available starting materials.

Compounds of the formula I, wherein m is 0 and W is -S(O)ₚ-NR²-, can be prepared from sulphonic acid chlorides of the type I-7, which are either commercially available or readily accessible from commercially available starting materials. Hence, compounds I-7 can directly be treated with an amine HNR¹R² to result in sulphonamides I-8. This reaction may be carried out in an aprotic solvent in the presence of a base at a temperature between 0 °C and 100 °C. In a typical procedure the sulphonic acid chlorid is dissvolved in dichloromethane or tertahydrofurane and treated with triethylamine, diisopropyethylamine or pyridine at temperatures between 0 °C and room temperature before the amine is added (see, for example, WO 2012160447).

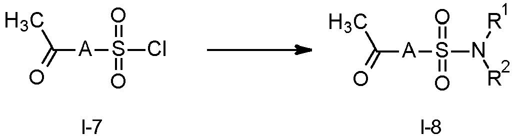

In case the sulphonic acid chloride is not commercially available, it can be synthesized starting from the corresponding sulfides as described for example in WO 2015151001 or in JP 4588121. Amines I-9 are either commercially available or can be synthesized by a person skilled in the art. Treatment of I-9 with acid halides, acid anhydrides, sulphonic acid halides, isocyanates or similar reactive carbonyl compounds in the presence of a base and in an organic solvent at 0 °C to 100 °C results in the formation of compounds I-10a, I-10b or I-10c, respectively. In a typical procedure, compound I-9 is treated with an anhydride or an acid chloride in dichloromethane or chloroform in the presence of triethylamine, diisopropylethylamine or pyridine at temperatures between 0 °C and room temperature as described, for example, in WO 2011153192, WO 2003022835, Asian Journal of Chemistry, 2012, 24(3), 1316-1318.

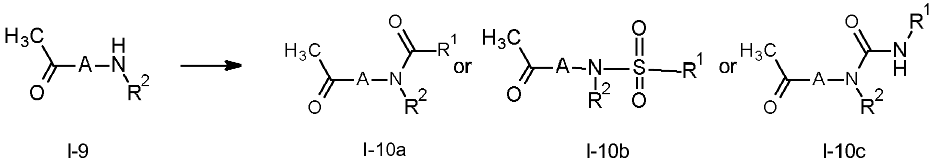

Methylketones I-8, I-10a, I-10b and I-10c can be converted to compounds of the formula I as described above.

Compounds of the formula I, wherein m is 1, are accessible starting from benzyl bromides of type I-13, which are either commercially available or can be prepared from the corresponding methyl ketones I-12 (WO 2009109999). Substitution of the bromine by an amine functionality leads to the amines of type I-14 (Journal of Medicinal Chemistry 2007 50(20), 4898-4908; WO 2005082859; JP 2011178724). Compounds I-14 can be further functionalized as described above.

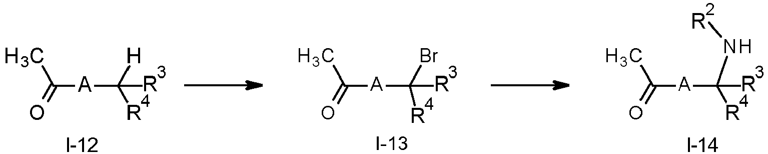

Methylketones I-14 can be transferred into the hydroxy isoxazolines of the formula I as described above. In some cases it may be advantageous to prepare the hydroxy isoxazoline moiety at an earlier stage. For this approach intermediates I-12 or I-13 can be treated with a suitable acylating agent as described for the synthesis of I-1a and I-1b.

A further strategy for the synthesis of compounds of the formula I, wherein m is 1, starts from aryl bromide I-15, which is either commercially available or can be prepared by means of standard procedures. These compounds and bromides of type I-17 can also be used in a sequence comprising transmetallation and Pd-catalyzed cross coupling, which leads to the corresponding aryl acetic acid derivative I-16 or I-18 as described, for example, in Journal of the American Chemical Society 2006 128(15), 4976-4985; Tetrahedron Letters 2003, 44(16), 3423-3426.

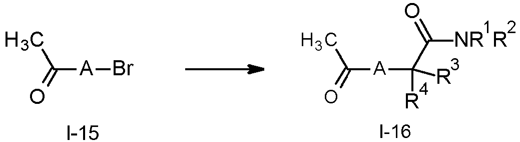

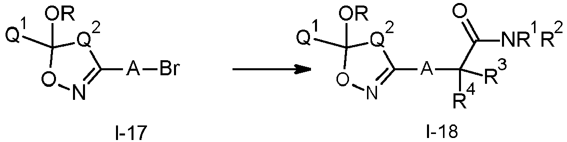

Carboxamide derivatives of the formula I can be transferred into the corresponding thiocarbonyl derivatives by reaction with Lawsson's reagent or other suphurizing agents under conditions that are known to a skilled person and which are described in literature (see, for example, WO 2014065413, WO 2011054871).

The compounds of the formula I or compositions comprising said compounds according to the invention and the mixtures comprising said compounds and compositions, respectively, are suitable as fungicides. They are distinguished by an outstanding effectiveness against a broad spectrum of phytopathogenic fungi, including soil-borne fungi, which derive especially from the following classes or are closely related to any of them: *Ascomycota (Ascomycetes),* for example, but not limited to the genus Cocholiobolus, Colletotrichum, Fusarium, Microdochium, Penicillium, Phoma, Magnaporte, Zymoseptoria, and Pseudocercosporella; *Basdiomycota (Basidiomycetes),* for example, but not limited to the genus Phakospora, Puccinia, Rhizoctonia, Sphacelotheca, Tilletia, Typhula, and Ustilago; *Chytridiomycota (Chytridiomycetes),* for example, but not limited to the genus Chytridiales, and Synchytrium; *Deuteromycetes* (syn. Fungi imperfecti), for example, but not limited to the genus *Ascochyta, Diplodia, Erysiphe, Fusarium, Phomopsis, and Pyrenophora; Peronosporomycetes* (syn. Oomycetes), for example but not limited to the genus Peronospora, Pythium, Phytophthora; *P*/*asmodiophoromycetes,* for example but not limited to the genus Plasmodiophora; *Zygomycetes,* for example, but not limited to the genus Rhizopus.

Some of the compounds of the formula I and the compositions according to the invention are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants. Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, to create mutations at a specific locus of a plant genome. Targeted mutagenesis techniques frequently use oligonucleotides or proteins like CRISPR/Cas, zinc-finger nucleases, TALENs or meganucleases to achieve the targeting effect. Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve a trait. These integrated genes are also referred to as transgenes in the art, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by mutagenesis and breeding comprise plant varieties commercially available under the name Clearfield®.

Herbicide tolerance has been created via the use of transgenes to glyphosate, glufosinate, 2,4-D, dicamba, oxynil herbicides, like bromoxynil and ioxynil, sulfonylurea herbicides, ALS inhibitors and 4-hydroxyphenylpyruvate dioxygenase (HPPD) inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621, goxv247; for tolerance to glufosinate: pat and bar, for tolerance to 2,4-D: aad-1, aad-12; for tolerance to dicamba: dmo; for tolerance to oxynil herbicies: bxn; for tolerance to sulfonylurea herbicides: zm-hra, csr1-2, gm-hra, S4-HrA; for tolerance to ALS inhibitors: csr1-2; and for tolerance to HPPD inhibitors: hppdPF, W336, avhppd-03.

Transgenic corn events comprising herbicide tolerance genes include, but are not limited to, DAS40278, MON801, MON802, MON809, MON810, MON832, MON87411, MON87419, MON87427, MON88017, MON89034, NK603, GA21, MZHG0JG, HCEM485, VCO-∅1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes include, but are not limited to, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHT∅H2, W62, W98, FG72 and CV127.

Transgenic cotton events comprising herbicide tolerance genes include, but are not limited to, 19-51a, 31707, 42317, 81910, 281-24-236, 3006-210-23, BXN 10211, BXN 10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants: Transgenes which have most frequently been used are toxin genes of *Bacillus* spp. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin, such as genes coding for protease inhibitors, like CpTI and pinll, have been transferred to other plants. A further approach uses transgenes such as dvsnf7 to produce double-stranded RNA in plants.

Transgenic corn events comprising genes for insecticidal proteins or double stranded RNA include, but are not limited to, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098. Transgenic soybean events comprising genes for insecticidal proteins include, but are not limited to, MON87701, MON87751 and DAS-81419. Transgenic cotton events comprising genes for insecticidal proteins include, but are not limited to, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by using the transgene athb17, being present for example in corn event MON87403, or by using the transgene bbx32, being present for example in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, such as drought, has been created by using the transgene cspB, comprised by the corn event MON87460 and by using the transgene Hahb-4, comprised by soybean event IND-∅∅41∅-5.

Traits are frequently combined by combining genes in a transformation event or by combining different events during the breeding process resulting in a cultivated plant with stacked traits.

Preferred combinations of traits are combinations of herbicide tolerance traits to different groups of herbicides, combinations of insect tolerance to different kind of insects, in particular tolerance to lepidopteran and coleopteran insects, combinations of herbicide tolerance with one or several types of insect resistance, combinations of herbicide tolerance with increased yield as well as combinations of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase). Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527; for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571; for potato events E12, F10, J3, J55, V11, X17, Y9 in WO14/178910, WO14/178913, WO14/178941, WO14/179276, WO16/183445, WO17/062831, WO17/062825; for rice events LLRICE06, LLRICE601, LLRICE62 in WO00/026345, WO00/026356, WO00/026345; and for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compounds I and compositions according to the invention, respectively, on cultivated plants may result in effects which are specific to a cultivated plant comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

The compounds I and compositions thereof, respectively, are particularly suitable for controlling the following plant diseases:
*Albugo* spp. (white rust) on ornamentals, vegetables (e. g. *A. candida)* and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solanior A. alternata*), tomatoes (e. g. *A. solani* or *A. alternata*) and wheat; *Aphanomyces* spp. on sugar beets and vegetables; *Ascochyta* spp. on cereals and vegetables, e. g. *A. tritici* (anthracnose) on wheat and *A. hordei* on barley; *Bipolaris* and *Drechslera* spp. (teleomorph: *Cochliobolus* spp.), e. g. Southern leaf blight (*D. maydis)* or Northern leaf blight (*B. zeicola*) on corn, e. g. spot blotch (*B. sorokiniana*) on cereals and e. g. *B. oryzae* on rice and turfs; *Blumeria* (formerly *Erysiphe) graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. u*/*mi*(Dutch elm disease) on elms; *Cercospora* spp. (Cercospora leaf spots) on corn (e. g. Gray leaf spot: *C. zeae-maydis),* rice, sugar beets (e. g. *C. beticola*), sugar cane, vegetables, coffee, soybeans (e. g. *C. sojina* or *C. kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (C. *carbonum),* cereals (e. g. *C. sativus,* anamorph: *B. sorokiniana*) and rice (e. g. *C. miyabeanus,* anamorph: *H*. *oryzae*); *Colletotrichum* (teleomorph: *Glomerella*) spp. (anthracnose) on cotton (e. g. *C*. *gossypii*), corn (e. g. *C. graminicola:* Anthracnose stalk rot), soft fruits, potatoes (e. g. *C*. *coccodes*: black dot), beans (e. g. *C. lindemuthianum*) and soybeans (e. g. *C. truncatum* or *C*. *gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri.* Black Foot Disease) and ornamentals; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D. tritici-repentis*: tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa*; *Elsinoe* spp. on pome fruits (*E. pyri*), soft fruits (*E. veneta*: anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella)* spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M. fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina)* and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans.* late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii*(orange rust) on sugar cane and *P. asparagi* on asparagus; *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer*(black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*); *Septoria* spp. on various plants, e. g. *S. glycines* (brown spot) on soybeans, *S. tritici*(*Septoria* blotch) on wheat and *S.* (syn. *Stagonospora*) nodorum (Stagonospora blotch) on cereals; *Uncinula* (syn. *Erysiphe*) *necator*(powdery mildew, anamorph: *Oidium tuckeri*) on vines; *Setospaeria* spp. (leaf blight) on corn (e. g. *S. turcicum,* syn. *Helminthosporium turcicum*) and turf; *Sphacelotheca* spp. (smut) on corn, (e. g. *S. reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae); Ustilago* spp. (loose smut) on cereals (e. g. *U. nuda and U. avaenae),* corn (e. g. *U. maydis*: corn smut) and sugar cane; *Venturia* spp. (scab) on apples (e. g. *V. inaequa*/*is*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

In a preferred embodiment the compounds I, their mixtures with other active compounds as defined herein and compositions thereof, respectively, are particularly suitable for controlling the following plant diseases: *Puccinia* spp. (rusts) on various plants, for example, but not limited to *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, and *Puccinia sorghi* (common rust) on maize, *Puccinia polysora* (southern rust) on maize; and *Phakopsoraceae* spp. on various plants, in particular *Phakopsora pachyrhizi* and *P. meibomiae* (soybean rust) on soybeans.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucorspp.,* and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting. The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof. Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof. Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids. Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants). Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.
ii) Dispersible concentrates (DC)
   5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.
iii) Emulsifiable concentrates (EC)
   15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.
iv) Emulsions (EW, EO, ES)
   5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.
vi) Water-dispersible granules and water-soluble granules (WG, SG)
   50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e. g. finely divided kaolin) ad 100 wt%.
xii) Granules (GR, FG)
   0.5-30 wt% of a compound I is ground finely and associated with solid carrier (e. g. silicate) ad 100 wt%. Granulation is achieved by extrusion, spray-drying or fluidized bed.
xiii) Ultra-low volume liquids (UL)
   1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, more preferably between 1 and 70%, and in particular between 10 and 60%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21a), methyl-*N*-[2-[(1,4-dimethyl-5-phenyl-pyrazol-3-yl)oxylmethyl]phenyl]-*N-*methoxy-carbamate (A.1.22), metyltetrapole (A.1.25), (*Z,*2*E*)-5-[1-(2,4-dichlorophenyl)pyrazol-3-yl]-oxy-2-methoxyimino-*N*,3-dimethyl-pent-3-enamide (A.1.34), (*Z,2E*)-5-[1-(4-chlorophenyl)pyrazol-3-yl]oxy-2-methoxyimino-*N,*3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoro¬methyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)pyridine-3-carboxamide (A.3.32), 2-(difluoromethyl)-*N-*[(3*R*)-1,1,3-trimethylindan-4-yl]pyridine-3-carboxamide (A.3.33), 2-(difluoromethyl)-*N-*(3-ethyl-1,1-dimethyl-indan-4-yl)-pyridine-3-carboxamide (A.3.34), 2-(difluoromethyl)-*N*-[(3*R*)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3*R*)-3-isobutyl-1,1-dimethyl-indan-4-yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), ipfentrifluconazole (B.1.37), mefentrifluconazole (B.1.38), 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N-*ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), 2-{3-[2-(1-{[3,5-bis(difluoromethyl-1*H*-pyrazol-1-yl]acetyl}piperidin-4-yl)-1,3-thiazol-4-yl]-4,5-dihydro-1,2-oxazol-5-yl}-phenyl methanesulfonate (G.5.2), 2-{3-[2-(1-{[3,5-bis(difluoromethyl)-1*H*-pyrazol-1-yl]-acetyl}piperidin-4-yl) 1,3-thiazol-4-yl]-4,5-dihydro-1,2-oxazol-5-yl}-3-chlorophenyl methanesulfonate (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N-*tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifiuoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (1.1.2);
   - melanin synthesis inhibitors: pyroquilon (1.2.1), tricyclazole (I.2.2), carpropamid (1.2.3), dicyclomet (I.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N-*(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), metha-sulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), *N'*-(4-(4-chloro-3-trifluoromethyl-phenoxy)-2,5-dimethyl-phenyl)-*N-*ethyl-*N-*methyl formamidine (K.1.27), *N'*-(4-(4-fluoro-3-trifluoromethyl-phenoxy)-2,5-dimethyl-phenyl)-*N-*ethyl-*N*-methyl formamidine (K.1.28), *N'*-[4-[[3-[(4-chlorophenyl)methyl]-1,2,4-thiadiazol-5-yl]-oxy]-2,5-dimethyl-phenyl]-*N*-ethyl-*N*-methyl-formamidine (K.1.29), *N'*-(5-bromo-6-indan-2-yl-oxy-2-methyl-3-pyridyl)-*N*-ethyl-*N*-methyl-formamidine (K.1.30), *N'-*[5-bromo-6-[1-(3,5-difluorophenyl)ethoxy]-2-methyl-3-pyridyl]-*N-*ethyl-*N-*methyl-formamidine (K.1.31), *N'-*[5-bromo-6-(4-isopropylcyclohexoxy)-2-methyl-3-pyridyl]-*N-*ethyl-*N-*methyl-formamidine (K.1.32), *N'*-[5-bromo-2-methyl-6-(1-phenylethoxy)-3-pyridyl]-*N*-ethyl-*N*-methyl-formamidine (K.1.33), *N'*-(2-methyl-5-trifluoromethyl-4-(3-trimethylsilanyl-propoxy)-phenyl)-*N*-ethyl-*N*-methyl formamidine (K.1.34), *N'*-(5-difluoromethyl-2-methyl-4-(3-trimethylsilanyl-propoxy)-phenyl)-*N-*ethyl-*N-*methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1-(4,6-dimethoxy-pyrimidin-2-yl)-2-methyl-1*H-*benzoimidazole (K.1.39), ethyl (2)-3-amino-2-cyano-3-phenyl-prop-2-enoate (K.1.40), picarbutrazox (K.1.41), pentyl *N-*[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N'*-(2,5-dimethyl-4-phenoxy-phenyl)-*N-*ethyl-*N*-methyl-formamidine (K.1.53), pyrifenamine (K.1.54);
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat, mepiquat chloride, naphthaleneacetic acid, *N-*6-benzyladenine, paclobutrazol, prohexadione, prohexadione-calcium, prohydrojasmon, thidiazuron, triapenthenol, tributyl phosphorotrithioate, 2,3,5-tri-iodobenzoic acid, trinexapac-ethyl, uniconazole;
N) Herbicides from classes N.1 to N.15
   N.1 Lipid biosynthesis inhibitors: alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-chloro-4-cyclo¬propyl-2'-fluoro[1,1'-biphenyl]-3-yl)-5-hydroxy-2,2,6,6-tetramethyl-2*H-*pyran-3(6*H*-one (1312337-72-6); 4-(2',4'-dichloro-4-cyclopropyl[1,1'-biphenyl]-3-yl)-5-hydroxy-2,2,6,6-tetramethyl-2*H*-pyran-3(6*H*-one (1312337-45-3); 4-(4'-chloro-4-ethyl-2'-fluoro[1,1-'-biphenyl]-3-yl)-5-hydroxy-2,2,6,6-tetramethyl-2*H-*pyran 3(6*H*)-one (1033757-93-5); 4-(2',4'-dichloro-4-ethyl[1,1'-biphenyl]-3-yl)-2,2,6,6-tetramethyl-2*H-*pyran-3,5(4*H*,6*H*)-dione (1312340-84-3); 5-(acetyloxy)-4-(4'-chloro-4-cyclopropyl-2'-fluoro[1,1'-biphenyl]-3-yl)-3,6-dihydro-2,2,6,6-tetramethyl-2*H*-pyran-3-one (1312337-48-6); 5-(acetyloxy)-4-(2',4'-dichloro-4-cyclopropyl-[1,1'-biphenyl]-3-yl)-3,6-dihydro-2,2,6,6-tetramethyl-2*H-*pyran-3-one; 5-(acetyloxy)-4-(4'-chloro-4-ethyl-2'-fluoro[1,1'-biphenyl]-3-yl)-3,6-dihydro-2,2,6,6-tetramethyl-2*H*-pyran-3-one (1312340-82-1); 5-(acetyloxy)-4-(2',4'-dichloro-4-ethyl[1,1'-biphenyl]-3-yl)-3,6-dihydro-2,2,6,6-tetramethyl-2*H-*pyran-3-one (1033760-55-2);4-(4'-chloro-4-cyclopropyl-2'-fluoro[1,1'-biphenyl]-3-yl)-5,6-dihydro-2,2,6,6-tetramethyl-5-oxo-2*H*-pyran-3-yl carbonic acid methyl ester (1312337-51-1); 4-(2',4'-dichloro-4-cyclopropyl- [1,1'-biphenyl]-3-yl)-5,6-dihydro-2,2,6,6-tetramethyl-5-oxo-2*H*-pyran-3-yl carbonic acid methyl ester; 4-(4'-chloro-4-ethyl-2'-fluoro[1,1'-biphenyl]-3-yl)-5,6-dihydro-2,2,6,6-tetramethyl-5-oxo-2*H*-pyran-3-yl carbonic acid methyl ester (1312340-83-2); 4-(2',4'-dichloro-4-ethyl-[1,1'-biphenyl]-3-yl)-5,6-dihydro-2,2,6,6-tetramethyl-5-oxo-2*H*-pyran-3-yl carbonic acid methyl ester (1033760-58-5); benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate, vernolate;
   N.2 ALS inhibitors: amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethamet-sulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl, tritosulfuron, imazamethabenz, imazamethabenz-methyl, imazamox, imazapic, imazapyr, imazaquin, imazethapyr; cloransulam, cloransulam-methyl, diclosulam, flumetsulam, florasulam, metosulam, penoxsulam, pyrimisulfan, pyroxsulam; bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methyl¬ethyl ester (420138-41-6), 4-[[[2-[(4,6-dimethoxy-2-pyrimidinyl)oxy]-phenyl]¬methyl]amino]-benzoic acid propyl ester (420138-40-5), *N*-(4-bromophenyl)-2-[(4,6-dimethoxy-2-pyrimidinyl)oxy]benzenemethanamine (420138-01-8); flucarbazone, flucarbazone-sodium, propoxycarbazone, propoxycarbazone-sodium, thiencarbazone, thiencarbazone-methyl; triafamone;
   N.3 Photosynthesis inhibitors: amicarbazone; chlorotriazine; ametryn, atrazine, chloridazone, cyanazine, desmetryn, dimethametryn,hexazinone, metribuzin, prometon, prometryn, propazine, simazine, simetryn, terbumeton, terbuthylazin, terbutryn, trietazin; chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron, thiadiazuron, desmedipham, karbutilat, phenmedipham, phenmediphamethyl, bromofenoxim, bromoxynil and its salts and esters, ioxynil and its salts and esters, bromacil, lenacil, terbacil, bentazon, bentazon-sodium, pyridate, pyridafol, pentanochlor, propanil; diquat, diquat-dibromide, paraquat, paraquat-dichloride, paraquat-dimetilsulfate;
   N.4 protoporphyrinogen-IX oxidase inhibitors: acifluorfen, acifluorfen-sodium, azafenidin, bencarbazone, benzfendizone, bifenox, butafenacil, carfentrazone, carfentrazone-ethyl, chlormethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (353292-31-6), *N-*ethyl-3-(2,6-dichloro-4-trifluoro-methylphenoxy)-5-methyl-1*H-*pyrazole-1-carboxamide (452098-92-9), *N*-tetrahydrofurfuryl-3-(2,6-dichloro-4-trifluoromethylphenoxy)-5-methyl-1*H*-pyrazole-1-carboxamide (915396-43-9), *N*-ethyl-3-(2-chloro-6-fluoro-4-trifluoromethyl¬phenoxy)-5-methyl-1*H-*pyrazole-1-carboxamide (452099-05-7), *N*-tetrahydro¬furfuryl-3-(2-chloro-6-fluoro-4-trifluoro¬methylphenoxy)-5-methyl-1*H-*pyrazole-1-carboxamide (452100-03-7), 3-[7-fluoro-3-oxo-4-(prop-2-ynyl)-3,4-dihydro-2*H*-benzo[1,4]oxazin-6-yl]-1,5-dimethyl-6-thioxo-[1,3,5]triazinan-2,4-dione (451484-50-7), 2-(2,2,7-trifluoro-3-oxo-4-prop-2-ynyl-3,4-dihydro-2*H-*benzo[1,4]oxazin-6-yl)-4,5,6,7-tetrahydro-isoindole-1,3-dione (1300118-96-0), 1-methyl-6-trifluoro¬methyl-3-(2,2,7-trifluoro-3-oxo-4-prop-2-ynyl-3,4-dihydro-2*H-*benzo[1,4]oxazin-6-yl)-1*H-*pyrimidine-2,4-dione (1304113-05-0), methyl (*E*)-4-[2-chloro-5-[4-chloro-5-(difluoromethoxy)-1*H*-methylpyrazol-3-yl]-4-fluoro-phenoxy]-3-methoxy-but-2-enoate (948893-00-3), 3-[7-chloro-5-fluoro-2-(trifluoromethyl)-1*H-*benzimidazol-4-yl]-1-methyl-6-(trifluoromethyl)-1*H-*pyrimidine-2,4-dione (212754-02-4);
   N.5 Bleacher herbicides: beflubutamid, diflufenican, fluridone, flurochloridone, flurtamone, norflurazon, picolinafen, 4-(3-trifluoromethyl-phenoxy)-2-(4-trifluoromethylphenyl)pyrimidine (180608-33-7); benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone; aclonifen, amitrole, flumeturon;
   N.6 EPSP synthase inhibitors: glyphosate, glyphosate-isopropylammonium, glyposate-potassium, glyphosate-trimesium (sulfosate);
   N.7 Glutamine synthase inhibitors: bilanaphos (bialaphos), bilanaphos-sodium, glufosinate, glufosinate-P, glufosinate-ammonium;
   N.8 DHP synthase inhibitors: asulam;
   N.9 Mitosis inhibitors: benfluralin, butralin, dinitramine, ethalfluralin, fluchloralin, oryzalin, pendimethalin, prodiamine, trifluralin; amiprophos, amiprophos-methyl, butamiphos; chlorthal, chlorthal-dimethyl, dithiopyr, thiazopyr, propyzamide, tebutam; carbetamide, chlorpropham, flamprop, flamprop-isopropyl, flamprop-methyl, flamprop-M-isopropyl, flamprop-M-methyl, propham;
   N.10 VLCFA inhibitors: acetochlor, alachlor, butachlor, dimethachlor, dimethenamid, dimethenamid-P, metazachlor, metolachlor, metolachlor-S, pethoxamid, pretilachlor, propachlor, propisochlor, thenylchlor, flufenacet, mefenacet, diphenamid, naproanilide, napropamide, napropamide-M, fentrazamide, anilofos, cafenstrole, fenoxasulfone, ipfencarbazone, piperophos, pyroxasulfone, isoxazoline compounds of the formulae II.1, II.2, II.3, II.4, II.5, II.6, II.7, II.8 and II.9 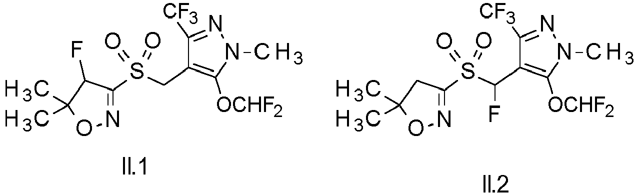 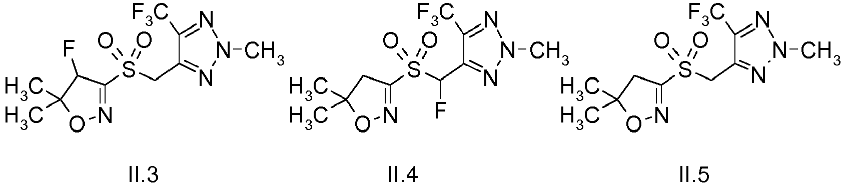 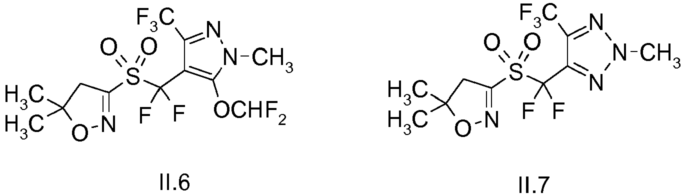 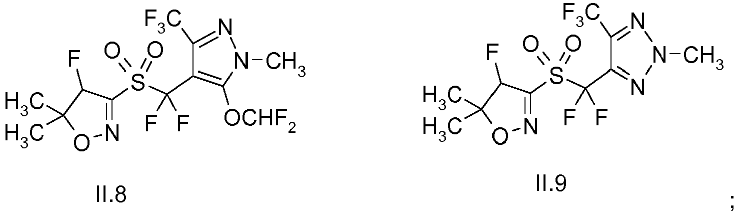
   N.11 Cellulose biosynthesis inhibitors: chlorthiamid, dichlobenil, flupoxam, indaziflam, isoxaben, triaziflam, 1-cyclohexyl-5-pentafluorphenyloxy-14-[1,2,4,6]thiatriazin-3-ylamine (175899-01-1);
   N.12 Decoupler herbicides: dinoseb, dinoterb, DNOC and its salts;
   N.13 Auxinic herbicides: 2,4-D and its salts and esters, clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopy-ralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters (943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 4-amino-3-chloro-6-(4-chloro-2-fluoro-3-methoxyphenyl)-5-fluoropyridine-2-carboxylic acid, benzyl 4-amino-3-chloro-6-(4-chloro-2-fluoro-3-methoxyphenyl)-5-fluoropyridine-2-carboxylate (1390661-72-9);
   N.14 Auxin transport inhibitors: diflufenzopyr, diflufenzopyr-sodium, naptalam, naptalam-sodium;
   N.15 Other herbicides: bromobutide, chlorflurenol, chlorflurenol-methyl, cinmethylin, cumyluron, cyclopyrimorate (499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (403640-27-7), methyl azide, methyl bromide, methyl-dymron, methyl iodide, MSMA, oleic acid, oxaziclomefone, pelargonic acid, pyributicarb, quinoclamine, tridiphane;
O) Insecticides from classes O.1 to O.29
   O.1 Acetylcholine esterase (AChE) inhibitors: aldicarb (O.1.1), alanycarb (O.1.2), bendiocarb (O.1.3), benfuracarb (O.1.4), butocarboxim (0.1.5), butoxycarboxim (O.1.6), carbaryl (O.1.7), carbofuran (O.1.8), carbosulfan (O.1.9), ethiofencarb (O.1.10), fenobucarb (O.1.11), formetanate (O.1.12), furathiocarb (O.1.13), isoprocarb (O.1.14), methiocarb (O.1.15), methomyl (O.1.16), metolcarb (O.1.17), oxamyl (O.1.18), pirimicarb (O.1.19), propoxur (O.1.20), thiodicarb (O.1.21), thiofanox (O.1.22), trimethacarb (O.1.23), XMC (O.1.24), xylylcarb (O.1.25), triazamate (O.1.26), acephate (O.1.27), azamethiphos (0.1.28), azinphos-ethyl (O.1.29), azinphosmethyl (O.1.30), cadusafos (O.1.31), chlorethoxyfos (O.1.32), chlorfenvinphos (O.1.33), chlormephos (O.1.34), chlorpyrifos (O.1.35), chlorpyrifos-methyl (O.1.36), coumaphos (O.1.37), cyanophos (O.1.38), demeton-S-methyl (O.1.39), diazinon (O.1.40), dichlorvos/ DDVP (O.1.41), dicrotophos (O.1.42), dimethoate (O.1.43), dimethylvinphos (O.1.44), disulfoton (O.1.45), EPN (O.1.46), ethion (O.1.47), ethoprophos (O.1.48), famphur (O.1.49), fenamiphos (O.1.50), fenitrothion (O.1.51), fenthion (O.1.52), fosthiazate (O.1.53), heptenophos (O.1.54), imicyafos (O.1.55), isofenphos (O.1.56), isopropyl O-(methoxyaminothio-phosphoryl) salicylate (O.1.57), isoxathion (O.1.58), malathion (O.1.59), mecarbam (O.1.60), methamidophos (O.1.61), methidathion (O.1.62), mevinphos (O.1.63), monocrotophos (O.1.64), naled (O.1.65), omethoate (O.1.66), oxydemeton-methyl (O.1.67), parathion (O.1.68), parathion-methyl (O.1.69), phenthoate (O.1.70), phorate (O.1.71), phosalone (O.1.72), phosmet (O.1.73), phosphamidon (O.1.74), phoxim (O.1.75), pirimiphos- methyl (O.1.76), profenofos (O.1.77), propetamphos (O.1.78), prothiofos (O.1.79), pyraclofos (O.1.80), pyridaphenthion (O.1.81), quinalphos (O.1.82), sulfotep (O.1.83), tebupirimfos (O.1.84), temephos (O.1.85), terbufos (O.1.86), tetrachlorvinphos (O.1.87), thiometon (O.1.88), triazophos (O.1.89), trichlorfon (O.1.90), vamidothion (O.1.91);
   O.2 GABA-gated chloride channel antagonists: endosulfan (O.2.1), chlordane (O.2.2), ethiprole (O.2.3), fipronil (O.2.4), flufiprole (O.2.5), pyrafluprole (O.2.6), pyriprole (O.2.7);
   O.3 Sodium channel modulators: acrinathrin (O.3.1), allethrin (O.3.2), d-cis-trans allethrin (O.3.3), d-trans allethrin (O.3.4), bifenthrin (O.3.5), kappa-bifenthrin (O.3.6), bioallethrin (O.3.7), bioallethrin S-cylclopentenyl (O.3.8), bioresmethrin (O.3.9), cycloprothrin (O.3.10), cyfluthrin (O.3.11), beta-cyfluthrin (O.3.12), cyhalothrin (O.3.13), lambda-cyhalothrin (O.3.14), gamma-cyhalothrin (O.3.15), cypermethrin (O.3.16), alpha-cypermethrin (O.3.17), beta-cypermethrin (O.3.18), theta-cypermethrin (O.3.19), zeta-cypermethrin (O.3.20), cyphenothrin (O.3.21), deltamethrin (O.3.22), empenthrin (O.3.23), esfenvalerate (O.3.24), etofenprox (O.3.25), fenpropathrin (O.3.26), fenvalerate (O.3.27), flucythrinate (O.3.28), flumethrin (O.3.29), tau-fluvalinate (O.3.30), halfenprox (O.3.31), heptafluthrin (O.3.32), imiprothrin (O.3.33), meperfluthrin (O.3.34), metofluthrin (O.3.35), momfluorothrin (O.3.36), epsilon-momfluorothrin (O.3.37), permethrin (O.3.38), phenothrin (O.3.39), prallethrin (O.3.40), profluthrin (O.3.41), pyrethrin (pyrethrum) (O.3.42), resmethrin (O.3.43), silafluofen (O.3.44), tefluthrin (O.3.45), kappa-tefluthrin (O.3.46), tetramethylfluthrin (O.3.47), tetramethrin (O.3.48), tralomethrin (O.3.49), transfluthrin (O.3.50), DDT (O.3.51), methoxychlor (O.3.52);
   O.4 Nicotinic acetylcholine receptor agonists (nAChR): acetamiprid (0.4.1), clothianidin (O.4.2), cycloxaprid (O.4.3), dinotefuran (O.44), imidacloprid (O.4.5), nitenpyram (O.4.6), thiacloprid (O.4.7), thiamethoxam (O.4.8), 4,5-dihydro-*N-*nitro-1-(2-oxiranylmethyl)-1*H*imidazol-2-amine (O.4.9), (2*E*)-1-[(6-chloropyridin-3-yl)methyl]-*N*'-nitro-2-pentylidene-hydrazinecarboximidamide (O.4.10), 1-[(6-chloropyridin-3-yl)methyl]-7-methyl-8-nitro-5-propoxy-1,2,3,5,6,7-hexahydroimidazo[1,2-a]pyridine (O.4.11), nicotine (O.4.12), sulfoxaflor (O.4.13), flupyradifurone (O.4.14), triflumezopyrim (O.4.15);
   O.5 Nicotinic acetylcholine receptor allosteric activators: spinosad (O.5.1), spinetoram (O.5.2);
   O.6 Chloride channel activators: abamectin (O.6.1), emamectin benzoate (O.6.2), ivermectin (O.6.3), lepimectin (O.6.4), milbemectin (O.6.5);
   O.7 Juvenile hormone mimics: hydroprene (0.7.1), kinoprene (O.7.2), methoprene (O.7.3), fenoxycarb (O.7.4), pyriproxyfen (O.7.5);
   O.8 miscellaneous non-specific (multi-site) inhibitors: methyl bromide (O.8.1) and other alkyl halides, chloropicrin (O.8.2), sulfuryl fluoride (O.8.3), borax (O.8.4), tartar emetic (O.8.5);
   O.9 Chordotonal organ TRPV channel modulators: pymetrozine (O.9.1), pyrifluquinazon (O.9.2), flonicamid (O.9.3);
   O.10 Mite growth inhibitors: clofentezine (O.10.1), hexythiazox (O.10.2), diflovidazin (O.10.3), etoxazole (O.10.4);
   O.11 Microbial disruptors of insect midgut membranes: *Bacillus thuringiensis, Bacillus sphaericus* and the insecticdal proteins they produce: *Bacillus thuringiensis* subsp. *Israelensis* (O.11.1), *Bacillus sphaericus* (O.11.2), *Bacillus thuringiensis* subsp. *aizawai* (O.11.3), *Bacillus thuringiensis* subsp. *kurstaki* (O.11.4), *Bacillus thuringiensis* subsp. *tenebrionis* (O.11.5), the Bt crop proteins: Cry1Ab (O.11.6), Cry1Ac (O.11.7), Cry1Fa (O.11.8), Cry2Ab (O.11.9), mCry3A (O.11.10), Cry3Ab (O.11.11), Cry3Bb (O.11.12), Cry34/35Ab1 (O.11.13);
   O.12 Inhibitors of mitochondrial ATP synthase: diafenthiuron (O.12.1), azocyclotin (O.12.2), cyhexatin (O.12.3), fenbutatin oxide (O.12.4), propargite (O.12.5), tetradifon (O.12.6);
   O.13 Uncouplers of oxidative phosphorylation via disruption of the proton gradient: chlorfenapyr (O.13.1), DNOC (O.13.2), sulfluramid (O.13.3);
   O.14 Nicotinic acetylcholine receptor (nAChR) channel blockers: bensultap (O.14.1), cartap hydrochloride (O.14.2), thiocyclam (O.14.3), thiosultap sodium (O.14.4);
   O.15 Inhibitors of the chitin biosynthesis type 0: bistrifluron (O.15.1), chlorfluazuron (O.15.2), diflubenzuron (O.15.3), flucycloxuron (O.15.4), flufenoxuron (O.15.5), hexaflumuron (O.15.6), lufenuron (O.15.7), novaluron (O.15.8), noviflumuron (O.15.9), teflubenzuron (O.15.10), triflumuron (O.15.11);
   O.16 Inhibitors of the chitin biosynthesis type 1: buprofezin (O.16.1);
   O.17 Moulting disruptors: cyromazine (O.17.1);
   O.18 Ecdyson receptor agonists: methoxyfenozide (O.18.1), tebufenozide (O.18.2), halofenozide (O.18.3), fufenozide (O.18.4), chromafenozide (O.18.5);
   O.19 Octopamin receptor agonists: amitraz (O.19.1);
   O.20 Mitochondrial complex III electron transport inhibitors: hydramethylnon (O.20.1), acequinocyl (O.20.2), fluacrypyrim (O.20.3), bifenazate (O.20.4);
   O.21 Mitochondrial complex I electron transport inhibitors: fenazaquin (O.21.1), fenpyroximate (O.21.2), pyrimidifen (O.21.3), pyridaben (O.21.4), tebufenpyrad (O.21.5), tolfenpyrad (O.21.6), rotenone (O.21.7);
   O.22 Voltage-dependent sodium channel blockers: indoxacarb (O.22.1), metaflumizonev (O.22.2), 2-[2-(4-cyanophenyl)-1-[3-(trifluoromethyl)phenyl]ethylidene]-*N-*[4-(difluoromethoxy)phenyl]-hydrazinecarboxamide (O.22.3), *N-*(3-chloro-2-methylphenyl)-2-[(4-chlorophenyl)-[4-[methyl(methylsulfonyl)amino]phenyl]methylene]-hydrazinecarboxamide (O.22.4);
   O.23 Inhibitors of the of acetyl CoA carboxylase: spirodiclofen (O.23.1), spiromesifen (O.23.2), spirotetramat (O.23.3), spiropidion (O.23.4);
   O.24 Mitochondrial complex IV electron transport inhibitors: aluminium phosphide (O.24.1), calcium phosphide (O.24.2), phosphine (O.24.3), zinc phosphide (O.24.4), cyanide (O.24.5);
   O.25 Mitochondrial complex II electron transport inhibitors: cyenopyrafen (O.25.1), cyflumetofen (O.25.2);
   O.26 Ryanodine receptor-modulators: flubendiamide (0.26.1), chlorantraniliprole (O.26.2), cyantraniliprole (O.26.3), cyclaniliprole (O.26.4), tetraniliprole (O.26.5), (*R*)-3-chloro-*N*¹-{2-methyl-4-[1,2,2,2-tetrafluoro-1-(trifluoromethyl)ethyl]phenyl}-*N*²-(1-methyl-2-methylsulfonylethyl)phthalamide (O.26.6), (*S*)-3-chloro-*N*¹-{2-methyl-4-[1,2,2,2-tetrafluoro-1-(trifluoromethyl)ethyl]phenyl}-*N*²-(1-methyl-2-methylsulfonylethyl)phthalamide (O.26.7), methyl-2-[3,5-dibromo-2-({[3-bromo-1-(3-chloropyridin-2-yl)-1*H*pyrazol-5-yl]carbonyl}-amino)benzoyl]-1,2-dimethylhydrazinecarboxylate (O.26.8), *N-*[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide (O.26.9), *N-*[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide (O.26.10), *N*-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide (O.26.11), *N-*[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide (O.26.12), *N*-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide (O.26.13), *N-*[2-(5-amino-1,3,4-thiadiazol-2-yl)-4-chloro-6-methylphenyl]-3-bromo-1-(3-chloro-2-pyridinyl)-1*H-*pyrazole-5-carboxamide (O.26.14), 3-chloro-1-(3-chloro-2-pyridinyl)-*N-*[2,4-dichloro-6-[[(1-cyano-1-methylethyl)amino]carbonyl]phenyl]-1*H-*pyrazole-5-carboxamide (O.26.15), 3-bromo-*N-*[2,4-dichloro-6-(methylcarbamoyl)phenyl]-1-(3,5-dichloro-2-pyridyl)-1*H-*pyrazole-5-carboxamide (O.26.16), *N-*[4-chloro-2-[[(1,1-dimethylethyl)amino]carbonyl]-6-methylphenyl]-1-(3-chloro-2-pyridinyl)-3-(fluoromethoxy)-1*H-*pyrazole-5-carboxamide (O.26.17), cyhalodiamide (O.26.18);
   O.27: Chordotonal organ Modulators - undefined target site: flonicamid (O.27.1);
   O.28. insecticidal active compounds of unknown or uncertain mode of action: afidopyropen (O.28.1), afoxolaner (O.28.2), azadirachtin (O.28.3), amidoflumet (O.28.4), benzoximate (O.28.5), broflanilide (O.28.6), bromopropylate (O.28.7), chinomethionat (O.28.8), cryolite (O.28.9), dicloromezotiaz (O.28.10), dicofol (O.28.11), flufenerim (O.28.12), flometoquin (O.28.13), fluensulfone (O.28.14), fluhexafon (O.28.15), fluopyram (O.28.16), fluralaner (O.28.17), metoxadiazone (O.28.18), piperonyl butoxide (0.28.19), pyflubumide (O.28.20), pyridalyl (O.28.21), tioxazafen (O.28.22), 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1*H*-1,2,4-triazole-5-amine (O.28.23), *Bacillus firmus* I-1582 (O.28.24), flupyrimin (O.28.25), fluazaindolizine (O.28.26), 4-[5-(3,5-dichlorophenyl)-5-(trifluoromethyl)-4*H-*isoxazol-3-yl]-2-methyl-*N-*(1-oxothietan-3-yl)benzamide (O.28.27), fluxametamide (O.28.28), 5-[3-[2,6-dichloro-4-(3,3-dichloroallyloxy)phenoxy]propoxy]-1*H-*pyrazole (O.28.1), 4-cyano-*N-*[2-cyano-5-[[2,6-dibromo-4-[1,2,2,3,3,3-hexafluoro-1-(trifluoromethyl)propyl]phenyl]carbamoyl]phenyl]-2-methyl-benzamide (O.28.29), 4-cyano-3-[(4-cyano-2-methyl-benzoyl)amino]-*N-*[2,6-dichloro-4-[1,2,2,3,3,3-hexafluoro-1-(trifluoromethyl)propyl]phenyl]-2-fluoro-benzamide (O.28.30), *N-*[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide (0.28.31), *N-*[5-[[2-bromo-6-chloro-4-[2,2,2-tri-fluoro-1-hydroxy-1-(trifluoromethyl)ethyl]phenyl]carbamoyl]-2-cyano-phenyl]-4-cyano-2-methyl-benzamide (O.28.32), *N-*[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide (O.28.33), 4-cyano-*N*-[2-cyano-5-[[2,6-dichloro-4-[1,2,2,3,3,3-hexafluoro-1-(trifluoromethyl)propyl]phenyl]-carbamoyl]phenyl]-2-methyl-benzamide (O.28.34), 4-cyano-*N-*[2-cyano-5-[[2,6-dichloro-4-[1,2,2,2-tetrafluoro-1-(trifluoromethyl)ethyl]phenyl]carbamoyl]phenyl]-2-methyl-benzamide (O.28.35), *N-*[5-[[2-bromo-6-chloro-4-[1,2,2,2-tetrafluoro-1-(trifluoromethyl)ethyl]phenyl]-carbamoyl]-2-cyano-phenyl]-4-cyano-2-methyl-benzamide (O.28.36); 2-(1,3-dioxan-2-yl)-6-[2-(3-pyridinyl)-5-thiazolyl]-pyridine (O.28.37), 2-[6-[2-(5-fluoro-3-pyridinyl)-5-thiazolyl]-2-pyridinyl]-pyrimidine (O.28.38), 2-[6-[2-(3-pyridinyl)-5-thiazolyl]-2-pyridinyl]-pyrimidine (O.28.39), *N-*methylsulfonyl-6-[2-(3-pyridyl)thiazol-5-yl]pyridine-2-carboxamide (O.28.40), *N-*methylsulfonyl-6-[2-(3-pyridyl)thiazol-5-yl]pyridine-2-carboxamide (O.28.41), 1-[(6-chloro-3-pyridinyl)methyl]-1,2,3,5,6,7-hexahydro-5-methoxy-7-methyl-8-nitro-imidazo[1,2-a]pyridine (O.28.42), 1-[(6-chloropyridin-3-yl)methyl]-7-methyl-8-nitro-1,2,3,5,6,7-hexahydroimidazo[1,2-a]pyridin-5-ol (O.28.43), 1-isopropyl-*N*,5-dimethyl-*N-*pyridazin-4-yl-pyrazole-4-carboxamide (O.28.44), 1-(1,2-dimethylpropyl)-*N-*ethyl-5-methyl-*N-*pyridazin-4-yl-pyrazole-4-carboxamide (O.28.45), *N*,5-dimethyl-*N-*pyridazin-4-yl-1-(2,2,2-trifluoro-1-methyl-ethyl)pyrazole-4-carboxamide (O.28.46), 1-[1-(1-cyanocyclopropyl)ethyl]-*N-*ethyl-5-methyl-*N-*pyridazin-4-yl-pyrazole-4-carboxamide (O.28.47), *N-*ethyl-1-(2-fluoro-1-methyl-propyl)-5-meth-yl-*N-*pyridazin-4-yl-pyrazole-4-carboxamide (O.28.48), 1-(1,2-dimethylpropyl)-*N*,5-dimethyl-*N*-pyridazin-4-yl-pyrazole-4-carboxamide (O.28.49), 1-[1-(1-cyanocyclopropyl)ethyl]-*N*,5-dimethyl-*N-*pyridazin-4-yl-pyrazole-4-carboxamide (O.28.50), *N-*methyl-1-(2-fluoro-1-methylpropyl]-5-methyl-*N-*pyridazin-4-yl-pyrazole-4-carboxamide (O.28.51), 1-(4,4-difluorocyclohexyl)-*N-*ethyl-5-methyl-*N-*pyridazin-4-yl-pyrazole-4-carboxamide (O.28.52), 1-(4,4-difluorocyclohexyl)-*N*,5-dimethyl-*N*-pyridazin-4-yl-pyrazole-4-carboxamide (O.28.53), *N-*(1-methylethyl)-2-(3-pyridinyl)-2*H-*indazole-4-carboxamide (O.28.54), *N-*cyclopropyl-2-(3-pyridinyl)-2*H-*indazole-4-carboxamide (O.28.55), *N-*cyclohexyl-2-(3-pyridinyl)-2*H-*indazole-4-carboxamide (O.28.56), 2-(3-pyridinyl)-*N-*(2,2,2-trifluoroethyl)-2*H*-indazole-4-carboxamide (O.28.57), 2-(3-pyridinyl)-*N-*[(tetrahydro-2-furanyl)methyl]-2*H-*indazole-5-carboxamide (O.28.58), methyl 2-[[2-(3-pyridinyl)-2*H-*indazol-5-yl]carbonyl]hydrazinecarboxylate (O.28.59), *N*-[(2,2-difluorocyclopropyl)methyl]-2-(3-pyridinyl)-2*H*indazole-5-carboxamide (O.28.60), *N-*(2,2-difluoropropyl)-2-(3-pyridinyl)-2*H-*indazole-5-carboxamide (O.28.61), 2-(3-pyridinyl)-*N-*(2-pyrimidinylmethyl)-2*H-*indazole-5-carboxamide (O.28.62), *N-*[(5-methyl-2-pyrazinyl)methyl]-2-(3-pyridinyl)-2*H-*indazole-5-carboxamide (O.28.63), tyclopyrazoflor (O.28.64), sarolaner (O.28.65), lotilaner (O.28.66), *N-*[4-chloro-3-[[(phenylmethyl)amino]carbonyl]phenyl]-1-methyl-3-(1,1,2,2,2-pentafluoroethyl)-4-(trifluoromethyl)-1*H-*pyrazole-5-carboxamide (O.28.67), M.UN.22a 2-(3-ethylsulfonyl-2-pyridyl)-3-methyl-6-(trifluoromethyl)imidazo[4,5-b]pyridine (O.28.68), 2-[3-ethylsulfonyl-5-(trifluoromethyl)-2-pyridyl]-3-methyl-6-(trifluoromethyl)imidazo[4,5-b]pyridine (O.28.69), 4-[5-(3,5-dichlorophenyl)-5-(trifluoromethyl)-4*H-*isoxazol-3-yl]-*N-*[(4*R*)-2-ethyl-3-oxo-isoxazolidin-4-yl]-2-methyl-benzamide (O.28.70), 4-[5-(3,5-dichloro-4-fluoro-phenyl)-5-(trifluoromethyl)-4*H-*isoxazol-3-yl]-*N-*[(4*R*)-2-ethyl-3-oxo-isoxazolidin-4-yl]-2-methyl-benzamide (O.28.71), *N-*[4-chloro-3-(cyclopropylcarbamoyl)phenyl]-2-methyl-5-(1,1,2,2,2-pentafluoroethyl)-4-(trifluoromethyl)pyrazole-3-carboxamide (O.28.72), *N-*[4-chloro-3-[(1-cyanocyclopropyl)carbamoyl]phenyl]-2-methyl-5-(1,1,2,2,2-pentafluoroethyl)-4-(trifluoromethyl)-pyrazole-3-carboxamide (O.28.73), acynonapyr (O.28.74), benzpyrimoxan (O.28.75), chloro-*N-*(1-cyanocyclopropyl)-5-[1-[2-methyl-5-(1,1,2,2,2-pentafluoroethyl)-4-(trifluoromethyl)pyrazol-3-yl]pyrazol-4-yl]benzamide (O.28.76), oxazosulfyl (O.28.77), [(2*S*,3*R*,4*R*,5*S*,6*S*)-3,5-dimethoxy-6-methyl-4-propoxy-tetrahydropyran-2-yl]-*N-*[4-[1-[4-(trifluoromethoxy)phenyl]-1,2,4-triazol-3-yl]phenyl]carbamate (O.28.78), [(2*S*,3*R*,4*R*,5*S*,6*S*)-3,4,5-trimethoxy-6-methyl-tetrahydropyran-2-yl] N-[4-[1-[4-(trifluoro-methoxy)phenyl]-1,2,4-triazol-3-yl]phenyl]carbamate (O.28.79), [(2*S*,3*R*,4*R*,5*S*,6*S*)-3,5-dimethoxy-6-methyl-4-propoxy-tetrahydropyran-2-yl]-*N-*[4-[1-[4-(1,1,2,2,2-pentafluoroethoxy)phenyl]-1,2,4-triazol-3-yl]phenyl]carbamate (O.28.80), [(2*S*,3*R*,4*R*,5*S*,6*S*)-3,4,5-trimethoxy-6-methyl-tetrahydropyran-2-yl]-*N-*[4-[1-[4-(1,1,2,2,2-penta-fluoroethoxy)phenyl]-1,2,4-triazol-3-yl]phenyl]carbamate (0.28.81), (2*Z*)-3-(2-isopropylphenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one (O.28.82).

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day.

In the binary mixtures and compositions according to the invention the weight ratio of the component 1) and the component 2) generally depends from the properties of the active components used, usually it is in the range of from 1:10,000 to 10,000:1, often it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

Accordingly, the present invention furthermore relates to mixtures comprising one compound of the formula I (component 1, a group represented by the expression "(I)") and one pesticide II (component 2), wherein pesticide II is an active ingredients selected from the groups A) to O) defined above.

Further embodiments B-1 to B-684 listed in Table B below relate to mixtures comprising as active components one of the in the present specification individualized compounds of the formula I, which is selected from the group of compounds I.A.A-1 to I.A.A-1700, I.B.A-1 to I.B.A-1700, I.C.A-1 to I.C.A-1700, I.D.A-1 to I.D.A-1700, I.E.A-1 to I.E.A-1700, I.F.A-1 to I.F.A-1700, I.G.A-1 to I.G.A-1700, I.H.A-1 to I.H.A-1700, I.J.A-1 to I.J.A-1700, I.K.A-1 to I.K.A-1700, I.L.A-1 to I.L.A-1700, I.M.A-1 to I.M.A-1700, I.N.A-1 to I.N.A-1700, I.O.A-1 to I.O.A-1700, I.P.A-1 to I.P.A-1700, I.Q.A-1 to I.Q.A-1700, I.R.A-1 to I.R.A-1700, I.S.A-1 to I.S.A-1700, I.T.A-1 to I.T.A-1700, I.U.A-1 to I.U.A-1700, I.V.A-1 to I.V.A-1700, I.W.A-1 to I.W.A-1700, I.X.A-1 to I.X.A-1700, I.Y.A-1 to I.Y.A-1700, I.B2.A-1 to I.B2.A-1700, I.H2.A-1 to I.H2.A-1700, I.M2.A-1 to I.M2.A-1700, , as defined in tables 1 to 27 (component 1, a group represented by the expression "(I)") and one pesticide II selected from the groups A) to O) as defined herein (component 2, for example, (A.1.1) or azoxystrobin, in embodiment B-1).

Further embodiments B-1 to B-684 listed in Table B below relate to the mixtures comprising as active components one of the in the present specification individualized compounds of the formula I, which is selected from the group of compounds Ex-1 to Ex-44 of formula I as defined in Table I below (component 1, a group represented by the expression "(I)") and one pesticide II selected from the groups A) to O) as defined herein (component 2, for example, (A.1.1) or azoxystrobin, in embodiment B-1).

Preferably, the compositions described in Table B comprise the active components in synergistically effective amounts.

**Table B:**

| |
|---|
| B-1: (I) + (A.1.1), B-2: (I) + (A.1.2), B-3: (I) + (A.1.3), B-4: (I) + (A.1.4), B-5: (I) + (A.1.5), B-6: (I) + (A.1.6), B-7: (I) + (A.1.7), B-8: (I) + (A.1.8), B-9: (I) + (A.1.9), B-10: (I) + (A.1.10), B-11: (I) + (A.1.11), B-12: (I) + (A.1.12), B-13: (I) + (A.1.13), B-14: (I) + (A.1.14), B-15: (I) + (A.1.15), B-16: (I) + (A.1.16), B-17: (I) + (A.1.17), B-18: (I) + (A.1.18), B-19: (I) + (A.1.19), B-20: (I) + (A.1.20), B-21: (I) + (A.1.21), B-22: (I) + (A.1.21a), B-23: (I) + (A.1.22), B-24: (I) + (A.1.25), B-25: (I) + (A.1.34), B-26: (I) + (A.1.35), B-27: (I) + (A.1.36), B-28: (I) + (A.1.37), B-29: (I) + (A.1.38), B-30: (I) + (A.2.1), B-31: (I) + (A.2.2), B-32: (I) + (A.2.3), B-33: (I) + (A.2.4), B-34: (I) + (A.2.5), B-35: (I) + (A.3.1), B-36: (I) + (A.3.2), B-37: (I) + (A.3.3), B-38: (I) + (A.3.4), B-39: (I) + (A.3.5), B-40: (I) + (A.3.6), B-41: (I) + (A.3.7), B-42: (I) + (A.3.8), B-43: (I) + (A.3.9), B-44: (I) + (A.3.10), B-45: (I) + (A.3.11), B-46: (I) + (A.3.12), B-47: (I) + (A.3.13), B-48: (I) + (A.3.14), B-49: (I) + (A.3.15), B-50: (I) + (A.3.16), B-51: (I) + (A.3.17), B-52: (I) + (A.3.18), B-53: (I) + (A.3.19), B-54: (I) + (A.3.20), B-55: (I) + (A.3.21), B-56: (I) + (A.3.22), B-57: (I) + (A.3.23), B-58: (I) + (A.3.24), B-59: (I) + |
| (A.3.25), B-60: (I) + (A.3.26), B-61: (I) + (A.3.27), B-62: (I) + (A.3.28), B-63: (I) + (A.3.30), B-64: (I) + (A.3.31), B-65: (I) + (A.3.32), B-66: (I) + (A.3.33), B-67: (I) + (A.3.34), B-68: (I) + (A.3.35), B-69: (I) + (A.3.36), B-70: (I) + (A.3.37), B-71: (I) + (A.3.38), B-72: (I) + (A.3.39), B-73: (I) + (A.4.1), B-74: (I) + (A.4.2), B-75: (I) + (A.4.3), B-76: (I) + (A.4.4), B-77: (I) + (A.4.5), B-78: (I) + (A.4.6), B-79: (I) + (A.4.7), B-80: (I) + (A.4.8), B-81: (I) + (A.4.9), B-82: (I) + (A.4.10), B-83: (I) + (A.4.11), B-84: (I) + (A.4.12), B-85: (I) + (B.1.1), B-86: (I) + (B.1.2), B-87: (I) + (B.1.3), B-88: (I) + (B.1.4), B-89: (I) + (B.1.5), B-90: (I) + (B.1.6), B-91: (I) + (B.1.7), B-92: (I) + (B.1.8), B-93: (I) + (B.1.9), B-94: (I) + (B.1.10), B-95: (I) + (B.1.11), B-96: (I) + (B.1.12), B-97: (I) + (B.1.13), B-98: (I) + (B.1.14), B-99: (I) + (B.1.15), B-100: (I) + (B.1.16), B-101: (I) + (B.1.17), B-102: (I) + (B.1.18), B-103: (I) + (B.1.19), B-104: (I) + (B.1.20), B-105: (I) + (B.1.21), B-106: (I) + (B.1.22), B-107: (I) + (B.1.23), B-108: (I) + (B.1.24), B-109: (I) + (B.1.25), B-110: (I) + (B.1.26), B-111: (I) + (B.1.27), B-112: (I) + (B.1.28), B-113: (I) + (B.1.29), B-114: (I) + (B.1.30), B-115: (I) + (B.1.31), B-116: (I) + (B.1.32), B-117: (I) + (B.1.37), B-118: (I) + (B.1.38), B-119: (I) + (B.1.39), B-120: (I) + (B.1.40), B-121: (I) + (B.1.41), B-122: (I) + (B.1.42), B-123: (I) + (B.1.43), B-124: (I) + (B.1.44), B-125: (I) + (B.1.45), B-126: (I) + (B.1.46), B-127: (I) + (B.1.47), B-128: (I) + (B.1.48), B-129: (I) + (B.1.49), B-130: (I) + (B.1.50), B-131: (I) + (B.1.51), B-132: (I) + (B.1.52), B-133: (I) + (B.2.1), B-134: (I) + (B.2.2), B-135: (I) + (B.2.3), B-136: (I) + (B.2.4), B-137: (I) + (B.2.5), B-138: (I) + (B.2.6), B-139: (I) + (B.2.7), B-140: (I) + (B.2.8), B-141: (I) + (B.3.1), B-142: (I) + (B.4.1), B-143: (I) + (C.1.1), B-144: (I) + (C.1.2), B-145: (I) + (C.1.3), B-146: (I) + (C.1.4), B-147: (I) + (C.1.5), B-148: (I) + (C.1.6), B-149: (I) + (C.1.7), B-150: (I) + (C.2.1), B-151: (I) + (C.2.2), B-152: (I) + (C.2.3), B-153: (I) + (C.2.4), B-154: (I) + (C.2.5), B-155: (I) + (C.2.6), B-156: (I) + (C.2.7), B-157: (I) + (C.2.8), B-158: (I) + (D.1.1), B-159: (I) + (D.1.2), B-160: (I) + (D.1.3), B-161: (I) + (D.1.4), B-162: (I) + (D.1.5), B-163: (I) + (D.1.6), B-164: (I) + (D.1.7), B-165: (I) + (D.1.8), B-166: (I) + (D.1.9), B-167: (I) + (D.1.10), B-168: (I) + (D.1.11), B-169: (I) + (D.1.12), B-170: (I) + (D.1.13), B-171: (I) + (D.1.14), B-172: (I) + (D.1.15), B-173: (I) + (D.1.16), B-174: (I) + (D.2.1), B-175: (I) + (D.2.2), B-176: (I) + (D.2.3), B-177: (I) + (D.2.4), B-178: (I) + (D.2.5), B-179: (I) + (D.2.6), B-180: (I) + (D.2.7), B-181: (I) + (E.1.1), B-182: (I) + (E.1.2), B-183: (I) + (E.1.3), B-184: (I) + (E.2.1), B-185: (I) + (E.2.2), B-186: (I) + (E.2.3), B-187: (I) + (E.2.4), B-188: (I) + (E.2.5), B-189: (I) + (E.2.6), B-190: (I) + (F.1.1), B-191: (I) + (F.1.2), B-192: (I) + (F.1.3), B-193: (I) + (F.1.4), B-194: (I) + (F.1.5), B-195: (I) + (F.2.1), B-196: (I) + (G.1.1), B-197: (I) + (G.1.2), B-198: (I) + (G.1.3), B-199: (I) + (G.1.4), B-200: (I) + (G.2.1), B-201: (I) + (G.2.2), B-202: (I) + (G.2.3), B-203: (I) + (G.2.4), B-204: (I) + (G.2.5), B-205: (I) + (G.2.6), B-206: (I) + (G.2.7), B-207: (I) + (G.3.1), B-208: (I) + (G.3.2), B-209: (I) + (G.3.3), B-210: (I) + (G.3.4), B-211: (I) + (G.3.5), B-212: (I) + (G.3.6), B-213: (I) + (G.3.7), B-214: (I) + (G.4.1), B-215: (I) + (G.5.1), B-216: (I) + (G.5.2), B-217: (I) + (G.5.3), B-218: (I) + (G.5.4), B-219: (I) + (G.5.5), B-220: (I) + (G.5.6), B-221: (I) + (G.5.7), B-222: (I) + (G.5.8), B-223: (I) + (G.5.9), B-224: (I) + (G.5.10), B-225: (I) + (G.5.11), B-226: (I) + (H.1.1), B-227: (I) + (H.1.2), B-228: (I) + (H.1.3), B-229: (I) + (H.1.4), B-230: (I) + (H.1.5), B-231: (I) + (H.1.6), B-232: (I) + (H.1.7), B-233: (I) + (H.2.1), B-234: (I) + (H.2.2), B-235: (I) + (H.2.3), B-236: (I) + (H.2.4), B-237: (I) + (H.2.5), B-238: (I) + (H.2.6), B-239: (I) + (H.2.7), B-240: (I) + (H.2.8), B-241: (I) + (H.2.9), B-242: (I) + (H.3.1), B-243: (I) + (H.3.2), B-244: (I) + (H.3.3), B-245: (I) + (H.3.4), B-246: (I) + (H.3.5), B-247: (I) + (H.3.6), B-248: (I) + (H.3.7), B-249: (I) + (H.3.8), B-250: (I) + (H.3.9), B-251: (I) + (H.3.10), B-252: (I) + (H.3.11), B-253: (I) + (H.4.1), B-254: (I) + (H.4.2), B-255: (I) + (H.4.3), B-256: (I) + (H.4.4), B-257: (I) + (H.4.5), B-258: (I) + (H.4.6), B-259: (I) + (H.4.7), B-260: (I) + (H.4.8), B-261: (I) + (H.4.9), B-262: (I) + (H.4.10), B-263: (I) + (I.1.1), B-264: |
| (I) + (1.1.2), B-265: (I) + (1.2.1), B-266: (I) + (I.2.2), B-267: (I) + (I.2.3), B-268: (I) + (I.2.4), B-269: (I) + (I.2.5), B-270: (I) + (J.1.1), B-271: (I) + (J.1.2), B-272: (I) + (J.1.3), B-273: (I) + (J.1.4), B-274: (I) + (J.1.5), B-275: (I) + (J.1.6), B-276: (I) + (J.1.7), B-277: (I) + (J.1.8), B-278: (I) + (J.1.9), B-279: (I) + (J.1.10), B-280: (I) + (K.1.1), B-281: (I) + (K.1.2), B-282: (I) + (K.1.3), B-283: (I) + (K.1.4), B-284: (I) + (K.1.5), B-285: (I) + (K.1.6), B-286: (I) + (K.1.7), B-287: (I) + (K.1.8), B-288: (I) + (K.1.9), B-289: (I) + (K.1.10), B-290: (I) + (K.1.11), B-291: (I) + (K.1.12), B-292: (I) + (K.1.13), B-293: (I) + (K.1.14), B-294: (I) + (K.1.15), B-295: (I) + (K.1.16), B-296: (I) + (K.1.17), B-297: (I) + (K.1.18), B-298: (I) + (K.1.19), B-299: (I) + (K.1.20), B-300: (I) + (K.1.21), B-301: (I) + (K.1.22), B-302: (I) + (K.1.23), B-303: (I) + (K.1.24), B-304: (I) + (K.1.25), B-305: (I) + (K.1.26), B-306: (I) + (K.1.27), B-307: (I) + (K.1.28), B-308: (I) + (K.1.29), B-309: (I) + (K.1.30), B-310: (I) + (K.1.31), B-311: (I) + (K.1.32), B-312: (I) + (K.1.33), B-313: (I) + (K.1.34), B-314: (I) + (K.1.35), B-315: (I) + (K.1.36), B-316: (I) + (K.1.37), B-317: (I) + (K.1.38), B-318: (I) + (K.1.39), B-319: (I) + (K.1.40), B-320: (I) + (K.1.41), B-321: (I) + (K.1.42), B-322: (I) + (K.1.43), B-323: (I) + (K.1.44), B-324: (I) + (K.1.45), B-325: (I) + (K.1.46), B-326: (I) + (K.1.47), B-327: (I) + (K.1.48), B-328: (I) + (K.1.49), B-329: (I) + (K.1.50), B-330: (I) + (K.1.51), B-331: (I) + (K.1.52), B-332: (I) + (K.1.53), B-333: (I) + (K.1.54), B-334: (I) + (O.1.1), B-335: (I) + (0.1.2), B-336: (I) + (0.1.3), B-337: (I) + (0.1.4), B-338: (I) + (0.1.5), B-339: (I) + (0.1.6), B-340: (I) + (0.1.7), B-341: (I) + (0.1.8), B-342: (I) + (0.1.9), B-343: (I) + (O.1.10), B-344: (I) + (O.1.11), B-345: (I) + (0.1.12), B-346: (I) + (0.1.13), B-347: (I) + (0.1.14), B-348: (I) + (0.1.15), B-349: (I) + (0.1.16), B-350: (I) + (0.1.17), B-351: (I) + (0.1.18), B-352: (I) + (0.1.19), B-353: (I) + (0.1.20), B-354: (I) + (0.1.21), B-355: (I) + (0.1.22), B-356: (I) + (0.1.23), B-357: (I) + (0.1.24), B-358: (I) + (0.1.25), B-359: (I) + (0.1.26), B-360: (I) + (0.1.27), B-361: (I) + (0.1.28), B-362: (I) + (0.1.29), B-363: (I) + (0.1.30), B-364: (I) + (0.1.31), B-365: (I) + (0.1.32), B-366: (I) + (0.1.33), B-367: (I) + (0.1.34), B-368: (I) + (0.1.35), B-369: (I) + (0.1.36), B-370: (I) + (0.1.37), B-371: (I) + (0.1.38), B-372: (I) + (0.1.39), B-373: (I) + (0.1.40), B-374: (I) + (0.1.41), B-375: (I) + (0.1.42), B-376: (I) + (0.1.43), B-377: (I) + (0.1.44), B-378: (I) + (0.1.45), B-379: (I) + (0.1.46), B-380: (I) + (0.1.47), B-381: (I) + (0.1.48), B-382: (I) + (0.1.49), B-383: (I) + (0.1.50), B-384: (I) + (0.1.51), B-385: (I) + (0.1.52), B-386: (I) + (0.1.53), B-387: (I) + (0.1.54), B-388: (I) + (0.1.55), B-389: (I) + (0.1.56), B-390: (I) + (0.1.57), B-391: (I) + (0.1.58), B-392: (I) + (0.1.59), B-393: (I) + (0.1.60), B-394: (I) + (0.1.61), B-395: (I) + (0.1.62), B-396: (I) + (0.1.63), B-397: (I) + (0.1.64), B-398: (I) + (0.1.65), B-399: (I) + (0.1.66), B-400: (I) + (0.1.67), B-401: (I) + (0.1.68), B-402: (I) + (0.1.69), B-403: (I) + (0.1.70), B-404: (I) + (0.1.71), B-405: (I) + (0.1.72), B-406: (I) + (0.1.73), B-407: (I) + (0.1.74), B-408: (I) + (0.1.75), B-409: (I) + (0.1.76), B-410: (I) + (0.1.77), B-411: (I) + (0.1.78), B-412: (I) + (0.1.79), B-413: (I) + (0.1.80), B-414: (I) + (0.1.81), B-415: (I) + (0.1.82), B-416: (I) + (0.1.83), B-417: (I) + (0.1.84), B-418: (I) + (0.1.85), B-419: (I) + (0.1.86), B-420: (I) + (0.1.87), B-421: (I) + (0.1.88), B-422: (I) + (0.1.89), B-423: (I) + (0.1.90), B-424: (I) + (0.1.91), B-425: (I) + (0.2.1), B-426: (I) + (O.2.2), B-427: (I) + (O.2.3), B-428: (I) + (O.2.4), B-429: (I) + (O.2.5), B-430: (I) + (O.2.6), B-431: (I) + (O.2.7), B-432: (I) + (0.3.1), B-433: (I) + (O.3.2), B-434: (I) + (O.3.3), B-435: (I) + (O.3.4), B-436: (I) + (O.3.5), B-437: (I) + (O.3.6), B-438: (I) + (O.3.7), B-439: (I) + (O.3.8), B-440: (I) + (O.3.9), B-441: (I) + (0.3.10), B-442: (I) + (0.3.11), B-443: (I) + (0.3.12), B-444: (I) + (0.3.13), B-445: (I) + (0.3.14), B-446: (I) + (0.3.15), B-447: (I) + (0.3.16), B-448: (I) + (0.3.17), B-449: (I) + (0.3.18), B-450: (I) + (0.3.19), B-451: (I) + (O.3.20), B-452: (I) + (0.3.21), B-453: (I) + (O.3.22), B-454: (I) + (O.3.23), B-455: (I) + (O.3.24), B-456: (I) + (O.3.25), B-457: (I) + (O.3.26), B-458: (I) + (O.3.27), B-459: (I) + (O.3.28), B-460: (I) |
| + (O.3.29), B-461: (I) + (O.3.30), B-462: (I) + (0.3.31), B-463: (I) + (O.3.32), B-464: (I) + (O.3.33), B-465: (I) + (O.3.34), B-466: (I) + (O.3.35), B-467: (I) + (O.3.36), B-468: (I) + (O.3.37), B-469: (I) + (O.3.38), B-470: (I) + (O.3.39), B-471: (I) + (O.3.40), B-472: (I) + (0.3.41), B-473: (I) + (O.3.42), B-474: (I) + (O.3.43), B-475: (I) + (O.3.44), B-476: (I) + (O.3.45), B-477: (I) + (O.3.46), B-478: (I) + (O.3.47), B-479: (I) + (O.3.48), B-480: (I) + (O.3.49), B-481: (I) + (O.3.50), B-482: (I) + (0.3.51), B-483: (I) + (O.3.52), B-484: (I) + (0.4.1), B-485: (I) + (O.4.2), B-486: (I) + (O.4.3), B-487: (I) + (O.4.4), B-488: (I) + (O.4.5), B-489: (I) + (O.4.6), B-490: (I) + (O.4.7), B-491: (I) + (O.4.8), B-492: (I) + (O.4.9), B-493: (I) + (0.4.10), B-494: (I) + (0.4.11), B-495: (I) + (0.4.12), B-496: (I) + (0.4.13), B-497: (I) + (0.4.14), B-498: (I) + (0.4.15), B-499: (I) + (0.5.1), B-500: (I) + (O.5.2), B-501: (I) + (0.6.1), B-502: (I) + (O.6.2), B-503: (I) + (O.6.3), B-504: (I) + (0.6.4), B-505: (I) + (O.6.5), B-506: (I) + (0.7.1), B-507: (I) + (O.7.2), B-508: (I) + (O.7.3), B-509: (I) + (O.7.4), B-510: (I) + (O.7.5), B-511: (I) + (0.8.1), B-512: (I) + (O.8.2), B-513: (I) + (O.8.3), B-514: (I) + (O.8.4), B-515: (I) + (O.8.5), B-516: (I) + (0.9.1), B-517: (I) + (O.9.2), B-518: (I) + (O.9.3), B-519: (I) + (O.10.1), B-520: (I) + (0.10.2), B-521: (I) + (0.10.3), B-522: (I) + (0.10.4), B-523: (I) + (O.11.1), B-524: (I) + (0.11.2), B-525: (I) + (0.11.3), B-526: (I) + (0.11.4), B-527: (I) + (0.11.5), B-528: (I) + (0.11.6), B-529: (I) + (0.11.7), B-530: (I) + (0.11.8), B-531: (I) + (0.11.9), B-532: (I) + (O.11.10), B-533: (I) + (O.11.11), B-534: (I) + (0.11.12), B-535: (I) + (0.11.13), B-536: (I) + (0.12.1), B-537: (I) + (0.12.2), B-538: (I) + (0.12.3), B-539: (I) + (0.12.4), B-540: (I) + (0.12.5), B-541: (I) + (0.12.6), B-542: (I) + (0.13.1), B-543: (I) + (0.13.2), B-544: (I) + (0.13.3), B-545: (I) + (0.14.1), B-546: (I) + (0.14.2), B-547: (I) + (0.14.3), B-548: (I) + (0.14.4), B-549: (I) + (0.15.1), B-550: (I) + (0.15.2), B-551: (I) + (0.15.3), B-552: (I) + (0.15.4), B-553: (I) + (0.15.5), B-554: (I) + (0.15.6), B-555: (I) + (0.15.7), B-556: (I) + (0.15.8), B-557: (I) + (0.15.9), B-558: (I) + (0.15.10), B-559: (I) + (0.15.11), B-560: (I) + (0.16.1), B-561: (I) + (0.17.1), B-562: (I) + (0.18.1), B-563: (I) + (0.18.2), B-564: (I) + (0.18.3), B-565: (I) + (0.18.4), B-566: (I) + (0.18.5), B-567: (I) + (0.19.1), B-568: (I) + (0.20.1), B-569: (I) + (O.20.2), B-570: (I) + (O.20.3), B-571: (I) + (O.20.4), B-572: (I) + (0.21.1), B-573: (I) + (0.21.2), B-574: (I) + (0.21.3), B-575: (I) + (0.21.4), B-576: (I) + (0.21.5), B-577: (I) + (0.21.6), B-578: (I) + (0.21.7), B-579: (I) + (0.22.1), B-580: (I) + (O.22.2), B-581: (I) + (O.22.3), B-582: (I) + (O.22.4), B-583: (I) + (0.23.1), B-584: (I) + (O.23.2), B-585: (I) + (O.23.3), B-586: (I) + (O.23.4), B-587: (I) + (0.24.1), B-588: (I) + (O.24.2), B-589: (I) + (O.24.3), B-590: (I) + (O.24.4), B-591: (I) + (O.24.5), B-592: (I) + (0.25.1), B-593: (I) + (O.25.2), B-594: (I) + (0.26.1), B-595: (I) + (O.26.2), B-596: (I) + (O.26.3), B-597: (I) + (O.26.4), B-598: (I) + (O.26.5), B-599: (I) + (O.26.6), B-600: (I) + (O.26.7), B-601: (I) + (O.26.8), B-602: (I) + (O.26.9), B-603: (I) + (0.26.10), B-604: (I) + (0.26.11), B-605: (I) + (0.26.12), B-606: (I) + (0.26.13), B-607: (I) + (0.26.14), B-608: (I) + (0.26.15), B-609: (I) + (0.26.16), B-610: (I) + (0.26.17), B-611: (I) + (0.26.18), B-612: (I) + (0.27.1), B-613: (I) + (0.28.1), B-614: (I) + (O.28.2), B-615: (I) + (O.28.3), B-616: (I) + (O.28.4), B-617: (I) + (O.28.5), B-618: (I) + (O.28.7), B-619: (I) + (O.28.8), B-620: (I) + (O.28.9), B-621: (I) + (0.28.10), B-622: (I) + (0.28.11), B-623: (I) + (0.28.12), B-624: (I) + (0.28.13), B-625: (I) + (0.28.14), B-626: (I) + (0.28.15), B-627: (I) + (0.28.16), B-628: (I) + (0.28.17), B-629: (I) + (0.28.18), B-630: (I) + (0.28.19), B-631: (I) + (0.28.20), B-632: (I) + (0.28.21), B-633: (I) + (O.28.22), B-634: (I) + (O.28.23), B-635: (I) + (O.28.24), B-636: (I) + (O.28.25), B-637: (I) + (O.28.26), B-638: (I) + (O.28.27), B-639: (I) + (O.28.28), B-640: (I) + (O.28.29), B-641: (I) + (O.28.30), B-642: (I) + (0.28.31), B-643: (I) + (O.28.42), B-644: (I) + (O.28.43), B-645: (I) + (O.28.44), B-646: (I) + (O.28.45), B-647: (I) + (O.28.46), B-648: (I) + (O.28.47), B-649: (I) + |
| (O.28.48), B-650: (I) + (O.28.49), B-651: (I) + (O.28.50), B-652: (I) + (O.28.51), B-653: (I) + (O.28.52), B-654: (I) + (O.28.53), B-655: (I) + (O.28.54), B-656: (I) + (O.28.55), B-657: (I) + (O.28.56), B-658: (I) + (O.28.57), B-659: (I) + (O.28.58), B-660: (I) + (0.28.59), B-661: (I) + (O.28.60), B-662: (I) + (O.28.61), B-663: (I) + (O.28.62), B-664: (I) + (O.28.63), B-665: (I) + (O.28.64), B-666: (I) + (O.28.65), B-667: (I) + (O.28.66), B-668: (I) + (O.28.67), B-669: (I) + (O.28.68), B-670: (I) + (O.28.69), B-671: (I) + (O.28.70), B-672: (I) + (O.28.71), B-673: (I) + (O.28.72), B-674: (I) + (O.28.73), B-675: (I) + (O.28.74), B-676: (I) + (O.28.75), B-677: (I) + (O.28.76), B-678: (I) + (O.28.77), B-679: (I) + (O.28.78), B-680: (I) + (O.28.79), B-681: (I) + (O.28.80), B-682: (I) + (0.28.81), B-683: (I) + (O.28.82), B-684: (I) + (A.3.29). |

The mixtures of active substances can be prepared as compositions comprising besides the active ingredients at least one inert ingredient (auxiliary) by usual means, e. g. by the means given for the compositions of compounds I.

Concerning usual ingredients of such compositions reference is made to the explanations given for the compositions containing compounds I.

The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

### I. Synthesis examples

The compounds of the formula I can be prepared according to the methods outlined below.

### I.1) Preparation of 4-acetyl-N-phenyl-benzamide

11.4 g (90.0 mmol) oxalyl chloride were added dropwise to a solution of 4.9 g (30.0 mmol) 4-acetyl benzoic acid and two drops of *N,N*-dimethylformamide in dichloromethane. After stirring for 3 hours at 35 °C all volatile components were removed under reduced pressure and the resulting acid chloride was used without further purification.

2.4 g (13.1 mmol) of the crude acid chloride were added dropwise to a solution of 1.24 g (13.1 mmol) aniline in 60 mL of tetrahydrofurane. Upon completion, the reaction mixture was diluted with methyl *tert*-butylether and washed twice with an aqueous solution of sodium hydroxide and twice with a saturated sodium chloride solution. Drying over sodium sulfate, evaporation of the solvent and recrystallization in methyl *tert*-butylether resulted in 2.5 g (10 mmol, 80 %) of the title amide. ¹H-NMR [400 MHz, DMSO-*d6*] δ = 10.4 (s, 1H), 8.1 (m, 4H), 7.8 (d, 2H), 7.4 (t, 2H), 7.1 (t, 1H), 2.7 (s, 3H) ppm.

### I.2) Preparation of N-Phenyl-4-(4,4,4-trifluoro-3-oxo-butanoyl) benzamide

8.47 g (31.3 mmol) of sodium ethanolate in ethanol were diluted with 50 mL of dry ethanol and treated with 2.50 g (10.4 mmol) of 4-acetyl-*N*-phenyl-benzamide at room temperature. 4.45 g (31.3 mmol) ethyl 2,2,2-trifluoroacetate were added and the solution was stirred at room temperature for 16 hours. Upon completion, the reaction mixture was acidified with diluted hydrochloric acid. The resulting precipitate was filtered off, washed twice with water, dried under vacuum and used without further purification. ¹H-NMR [400 MHz, DMSO-*d6*] δ = 10.5 (s, 1H), 8.3 (d, 2H), 8.1 (d, 2H), 7.8 (d, 2H), 7.4 (m, 2H), 7.1 (t, 1H), 7.0 (s, 1H) ppm.

### I.3) Preparation of 4-[5-Hydroxy-5-(trifluoromethyl)-4H-isoxazol-3-yl]-N-phenyl-benzamide (Ex-7)

0.29 (7.12 mmol) sodium hydroxide were dissolved in 5 mL water and added to a solution of 0.46 g (6.56 mmol) hydroxyl amine hydrochloride in 5 mL water. 2.00 g (5.97 mmol) *N*-phenyl-4-(4,4,4-trifluoro-3-oxo-butanoyl) benzamide were added to this solution and stirred for 3 hours at 60 °C. Upon completion, the reaction mixture was cooled to room temperature, treated with an aqueous saturated solution of ammonium chloride and extracted twice with ethyl acetate. The organic solution was washed three times with water, dried over sodium sulfate and the organic solvent was removed under vacuum. Recrystallization with methyl *tert*-butylether gave 1.35 g (65 %) of the title compound as a white solid. Melting point: 217 °C; ¹H-NMR [400 MHz, DMSO-*d6*] δ = 10.3 (s, 1H), 8.7 (s, 1H), 8.1 (d, 2H), 7.9 (d, 2H), 7.8 (d, 2H), 7.4 (m, 2H), 7.1 (t, 1H), 4.0 (d, 1H), 3.6 (d, 1H) ppm.

### I.4) Preparation of N-[[4-[5-hydroxy-5-(trifluoromethyl)-4H-isoxazol-3-yl]phenyl]methyl]cyclopropanecarboxamide (Ex-4)

To a solution of 0.17 g cyclopropanecarboxylic acid (2 mmol) in 5 mL dichloromethane, 0.36 g of 1,1'-carbonyldiimidazol (2.2 mmol) were added slowly at room temperature. After 30 minutes, a solution of 0.52 g of 3-[4-(aminomethyl)phenyl]-5-(trifluoromethyl)-4H-isoxazol-5-ol (2 mmol) in 10 mL tetrahydrofurane was added. The reaction mixture was allowed to stir for 2 hours, then it was diluted with water, and extracted twice with ethyl acetate. The organic solution was washed once with a saturated solution of sodium bicarbonate, then with water, dried over sodium sulfate and the organic solvent was removed under vacuum. Recrystallization in diisopropyl ether gave 0.65 g (94 %) of the title compound as a yellow solid. Melting point: 207 °C; ¹H-NMR [400 MHz, DMSO-*d6*] δ = 9.7 (t, 2H), 7.7 (d, 2H), 7.3 (d, 2H), 4.3 (d, 2H), 3.9 (d, 1H), 3.5 (d, 1H), 1.7 (m, 1H), 0.8 (dd, 4H) ppm.

### I.5) Preparation of N-[(4-acetylphenyl)methyl]-N-methyl-cyclopropanecarboxamide

To a solution of 9.4 g of 1-[4-(methylaminomethyl)phenyl]ethenone (57.6 mmol) in tetrahydrofurane, 14 g of triethylamine (138.2 mmol) and 7.2 g of cyclopropanecarboxylic chloride (69.1 mmol) were added at room temperature. The reaction mixture was allowed to stir overnight, then it was diluted with an aqueous 0.1 % solution of hydrochloric acid, and extracted twice with methyl *tert*-butylether. The organic solution was washed twice with brine, dried over sodium sulfate and the organic solvent was removed under vacuum. Column chromatography using a mixture of cyclohexane/ethyl acetate gave 8.9 g (67 %) of the title compound as a yellow oil. ¹H-NMR [400 MHz, CDCl₃, mixture of rotamers] δ = 8.0 (d, 2H), 7.8 (d, 2H), 7.4-7.2 (d, 2H+2H), 4.8 (s, 2H), 4.7 (2, 2H), 3.1 (s, 3H), 3.0 (s, 3H), 2.6 (s, 3H+3H), 1.8 (m, 1H), 1.7 (m, 1H), 1.1 (m, 2H+2H), 0.8 (m, 2H), 0.7 (m, 2H) ppm.

### I.6) Preparation of N-methyl-N-[[4-(4,4,4-trifluoro-3-oxo-butanoyl)phenyl]methyl]cyclopropanecarboxamide

To a solution of 68.05 g sodium ethoxide (96.2 mmol) in 40 mL ethanol, 8.9 g of *N*-[(4-acetylphenyl)methyl]-*N*-methyl-cyclopropanecarboxamide (38.48 mmol) in 30 mL ethanol was added dropwise at room temperature. After stirring for 30 minutes, a solution of 8.2 g of ethyl 2,2,2-trifluoroacetate (57.72 mmol) in 30 mL ethanol was added. The reaction mixture was allowed to stir overnight at room temperature, then it was diluted with an aqueous 0.1 % solution of hydrochloric acid and extracted twice with methyl *tert*-butylether. The organic solution was washed twice with water, dried over sodium sulfate and the organic solvent was removed under vacuum. 11.5 g (91 %) of the title compound were obtained as a yellow oil, which were used in the next step without further purification. ¹H-NMR [400 MHz, CDCl₃, mixture of rotamers] δ = 8.0 (d, 2H), 7.9 (d, 2H), 7.4 (d, 2H), 7.3 (d, 2H), 6.6 (s, 2H), 6.5 (s, 2H), 5.8 (s, 2H), 5.7 (s, 2H), 3.1 (s, 3H), 3.0 (s, 3H), 1.8 (m, 1H), 1.7 (m, 1H), 1.1 (m, 2H+2H), 0.9 (m, 2H), 0.8 (m, 2H) ppm.

### I.7) Preparation of N-[[4-[5-hydroxy-5-(trifluoromethy)-4H-isoxazol-3-yl]phenyl]methyl]-N-methyl-cyclopropanecarboxamide (Ex-11)

To a solution of 2.69 g hydroxylamine hydrochloride (38.9 mmol) in 17 mL water, a solution of 1.69 g sodium hydroxide (42.16 mmol) in 8 mL water was added. After 30 minutes, a solution of 11.5 g *N*-methyl-*N*-[[4-(4,4,4-trifluoro-3-oxo-butanoyl)phenyl]methyl]cyclopropanecarboxamide (35.14 mmol) in 30 mL ethanol was added. The reaction mixture was stirred for 5 hours at 60 °C, then it was diluted with a saturated solution of ammonium chloride, and extracted twice with methyl *tert*-butylether. The organic solution was washed 3 times with water, dried over sodium sulfate and the organic solvent was removed under vacuum. Column chromatography using a mixture of cyclohexane/ethyl acetate gave 7.7 g (64 %) of the title compound as a yellow oil. ¹H-NMR [400 MHz, CDCl₃, mixture of rotamers] δ = 7.7 (d, 2H), 7.6 (d, 2H), 7.3-7.2 (m, 2H +2H), 4.7-4.4 (d, 2H+2H), 3.8-3.7 (d, 1H+1H), 3.5 (d, 1H+1H), 3.1 (s, 3H), 2.9 (s, 3H), 1.8 (m, 1H), 1. 7 (m, 1H), 1.1 (d, 2H+2H), 0.8 (m, 2H), 0.7 (m, 2H) ppm.

### I.8) Preparation of N-[[4-[5-ethoxy-5-(trifluoromethyl)-4H-isoxazol-3-yl]phenyl]methyl]-N-methyl-cyclopropanecarboxamide (Ex-12)

To a solution of 0.20 g of *N*-[[4-[5-hydroxy-5-(trifluoromethyl)-4H-isoxazol-3-yl]phenyl]methyl]-*N-*methyl-cyclopropanecarboxamide (0.58 mmol) in 10 mL tetrahydrofurane, 0.12 g of triethylamine (1.17 mmol) and 0.076 g of trimethylsilyl chloride (0.70 mmol) were added at room temperature. The reaction mixture was allowed to stir overnight, then it was diluted with water and extracted 3 times with ethyl acetate. The organic phase was washed with a saturated solution of sodium chloride, dried over magnesium sulfate and the organic solvent was removed under vacuum. Column chromatography using a mixture of cyclohexane/ethyl acetate gave 0.217 g (37 %) of the title compound as a yellow oil. ¹H-NMR [400 MHz, CDCl₃, mixture of rotamers] δ = 7.7 (d, 2H), 7.6 (d, 2H), 7.3-7.2 (m, 2H+2H), 4.8 (s, 2H), 4.7 (s, 2H), 3.8-3.7 (d, 1H+1H), 3.6-3.4 (d, 1H+1H), 3.1 (s, 3H), 2.9 (s, 3H), 1.7(m, 1H), 1. 6 (m, 1H), 1.1 (d, 2H+2H), 0.8 (m, 2H), 0.7 (m, 2), 0.2 (s, 9H) ppm.

The compounds listed in Table I were prepared in an analogous manner.

**Table I: Compounds Ex-1 to Ex-44 of formulae I.A2, I.S2 and I.T2,**

| | | | | | | |
|---|---|---|---|---|---|---|
| 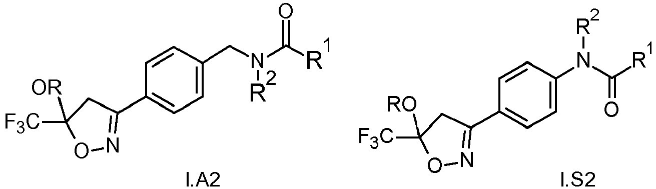 | | | | | | |
| 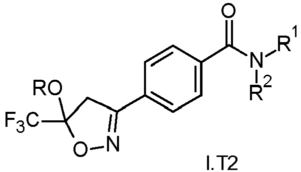 | | | | | | |

| wherein # in radical R indicates the point of attachement to the remainder of the compounds and "cyp" has the meaning cyclopropyl. | | | | | | |
|---|---|---|---|---|---|---|
| **Ex. no** | **Formula** | **R** | **R¹** | **R²** | **HPLC Rₜ (min)*** | **Melting point (°C)** |
| Ex-1 | I.T2 | H | CH₃ | CH₃ | 0.84 | 184 |
| Ex-2 | I.T2 | H | phenyl | CH₃ | 1.01 | 181 |
| Ex-3 | I.A2 | H | phenyl | H | 1.01 | 165 |
| Ex-4 | I.A2 | H | cyp | H | 0.89 | 207 |
| Ex-5 | 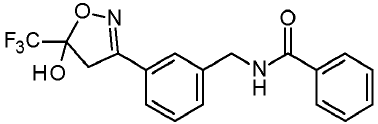 | | | | 0.997 | 217 |
| Ex-6 | 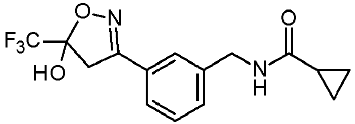 | | | | 0.918 | 175 |
| Ex-7 | I.T2 | H | phenyl | H | 1.038 | 217 |
| Ex-8 | I.S2 | H | phenyl | H | 1.03 | 248 |
| Ex-9 | I.T2 | H | cyp | H | 0.871 | 230 |
| Ex-10 | I.S2 | H | cyp | H | 0.934 | 237 |
| Ex-11 | I.A2 | H | cyp | CH₃ | 0.97 | 180 |
| Ex-12 | I.A2 | #-CH₂CH₃ | cyp | CH₃ | 1.16 | 89 |
| Ex-13 | I.A2 | #-CH₃ | cyp | CH₃ | 1.1 | - |
| Ex-14 | I.A2 | #-Si(CH₃)₃ | cyp | CH₃ | 0.959 | - |
| Ex-15 | I.A2 | #-C(=O)CH₃ | cyp | CH₃ | 1.09 | 120 |
| Ex-16 | I.A2 | #-CH₂CH₃ | cyp | H | 1.109 | 142 |
| Ex-17 | I.A2 | #-Si(CH₃)₃ | cyp | H | 1.24 | - |
| Ex-18 | I.A2 | #-C(=O)CH₃ | cyp | H | 1.02 | 178 |
| Ex-19 | I.A2 | #-CH₃ | cyp | H | 1.047 | - |
| Ex-20 | I.T2 | #-CH₂-O-C(=O)C(CH₃)₃ | phenyl | H | 1.33 | - |
| Ex-21 | I.T2 | #-C(=O)N(CH₃)₂ | phenyl | H | - | - |
| Ex-22 | I.T2 | #-C(=O)N(CH₃)₂ | CH₃ | H | 0.99 | 206 |
| Ex-23 | I.T2 | #-C(=O)CH(CH₃)₂ | CH₃ | H | 1.1 | 173 |
| Ex-24 | I.T2 | #-C(=O)C(CH₃)₃ | CH₃ | H | 1.16 | 193 |
| Ex-25 | I.T2 | 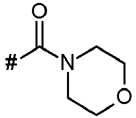 | CH₃ | H | 0.96 | 205 |
| Ex-26 | I.T2 | 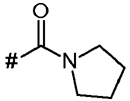 | phenyl | H | 1.26 | - |
| Ex-27 | I.T2 | 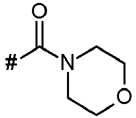 | phenyl | H | 1.18 | - |
| Ex-28 | I.T2 | 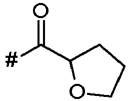 | phenyl | H | 1.12 | - |
| Ex-29 | I.T2 | #-C(=O)CH(CH₃)₂ | phenyl | H | 1.29 | 224 |
| Ex-30 | I.T2 | #-C(=O)CH₂-O-CH₂CH₃ | phenyl | H | 1.22 | - |
| Ex-31 | I.T2 | #-C(=O)-N(CH₃)(CH(CH₃)₂) | phenyl | H | 1.29 | - |
| Ex-32 | I.T2 | #-C(=O)CH₂-O-CH₂CH₃-O-CH₃ | CH₃ | H | 0.97 | - |
| Ex-33 | I.T2 | #-C(=O)CH₂-O-CH₂CH₃ | CH₃ | H | 1.02 | - |
| Ex-34 | I.T2 | #-CH₂-O-C(=O)-CH₂CH(CH₃)₂ | CH₃ | H | - | 107 |
| Ex-35 | I.T2 | #-CH₂-O-C(=O)CH₃ | CH₃ | H | - | 145 |
| Ex-36 | I.T2 | #-CH₂-O-C(=O)CH(CH₃)₂ | CH₃ | H | - | 116 |
| Ex-37 | I.T2 | H | CH₃ | H | 0.86 | 233 |
| Ex-38 | I.T2 | 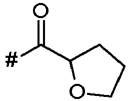 | CH₃ | H | 1.00 | 171 |
| Ex-39 | I.T2 | Na (sodium) | CH₃ | H | 0.81 | - |
| Ex-40 | I.T2 | #-CH₂-O-C(=O)-CH₂CH(CH₃)₂ | phenyl | H | 1.33 | 149 |
| Ex-41 | I.T2 | #-CH₂-O-C(=O)CH(CH₃)₂ | phenyl | H | 1.29 | - |
| Ex-42 | I.T2 | #-C(=O)-cyp | CH₃ | H | 1.04 | 189 |
| Ex-43 | I.T2 | H | cyp | H | - | 194 |
| Ex-44 | 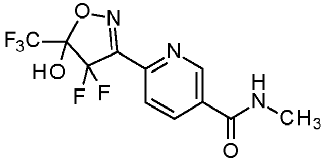 | | | | 0.78 | 232 |

| | | | | | | |
|---|---|---|---|---|---|---|
| * HPLC: High Performance Liquid Chromatography; HPLC-column Kinetex XB C18 1,7µ (50 x 2,1 mm); eluent: acetonitrile / water+0.1% trifluoroacetic acid (gradient from 5:95 to 100 : 0 in 1.5 min at 60°C, flow gradient from 0.8 to 1.0 ml/min in 1.5 min). MS: Quadrupol Electrospray lonisation, 80 V (positive mode). Rₜ: retention time in minutes. | | | | | | |

### II. Biological examples for fungicidal activity

### A. Glass house trials

The fungicidal action of the compounds of formula I was demonstrated by the following experiments. Spray solutions were prepared in several steps. A mixture was prepared of acetone and/or dimethylsulfoxide and the wetting agent/emulsifier Wettol, which is based on ethoxylated alkylphenoles, in a relation (volume) solvent-emulsifier of 99 to 1 was added to 25 mg of the compound to give a total of 5 ml. Water was then added to a total volume of 100 ml. This stock solution was diluted with the described solvent-emulsifier-water mixture to the given concentration.

### 1. Curative control of soy bean rust on soy beans caused by Phakopsora pachyrhizi

Leaves of pot-grown soy bean seedlings were inoculated with spores of Phakopsora pachyrhizi. To ensure the success of the artificial inoculation, the plants were transferred to a humid chamber with a relative humidity of about 95% and 20 to 24°C for 24 hours. The next day the plants were cultivated for 3 days in a greenhouse chamber at 23 to 27°C and a relative humidity between 60 and 80%. Then the plants were sprayed to run-off with an aqueous suspension, containing the concentration of active ingredient or their mixture as described below. The plants were allowed to air-dry. Then the trial plants were cultivated for 14 days in a greenhouse chamber at 23 to 27°C and a relative humidity between 60 and 80%. The extent of fungal attack on the leaves was visually assessed as % diseased leaf area.

In this test, the plants which had been treated with 600 ppm of the active compound Ex-1, Ex-3, Ex-4, Ex-7, Ex-8, Ex-9 and Ex-10 showed a diseased leaf area of at most 15%, whereas the untreated plants showed 90% diseased leaf area.

In this test, the plants which had been treated with 600 ppm of the active compound Ex-2, Ex-13, Ex-15, Ex-18, Ex-19, Ex-28, Ex-40 and Ex-42 showed a diseased leaf area of at most 3%, whereas the untreated plants showed 100% diseased leaf area.

### 2. Protective control of soy bean rust on soy beans caused by Phakopsora pachyrhizi

Leaves of pot-grown soy bean seedlings were sprayed to run-off with an aqueous suspension, containing the concentration of active ingredient or their mixture as described below. The plants were allowed to air-dry. The trial plants were cultivated for 1 day in a greenhouse chamber at 23 to 27°C and a relative humidity between 60 and 80%.Then the plants were inoculated with spores of Phakopsora pachyrhizi. To ensure the success the artificial inoculation, the plants were transferred to a humid chamber with a relative humidity of about 95% and 20 to 24°C for 24 hours. The trial plants were cultivated for fourteen days in a greenhouse chamber at 23 to 27°C and a relative humidity between 60 and 80%. The extent of fungal attack on the leaves was visually assessed as % diseased leaf area.

In this test, the plants which had been treated with 600 ppm of the active compound Ex-1, Ex-3, Ex-4, Ex-7, Ex-8, Ex-9 and Ex-10 showed a diseased leaf area of at most 1%, whereas the untreated plants showed 90% diseased leaf area.

In this test, the plants which had been treated with 600 ppm of the active compound Ex-2, Ex-13, Ex-14, Ex-15, Ex-18, Ex-19, Ex-28, Ex-30, Ex-40 and Ex-42 showed a diseased leaf area of 0%, whereas the untreated plants showed 100% diseased leaf area.

### 3. Curative control of soy bean rust on soy beans caused by Phakopsora pachyrhizi

Leaves of pot-grown soy bean seedlings were inoculated with spores of *Phakopsora pachyrhizi.* To ensure the success of the artificial inoculation, the plants were transferred to a humid chamber with a relative humidity of about 95% and 20 to 24· C for 24 h. The next day the plants were cultivated for 3 days in a greenhouse chamber at 23-27°C and a relative humidity between 60 and 80 %. Then the plants were sprayed to run-off with an aqueous suspension, containing the concentration of active ingredient or their mixture as described below. The plants could air-dry. Then the trial plants were cultivated for 14 days in a greenhouse chamber at 23-27°C and a relative humidity between 60 and 80 %. The extent of fungal attack on the leaves was visually assessed as % diseased leaf area.

In this test, the plants which had been treated with 300 ppm of the active compound Ex-1 and Ex-7 showed a diseased leaf area of at most 2%, whereas the untreated plants showed 100 % diseased leaf area.

### 4. Protective control of soy bean rust on soy beans caused by Phakopsora pachyrhizi

Leaves of pot-grown soy bean seedlings were sprayed to run-off with an aqueous suspension, containing the concentration of active ingredient or their mixture as described below. The plants could air-dry. The trial plants were cultivated for 2 days in a greenhouse chamber at 23 to 27°C and a relative humidity between 60 and 80%. Then the plants were inoculated with spores of *Phakopsora pachyrhizi.* To ensure the success the artificial inoculation, the plants were transferred to a humid chamber with a relative humidity of about 95 % and 20 to 24°C for 24 hours. The trial plants were cultivated for fourteen days in a greenhouse chamber at 23 to 27°C and a relative humidity between 60 and 80%. The extent of fungal attack on the leaves was visually assessed as % diseased leaf area.

In this test, the plants which had been treated with 300 ppm of the active compound Ex-1, Ex-2, Ex-3, Ex-7, Ex-8, Ex-9 and Ex-10 showed a diseased leaf area of at most 1%, whereas the untreated plants showed 90 % diseased leaf area.

In this test, the plants which had been treated with 63 ppm of the active compound Ex-23, Ex-33, Ex-34 and Ex-36 showed a diseased leaf area of at most 4%, whereas the untreated plants showed 90 % diseased leaf area.

### 5. Preventative fungicidal control of rape stem rot on soy beans caused by (Sclerotinia sclerotiorum

Young seedlings of soy beans were grown in pots. These plants were sprayed to run-off with an aqueous suspension, containing the concentration of active ingredient or mixture mentioned in the table below. The next day the treated plants were inoculated: Rye and millet grains were infected with Sclerotinia sclerotiorum. After the infection the grains were air dried for a week. The grains were powdered with a mixer. A small amount of powder was brought onto the soy bean (leaves). Then the trial plants were cultivated for 7 days in a greenhouse chamber at 23°C and a relative humidity between 80 an 85%. The extent of fungal attack on the leaves was visually assessed with a classification method: 0, 33, 50, 67 and 100% disease of leaf area and stem.

In this test, the plants which had been treated with 600 ppm of the active compound Ex-7, Ex-8 and Ex-10 showed a diseased leaf area of at most 1%, whereas the untreated plants showed 100% diseased leaf area.

In this test, the plants which had been treated with 300 ppm of the active compound Ex-2, Ex-15, Ex-18, Ex-26, Ex-27, Ex-28 and Ex-30 showed a diseased leaf area of 0%, whereas the untreated plants showed 90% diseased leaf area.

### 6. Preventative control of brown rust on wheat caused by Puccinia recondita

The first two developed leaves of pot-grown wheat seedling were sprayed to run-off with an aqueous suspension, containing the concentration of active ingredient or their mixture as described below. Seven days later the plants were inoculated with spores of *Puccinia recondita.* To ensure the success the artificial inoculation, the plants were transferred to a humid chamber without light and a relative humidity of 95 to 99% and 20 to 24°C for 24 hours. Then the trial plants were cultivated for 6 days in a greenhouse chamber at 20 to 24°C and a relative humidity between 65 and 70%. The extent of fungal attack on the leaves was visually assessed as % diseased leaf area.

In this test, the plants which had been treated with 300 ppm of the active compound Ex-1 and Ex-7 showed a diseased leaf area of at most 16%, whereas the untreated plants showed 80% diseased leaf area.

In this test, the plants which had been treated with 300 ppm of the active compound Ex-15 showed a diseased leaf area of 12%, whereas the untreated plants showed 100% diseased leaf area.

## Claims

1. The non-therapeutic use of compounds of the formula I, or the N-oxides, or the agriculturally acceptable salts thereof, for combating phytopathogenic harmful fungi, 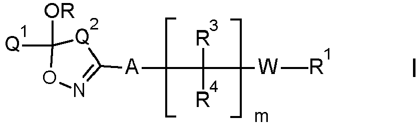 wherein:
Q¹ is CHF₂ or CF₃;
Q² is -CH₂- or -CF₂-;
R is hydrogen, C₁-C₄-alkyl, C₃-C₈-cycloalkyl, C₁-C₆-alkyl-C(=O)-O-CH₂-, C₃-C₆-cycloalkyl-C(=O)-O-CH₂-, -Si(C₁-C₄-alkyl)₃ or -(C=O)-R^{X};
R^{X} is C₁-C₆-alkyl, C₂-C₆-alkenyl, C₁-C₆-alkoxy, C₁-C₄-alkoxy-C₁-C₄-alkyl, C₃-C₈-cycloalkyl, -N(R^{Xa})₂, phenyl or a 3- to 6-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein any of the above-mentioned aliphatic or cyclic groups are unsubstituted or substituted with 1, 2, 3 or up to the maximum possible number of groups R^{xb}; wherein
R^{xa} is independently selected from the group consisting of hydrogen, C₁-C₆-alkyl, C₂-C₆-alkenyl, C₂-C₆-alkynyl, C₃-C₈-cycloalkyl, C₃-C₈-cycloalkenyl, C₁-C₆-alkoxy, C₁-C₄-alkoxy-C₁-C₄-alkyl and C₁-C₆-alkylthio;
R^{xb} is independently selected from the group consisting of halogen, C₁-C₆-alkyl, C₁-C₆-alkoxy, C₁-C₆-haloalkyl, C₁-C₆-haloalkoxy and C₃-C₈-cycloalkyl;
A is phenyl or a 5- or 6-membered aromatic heterocycle; wherein the ring member atoms of the aromatic heterocycle include besides carbon atoms 1, 2, 3 or 4 heteroatoms selected from N, O and S as ring member atoms with the provision that the heterocycle cannot contain 2 contiguous atoms selected from O and S; and wherein the phenyl ring or the aromatic heterocycle is unsubstituted or substituted with 1, 2, 3 or 4 identical or different groups R^{A}; wherein
R^{A} is halogen, cyano, C₁-C₆-alkyl, C₁-C₆-haloalkyl, C₁-C₆-alkoxy or C₁-C₆-haloalkoxy;
W is -(C=O)-NR²-#, -(C=S)-NR²-#, -S(=O)ₚ-NR²-#, -NR²-(C=O)-#, -NR²-(C=S)-#, -NR²-S(=O)ₚ-#, -NR²-(C=O)-NR²-#, -NR²-(C=S)-NR²-#, -NR²-S(=O)ₚ-NR²-#, -(C=O)-NR²-NR²-#, -(C=S)-NR²-NR²-#, -S(=O)ₚ-NR²-NR²-#, -NR²-NR²-(C=O)-#, -NR²-NR²-(C=S)-#, -NR²-NR²-S(=O)ₚ-#, -NR²-(C=O)-NR²-NR²-#, -NR²-(C=S)-NR²-NR²#, -NR²-S(=O)ₚ-NR²-NR²#, -NR²-NR²-(C=O)-NR²-#, -NR²-NR²-(C=S)-NR²-#, -NR²-NR²-S(=O)ₚ-NR²-#, -O-(C=O)-NR²-#, -O-(C=S)-NR²-#, -NR²-(C=O)-O-# or -NR²-(C=S)-O-#, wherein # denotes the position, which is attached to R¹;
p is 0, 1 or 2;
R² is independently selected from the group consisting of hydrogen, C₁-C₆-alkyl, C₂-C₆-alkenyl, C₂-C₆-alkynyl, C₁-C₆-alkoxy, C₃-C₈-cycloalkyl, C₃-C₈-cycloalkenyl, C₃-C₈-cycloalkyl-C₁-C₄-alkyl, phenyl-C₁-C₄-alkyl, phenyl, pyridinyl, C(=O)-(C₁-C₆-alkyl), C(=O)-(C₁-C₆-alkoxy) and -N(R^{2a})₂; wherein
R^{2a} is independently selected from the group consisting of hydrogen, OH, C₁-C₆-alkyl, C₂-C₆-alkenyl, C₂-C₆-alkynyl, C₃-C₈-cycloalkyl, C₃-C₈-cycloalkenyl, C₁-C₆-alkoxy, C₁-C₄-alkoxy-C₁-C₄-alkyl and C₁-C₆-alkylthio;
and wherein any of the aliphatic or cyclic groups in R² are unsubstituted or substituted with 1, 2, 3 or up to the maximum possible number of identical or different radicals selected from the group consisting of halogen, hydroxy, oxo, cyano, C₁-C₆-alkyl, C₁-C₆-alkoxy and C₃-C₈-cycloalkyl;
R¹ is C₁-C₆-alkyl, C₁-C₆-alkoxy, C₃-C₈-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O and S as ring member atoms with the provision that the heterocycle cannot contain 2 contiguous atoms selected from O and S; and wherein any of the above-mentioned aliphatic or cyclic groups are unsubstituted or substituted with 1, 2, 3 or up to the maximum possible number of identical or different groups R^{1a}; or
R¹ is a bicyclic carbocycle of the formula R^{a} 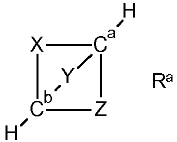 wherein
C^{a} and C^{b} are bridgehead carbon atoms;
X is a direct single bond or a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-;
Y and Z independently of each other are a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-;
or R¹ is a tricyclic carbocycle of the formula R^{b} 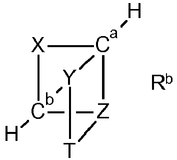
wherein
C^{a} and C^{b} are bridgehead carbon atoms;
X is a direct single bond or a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-;
Y and Z independently of each other are a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-; and wherein groups Y and Z are attached to the bridgehead carbon atoms C^{a} and C^{b};
T is a divalent group selected from the group consisting of -CH₂-, -CH₂-CH₂-, -(CH₂)₃-, -(CH₂)₄-, -CH=CH-, -CH₂-CH=CH-, -CH=CH-CH₂- and -CH=CH-CH=CH-; and wherein the group T is attached to one carbon atom in each of the groups Y and Z;
and with the proviso that, if R¹ is a tricyclic carbocycle of the formula R^{b}, wherein X is a direct single bond or a divalent group -CH₂-, the groups T and Z independently of each other are a divalent group selected from the group consisting of -CH₂-CH₂-, -(CH₂)₃-, -(CH₂)₄-, -CH=CH-, -CH₂-CH=CH-, -CH=CH-CH₂- and -CH=CH-CH=CH-; and wherein the groups R^{a} or R^{b} are connected to the group W through one of the ring carbon atoms; and wherein the groups R^{a} or R^{b} are unsubstituted or substituted with 1, 2, 3, 4 or up to the maximum possible number of radicals selected from the group consisting of oxo, hydroxy, halogen, C₁-C₃-alkyl, C₁-C₃-haloalkyl, C₃-C₆-cycloalkyl, vinylidene and dichlorovinylidene;
or R¹ and one of the groups R² together with the nitrogen atom to which R² is attached, and together with interjacent groups, if any, which are located between said nitrogen atom and the group R¹, form a saturated or partially unsaturated mono- or bicyclic 3-to 10-membered heterocycle, wherein the heterocycle includes beside one nitrogen atom and one or more carbon atoms no further heteroatoms or 1, 2 or 3 further heteroatoms independently selected from N, O and S as ring member atoms with the provision that the heterocycle cannot contain 2 contiguous atoms selected from O and S; and wherein the heterocycle is unsubstituted or substituted with 1, 2, 3, 4 or up to the maximum possible number of identical or different groups R^{1a};
or, if R² is -N(R^{2a})₂, R¹ and one of the two groups R^{2a}, together with the nitrogen atom to which R^{2a} is attached, and together with interjacent groups, which are located between said nitrogen atom and the group R¹, form a saturated or partially unsaturated mono- or bicyclic 3- to 10-membered heterocycle, wherein the heterocycle includes beside two nitrogen atoms and one or more carbon atoms no further heteroatoms or 1, 2 or 3 further heteroatoms independently selected from N, O and S as ring member atoms with the provision that the heterocycle cannot contain 2 contiguous atoms selected from O and S; and wherein the heterocycle is unsubstituted or substituted with 1, 2, 3, 4 or up to the maximum possible number of identical or different groups R^{1a};
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, (C=O)-C₁-C₄-alkyl, C(=O)-C₁-C₄-alkoxy, C₁-C₆-alkylsulfonyl, hydroxyC₁-C₄-alkyl, C(=O)-NH₂, C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl or C₁-C₄-alkoxy-C₁-C₄-alkyl;
m is 0 or 1;
R³, R⁴ independently of each other are selected from the group consisting of hydrogen, halogen, cyano, C₁-C₄-alkyl, C₁-C₄-alkenyl, C₁-C₄-alkynyl, C₁-C₄-haloalkyl and C₁-C₄-alkoxy; or
R³ and R⁴ together with the carbon atom to which they are bound form a saturated 3- to 7-membered carbocycle or a saturated 3- to 6-membered heterocycle; wherein the saturated heterocycle includes beside carbon atoms 1, 2 or 3 heteroatoms independently selected from the group consisiting of N, O and S as ring member atoms with the provision that the heterocycle cannot contain 2 contiguous atoms selected from O and S; and wherein said N ring member atom is substituted with the group R^{N}; wherein
R^{N} is hydrogen, C₁-C₆-alkyl or halogen;
and wherein said S ring member atom is unsubstituted or substituted with 1 or 2 oxo radicals; and wherein one or two CH₂ groups of the saturated carbocycle or of the saturated heterocycle may be replaced by one or two groups independently selected from -C(=O)- and -C(=S)-; and wherein the carbon ring member atoms of the saturated carbocycle or of the saturated heterocycle are unsubstituted or substituted with a total number of 1, 2, 3, 4 or up to the maximum possible number of identical or different radicals selected from the group consisting of halogen, cyano, C₁-C₆-alkyl, C₁-C₆-alkoxy and C₃-C₈-cycloalkyl.

2. The use according to claim 1, wherein A in compounds of the formula I is phenyl.

3. The non-therapeutic use of compounds of the formula I.1, or the N-oxides, or the agriculturally acceptable salts thereof, for combating phytopathogenic harmful fungi, 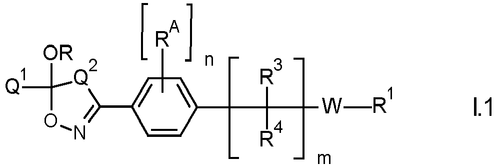 wherein n is 0 or 1, and wherein the meaning of the variables Q¹, Q², R, R^{A}, W, R², p, m, R³, R⁴ and R¹ are as defined for compounds of the formula I in claim 1.

4. The use according to any one of claims 1 to 3, wherein n is 0.

5. The use according to any one of claims 1 to 4, wherein W is -(C=O)-NR²-#, -(C=S)-NR²-#, -S(=O)ₚ-NR²-#, -NR²-(C=O)-#, -NR²-(C=S)-# or -NR²-S(=O)ₚ-#, wherein # denotes the position which is attached to R¹.

6. The use according to any one of claims 1 to 5, wherein m is 1 and R³ and R⁴ are independently selected from the group consisting of hydrogen, fluorine, chlorine, methyl or trifluoromethyl; or R³ and R⁴ together with the carbon atom to which they are bound form a cyclopropyl ring.

7. The use according to any one of claims 1 to 6, wherein R² independently of each other are selected from the group consisting of hydrogen, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, C₁-C₆-alkylamino or diC₁-C₆-alkylamino; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy.

8. The use according to any one of claims 1 to 7, wherein R is hydrogen, methyl or ethyl.

9. The use according to any one of claims 1 to 8, wherein Q² is -CH₂-.

10. The use according to any one of claims 1 to 9, wherein Q¹ is CF₃.

11. The use according to any one of claims 1 to 10, wherein m is 0.

12. Compounds of the formula I as defined in any one of claims 1 to 11, or the N-oxides, or the agriculturally acceptable salts thereof, wherein Q² is -CF₂-.

13. The non-therapeutic use of compounds of the formula I as defined in claim 12, or the N-oxides, or the agriculturally acceptable salts thereof, for combating phytopathogenic harmful fungi.

14. An agrochemical composition, which comprises an auxiliary and at least one compound of the formula I, or an N-oxide, or an agriculturally acceptable salt thereof, as defined in any one of claims 1 to 12; and further comprising seed, wherein the amount of the compound of the formula I, or an N-oxide, or an agriculturally acceptable salt thereof, is from 0.1 g to 10 kg per 100 kg of seed.

15. A non-therapeutic method for combating phytopathogenic harmful fungi, which process comprises treating the fungi, the plants, the soil or seeds to be protected against fungal attack, with an effective amount of at least one compound of formula I, or an N-oxide, or an agriculturally acceptable salt thereof, as defined in any one of claims 1 to 12.

## Patentansprüche

1. Nichttherapeutische Verwendung von Verbindungen der Formel I oder den N-Oxiden oder den landwirtschaftlich unbedenklichen Salzen davon zur Bekämpfung phytopathogener Schadpilze 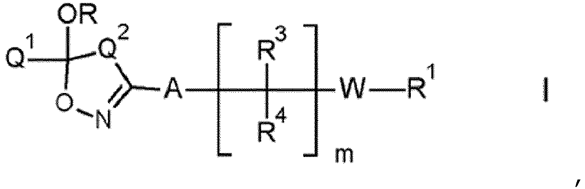 wobei:
Q¹ für CHF₂ oder CF₃ steht,
Q² für -CH₂- oder -CF₂- steht,
R für Wasserstoff, C₁-C₄-Alkyl, C₃-C₈-Cycloalkyl, C₁-C₆-Alkyl-C (=O)-O-CH₂-, C₃-C₆-Cycloalkyl-C(=O) -O-CH₂-, -Si (C₁-C₄-Alkyl)₃ oder -(C=O)-R^{X} steht,
R^{X} für C₁-C₆-Alkyl, C₂-C₆-Alkenyl, C₁-C₆-Alkoxy, C₁-C₄-Alkoxy-C₁-C₄-alkyl, C₃-C₈-Cycloalkyl, -N(R^{Xa})₂, Phenyl oder einen 3- bis 6-gliedrigen gesättigten, teilweise ungesättigten oder aromatischen mono- oder bicyclischen Heterocyclus steht, wobei die Ringgliederatome des mono- bzw. bicyclischen Heterocyclus neben Kohlenstoffatomen weitere 1,2, 3 oder 4 aus N, O und S ausgewählte Heteroatome als Ringgliederatome einschließen, mit der Maßgabe, dass der Heterocyclus nicht zwei benachbarte aus O und S ausgewählte Atome enthalten kann, und wobei die oben erwähnten aliphatischen oder cyclischen Gruppen jeweils unsubstituiert oder durch 1, 2, 3 oder bis zur maximal möglichen Anzahl an R^{xb}-Gruppen substituiert sind, wobei
R^{xa} unabhängig aus der aus Wasserstoff, C₁-C₆-Alkyl, C₂-C₆-Alkenyl, C₂-C₆-Alkinyl, C₃-C₈-Cycloalkyl, C₃-C₈-Cycloalkenyl, C₁-C₆-Alkoxy, C₁-C₄-Alkoxy-C₁-C₄-alkyl und C₁-C₆-Alkylthio bestehenden Gruppe ausgewählt ist,
R^{xb} unabhängig aus der aus Halogen, C₁-C₆-Alkyl, C₁-C₆-Alkoxy, C₁-C₆-Halogenalkyl, C₁-C₆-Halogenalkoxy und C₃-C₈-Cycloalkyl bestehenden Gruppe ausgewählt ist,
A für Phenyl oder einen 5- oder 6-gliedrigen aromatischen Heterocyclus steht, wobei die Ringgliederatome des aromatischen Heterocyclus neben Kohlenstoffatomen 1, 2, 3 oder 4 aus N, O und S ausgewählte Heteroatome als Ringgliederatome einschließen, mit der Maßgabe, dass der Heterocyclus nicht 2 benachbarte aus O und S ausgewählte Atome enthalten kann, und wobei der Phenylring bzw. der aromatische Heterocyclus unsubstituiert oder durch 1, 2, 3 oder 4 gleiche oder verschiedene R^{A}-Gruppen substituiert ist, wobei
R^{A} für Halogen, Cyano, C₁-C₆-Alkyl, C₁-C₆-Halogenalkyl, C₁-C₆-Alkoxy oder C₁-C₆-Halogenalkoxy steht,
W für - (C=O)-NR²-#, - (C=S)-NR²-#, -S (=O) p-NR²-#, -NR²-(C=O)-#, -NR²-(C=S)-#, -NR²-S(=O)ₚ-#,-NR²-(C=O)-NR²-#, -NR²-(C=S)-NR²-#, -NR²-S(=O)ₚ-NR²-#, - (C=O)-NR²-NR²-#, - (C=S)-NR²-NR²-#,-S(=O)ₚ-NR²-NR²-#, -NR²-NR²-(C=O)-#, -NR²-NR²-(C=S) -#, -NR²-NR²-S(=O)ₚ-#, -NR²-(C=O)-NR²-NR²#, -NR²-(C=S)-NR²-NR²-#, -NR²-S(=O)ₚ-NR²-NR²-#, -NR²-NR²-(C=O)-NR²#, -NR²-NR²-(C=S)-NR²-#,-NR²-NR²-S(=O)ₚ-NR²-#, -O-(C=O)-NR²-#, -O-(C=S)-NR²-#, -NR²-(C=O)-O-# oder -NR²-(C=S)-O-# steht, wobei # die Position kennzeichnet, die an R¹ gebunden ist,
p für 0, 1 oder 2 steht,
R² unabhängig aus der aus Wasserstoff, C₁-C₆-Alkyl, C₂-C₆-Alkenyl, C₂-C₆-Alkinyl, C₁-C₆-Alkoxy, C₃-C₈-Cycloalkyl, C₃-C₈-Cycloalkenyl, C₃-C₈-Cycloalkyl-C₁-C₄-alkyl, Phenyl-C₁-C₄-Alkyl, Phenyl, Pyridinyl, C(=O)-(C₁-C₆-Alkyl), C (=O) - (C₁-C₆-Alkoxy) und -N(R^{2a})₂ bestehenden Gruppe ausgewählt ist, wobei
R^{2a} unabhängig aus der aus Wasserstoff, OH, C₁-C₆-Alkyl, C₂-C₆-Alkenyl, C₂-C₆-Alkinyl, C₃-C₈-Cycloalkyl, C₃-C₈-Cycloalkenyl, C₁-C₆-Alkoxy, C₁-C₄-Alkoxy-C₁-C₄-alkyl und C₁-C₆-Alkylthio bestehenden Gruppe ausgewählt ist,
und wobei die aliphatischen bzw. cyclischen Gruppen in R² jeweils unsubstituiert oder durch 1, 2, 3 oder bis zur maximal möglichen Anzahl an gleichen oder verschiedenen, aus der aus Halogen, Hydroxy, Oxo, Cyano, C₁-C₆-Alkyl, C₁-C₆-Alkoxy und C₃-C₈-Cycloalkyl bestehenden Gruppe ausgewählte Reste substituiert sind,
R¹ für C₁-C₆-Alkyl, C₁-C₆-Alkoxy, C₃-C₈-Cycloalkyl, C₃-C₈-Cycloalkenyl, C₂-C₆-Alkenyl, C₂-C₆-Alkinyl, C₁-C₆-Alkoxyimino-C₁-C₄-alkyl, C₂-C₆-Alkenyloxyimino-C₁-C₄-alkyl, C₂-C₆-Alkinyloxyimino-C₁-C₄-alkyl, Phenyl-C₁-C₄-alkyl, Phenyl-C₁-C₄-alkenyl, Phenyl-C₁-C₄-alkinyl, Heteroaryl-C₁-C₄-alkyl, Phenyl, Naphthyl oder einen 3- bis 10-gliedrigen gesättigten, teilweise ungesättigten oder aromatischen mono- oder bicyclischen Heterocyclus steht, wobei die Ringgliederatome des mono- bzw. bicyclischen Heterocyclus neben Kohlenstoffatomen weitere 1, 2, 3 oder 4 aus N, O und S ausgewählte Heteroatome als Ringgliederatome einschließen, mit der Maßgabe, dass der Heterocyclus nicht 2 benachbarte aus O und S ausgewählte Atome enthalten kann, und wobei es sich bei der Heteroarylgruppe in der Heteroaryl-C₁-C₄-alkyl-Gruppe um einen 5- oder 6-gliedrigen aromatischen Heterocyclus handelt, wobei die Ringgliederatome des heterocyclischen Rings neben Kohlenstoffatomen 1,2, 3 oder 4 aus N, O und S ausgewählte Heteroatome als Ringgliederatome einschließen, mit der Maßgabe, dass der Heterocyclus nicht 2 benachbarte aus O und S ausgewählte Atome enthalten kann, und wobei die oben erwähnten aliphatischen bzw. cyclischen Gruppen jeweils unsubstituiert oder durch 1, 2, 3 oder bis zur maximal möglichen Anzahl an gleichen oder verschiedenen R^{1a}-Gruppen substituiert sind, oder
R¹ für einen bicyclischen Carbocyclus der Formel R^{a} steht 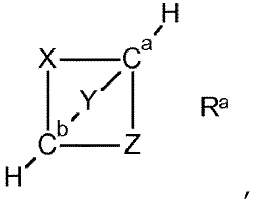
wobei
C^{a} und C^{b} für Brückenkopf-Kohlenstoffatome stehen,
X für eine direkte Einfachbindung oder eine aus der aus -CH₂-, -CH₂-CH₂-, - (CH₂)₃-, -(CH₂)₄-, -CH=CH-, -CH₂-CH=CH-, -CH=CH-CH₂-und -CH=CH-CH=CH- bestehenden Gruppe ausgewählte zweiwertige Gruppe steht,
Y und Z unabhängig voneinander für eine aus der aus -CH₂-, -CH₂-CH₂-, -(CH₂)₃-, -(CH₂)₄-, -CH=CH-, -CH₂-CH=CH-, -CH=CH-CH₂- und -CH=CH-CH=CH- bestehenden Gruppe ausgewählte zweiwertige Gruppe stehen, oder R¹ für einen tricyclischen Carbocyclus der Formel R^{b} steht 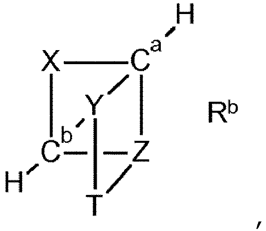
wobei
C^{a} und C^{b} für Brückenkopf-Kohlenstoffatome stehen,
X für eine direkte Einfachbindung oder eine aus der aus -CH₂-, -CH₂-CH₂-, -(CH₂)₃-, -(CH₂)₄-, -CH=CH-, -CH₂-CH=CH-, -CH=CH-CH₂- und -CH=CH-CH=CH- bestehenden Gruppe ausgewählte zweiwertige Gruppe steht,
Y und Z unabhängig voneinander für eine aus der aus -CH₂-, -CH₂-CH₂-, -(CH₂)₃-, -(CH₂)₄-, -CH=CH-, -CH₂-CH=CH-, -CH=CH-CH₂- und -CH=CH-CH=CH- bestehenden Gruppe ausgewählte zweiwertige Gruppe stehen, und wobei die Gruppen Y und Z an die Brückenkopf-Kohlenstoffatome C^{a} und C^{b} gebunden sind,
T für eine aus der aus -CH₂-, -CH₂-CH₂-, -(CH₂)₃-, -(CH₂)₄-, -CH=CH-, -CH₂CH=CH-, -CH=CH-CH₂- und -CH=CH-CH=CH- bestehenden Gruppe ausgewählte zweiwertige Gruppe steht, und wobei die Gruppe T an ein Kohlenstoffatom in jeder der Gruppen Y und Z gebunden ist,und mit der Maßgabe, dass, wenn R¹ für einen tricyclischen Carbocyclus der Formel R^{b} steht, in welcher X für eine direkt Einfachbindung oder eine zweiwertige Gruppe -CH₂- steht, die Gruppen T und Z unabhängig voneinander für eine aus der aus -CH₂-CH₂-, -(CH₂)₃-, -(CH₂)₄-, -CH=CH-, -CH₂-CH=CH-, -CH=CH-CH₂- und -CH=CH-CH=CH- bestehenden Gruppe ausgewählte zweiwertige Gruppe stehen, und wobei die Gruppen R^{a} bzw. R^{b} über eines der Ringkohlenstoffatome an die Gruppe W gebunden sind, und wobei die Gruppen R^{a} bzw. R^{b} unsubstituiert oder durch 1, 2, 3, 4 oder bis zur maximal möglichen Anzahl an aus der aus Oxo, Hydroxy, Halogen, C₁-C₃-Alkyl, C₁-C₃-Halogenalkyl, C₃-C₆-Cycloalkyl, Vinyliden und Dichlorvinyliden bestehenden Gruppe ausgewählte Reste substituiert sind, oder R¹ und eine der R²-Gruppen zusammen mit dem Stickstoffatom, an das R² gebunden ist, und zusammen mit den dazwischenliegenden Gruppen, falls vorhanden, die sich zwischen dem Stickstoffatom und der R¹-Gruppe befinden, einen gesättigten oder teilweise ungesättigten mono- oder bicyclischen 3- bis 10-gliedrigen Heterocyclus bilden, wobei der Heterocyclus neben einem Stickstoffatom und einem oder mehreren Kohlenstoffatomen keine weiteren Heteroatome oder 1, 2 oder 3 weitere, unabhängig aus N, O und S ausgewählte Heteroatome als Ringgliederatome einschließt, mit der Maßgabe, dass der Heterocyclus keine 2 benachbarten aus O und S ausgewählte Atome enthalten kann, und wobei der Heterocyclus unsubstituiert oder durch 1, 2, 3, 4 oder bis zur maximal möglichen Anzahl an gleichen oder verschiedenen R^{1a}-Gruppen substituiert ist,
oder, wenn R² für -N(R^{2a})₂ steht, R¹ und eine der beiden R^{2a}-Gruppen, zusammen mit dem Stickstoffatom, an das R^{2a} gebunden ist, und zusammen mit dazwischenliegenden Gruppen, die sich zwischen dem Stickstoffatom und der R¹-Gruppe befinden, einen gesättigten oder teilweise ungesättigten mono- oder bicyclischen 3- bis 10-gliedrigen Heterocyclus bilden, wobei der Heterocyclus neben zwei Stickstoffatomen und einem oder mehreren Kohlenstoffatomen keine weiteren Heteroatome oder 1, 2 oder 3 weitere unabhängig aus N, O und S ausgewählte Heteroatome als Ringgliederatome einschließt, mit der Maßgabe, dass der Heterocyclus keine 2 benachbarten aus O und S ausgewählte Atome enthalten kann, und wobei der Heterocyclus unsubstituiert oder durch 1, 2, 3, 4 oder bis zur maximal möglichen Anzahl an gleichen oder verschiedenen R^{1a}-Gruppen substituiert ist,
R^{1a} für Halogen, Oxo, Cyano, NO₂, OH, SH, NH₂-C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, -NHSO₂-C₁-C₄-Alkyl, (C=O)-C₁-C₄-Alkyl, C (=O) -C₁-C₄-Alkoxy, C₁-C₆-Alkylsulfonyl, Hydroxy-C₁-C₄-alkyl, C(=O)-NH₂, C(=O)-NH(C₁-C₄-Alkyl), C₁-C₄-Alkylthio-C₁-C₄-alkyl, Amino-C₁-C₄-alkyl, C₁-C₄-Alkylamino-C₁-C₄-alkyl, Di-C₁-C₄-alkylamino-C₁-C₄-alkyl, Aminocarbonyl-C₁-C₄-alkyl oder C₁-C₄-Alkoxy-C₁-C₄-Alkyl steht,
m für 0 oder 1 steht,
R³, R⁴ unabhängig voneinander aus der aus Wasserstoff, Halogen, Cyano, C₁-C₄-Alkyl, C₁-C₄-Alkenyl, C₁-C₄-Alkinyl, C₁-C₄-Halogenalkyl und C₁-C₄-Alkoxy bestehenden Gruppe ausgewählt sind oder
R³ und R⁴ zusammen mit dem Kohlenstoffatom, an das sie gebunden sind, einen gesättigten 3- bis 7-gliedrigen Carbocyclus oder einen gesättigten 3- bis 6-gliedrigen Heterocyclus bilden, wobei der gesättigte Heterocyclus neben Kohlenstoffatomen 1, 2 oder 3 unabhängig voneinander aus der aus N, O und S bestehenden Gruppe ausgewählte Heteroatome als Ringgliederatome einschließt, mit der Maßgabe, dass der Heterocyclus nicht 2 benachbarte aus O und S ausgewählte Atome enthalten kann, und wobei das N-Ringgliedatom durch die Gruppe R^{N} substituiert ist, wobei R^{N} für Wasserstoff, C₁-C₆-Alkyl oder Halogen steht,
und wobei das S-Ringgliedatom unsubstituiert oder durch 1 oder 2 Oxoreste substituiert ist, und wobei einer oder zwei CH₂-Gruppen des gesättigten Carbocyclus bzw. des gesättigten Heterocyclus durch ein oder zwei unabhängig voneinander aus -C(=O)- und -C(=S)-ausgewählte Gruppen ersetzt sein können, und wobei die Kohlenstoffringgliederatome des gesättigten Carbocyclus bzw. des gesättigten Heterocyclus unsubstituiert oder durch eine Gesamtanzahl an 1, 2, 3, 4 oder bis zur maximal möglichen Anzahl an gleichen oder verschiedenen, aus der aus Halogen, Cyano, C₁-C₆-Alkyl, C₁-C₆-Alkoxy und C₃-C₈-Cycloalkyl bestehenden Gruppe ausgewählte Reste substituiert sind.

2. Verwendung nach Anspruch 1, wobei A in den Verbindungen der Formel I für Phenyl steht.

3. Nichttherapeutische Verwendung von Verbindungen der Formel I.1 oder der N-Oxide oder der landwirtschaftlich unbedenklichen Salze davon zur Bekämpfung phytopathogener Schadpilze 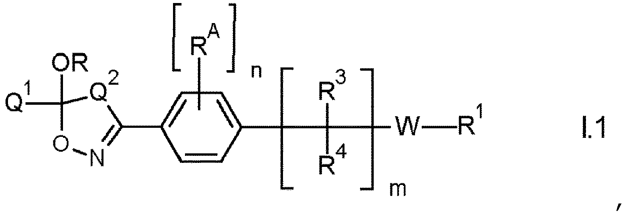 wobei n für 0 oder 1 steht und wobei die Bedeutung der Variablen Q¹, Q², R, R^{A}, W, R², p, m, R³, R⁴ und R¹ wie für Verbindungen der Formel I in Anspruch 1 definiert sind.

4. Verwendung nach einem der Ansprüche 1 bis 3, wobei n für 0 steht.

5. Verwendung nach einem der Ansprüche 1 bis 4, wobei W für -(C=O)-NR²-#, -(C=S)-NR²-#, -S(=O)ₚ-NR²-#, -NR²-(C=O)-#, -NR²-(C=S)-# oder -NR²-S(=O)ₚ-# steht, wobei # die Position, die an R¹ gebunden ist, kennzeichnet.

6. Verwendung nach einem der Ansprüche 1 bis 5, wobei m für 1 steht und R³ und R⁴ unabhängig voneinander aus der aus Wasserstoff, Fluor, Chlor, Methyl und Trifluormethyl bestehenden Gruppe ausgewählt sind oder R³ und R⁴ zusammen mit dem Kohlenstoffatom, an das sie gebunden sind, einen Cyclopropylring bilden.

7. Verwendung nach einem der Ansprüche 1 bis 6, wobei R² unabhängig voneinander aus der aus Wasserstoff, C₁-C₆-Alkyl, C₁-C₆-Alkoxy, C₂-C₆-Alkenyl, Propargyl, C₃-C₈-Cycloalkyl, C₃-C₈-Cycloalkenyl, C₃-C₈-Cycloalkyl-C₁-C₄-alkyl, Phenyl, C₁-C₆-Alkylamino und Di-C₁-C₆-alkylamino bestehenden Gruppe ausgewählt ist und wobei die aliphatischen bzw. cyclischen Gruppen jeweils unsubstituiert oder durch 1, 2, 3, 4 oder bis zur maximal möglichen Anzahl an gleichen oder verschiedenen, aus der aus Halogen, Cyano, C₁-C₆-Alkyl und C₁-C₆-Alkoxy bestehenden Gruppe ausgewählte Reste substituiert sind.

8. Verwendung nach einem der Ansprüche 1 bis 7, wobei R für Wasserstoff, Methyl oder Ethyl steht.

9. Verwendung nach einem der Ansprüche 1 bis 8, wobei Q² für -CH₂- steht.

10. Verwendung nach einem der Ansprüche 1 bis 9, wobei Q¹ für CF₃ steht.

11. Verwendung nach einem der Ansprüche 1 bis 10, wobei m für 0 steht.

12. Verbindungen der Formel I, definiert wie in einem der Ansprüche 1 bis 11, oder die N-Oxide oder die landwirtschaftlich unbedenklichen Salze davon, wobei Q² für -CF₂- steht.

13. Nichttherapeutische Verwendung von wie in Anspruch 12 definierten Verbindungen der Formel I oder den N-Oxiden oder den landwirtschaftlich unbedenklichen Salzen davon zur Bekämpfung phytopathogener Schadpilze.

14. Agrochemische Zusammensetzung, umfassend einen Hilfsstoff und mindestens eine Verbindung der Formel I oder ein N-Oxid oder ein landwirtschaftlich unbedenkliches Salz davon, wie in einem der Ansprüche 1 bis 12 definiert, und weiterhin umfassend Saatgut, wobei die Menge der Verbindung der Formel I oder eines N-Oxids oder eines landwirtschaftlich unbedenklichen Salzes davon 0,1 g bis 10 kg pro 100 kg Saatgut beträgt.

15. Nichttherapeutisches Verfahren zur Bekämpfung phytopathogener Schadpilze, wobei das Verfahren die Behandlung der Pilze, der Pflanzen, des Bodens oder von gegen Pilzbefall zu schützenden Samen mit einer wirksamen Menge mindestens einer Verbindung der Formel I oder eines N-Oxids oder eines landwirtschaftlich unbedenklichen Salzes davon, definiert wie in einem der Ansprüche 1 bis 12, umfasst.

## Revendications

1. Utilisation non thérapeutique de composés de la formule I, ou des N-oxydes, ou des sels acceptables sur le plan agricole correspondants, pour la lutte contre des champignons nuisibles phytopathogènes, 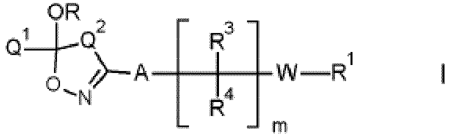
Q¹ étant CHF₂ ou CF₃ ;
Q² étant -CH₂- ou -CF₂- ;
R étant hydrogène, C₁-C₄-alkyle, C₃-C₈-cycloalkyle, C₁-C₆-alkyl-C(=O)-O-CH₂-, C₃-C₆-cycloalkyl-C(=O)-O-CH₂-, -Si (C₁-C₄-alkyle)₃ ou -(C=O)-R^{x};
R^{x} étant C₁-C₆-alkyle, C₂-C₆-alcényle, C₁-C₆-alcoxy, C₁-C₄-alcoxy-C₁-C₄-alkyle, C₃-C₈-cycloalkyle,-N(R^{xa})₂, phényle ou un hétérocycle saturé, partiellement insaturé ou aromatique, monocyclique ou bicyclique, à 3 à 6 chaînons, les atomes de cycle dudit hétérocycle monocyclique ou bicyclique comprenant outre les atomes de carbone en outre 1, 2, 3 ou 4 hétéroatomes choisis parmi N, O et S en tant qu'atomes de cycle à la condition que l'hétérocycle ne peut pas contenir 2 atomes contigus choisis parmi O et S ; et tous les groupes aliphatiques ou cycliques mentionnés ci-dessus étant non substitués ou substitués par 1, 2, 3 ou jusqu'au nombre maximal possible de groupes R^{xb};
R^{xa} étant indépendamment choisi dans le groupe constitué par hydrogène, C₁-C₆-alkyle, C₂-C₆-alcényle, C₂-C₆-alcynyle, C₃-C₈-cycloalkyle, C₃-C₈-cycloalcényle, C₁-C₆-alcoxy, C₁-C₄-alcoxy-C₁-C₄-alkyle et C₁-C₆-alkylthio ;
R^{xb} étant indépendamment choisi dans le groupe constitué par halogène, C₁-C₆-alkyle, C₁-C₆-alcoxy, C₁-C₆-halogénoalkyle, C₁-C₆-halogénoalcoxy et C₃-C₃-cycloalkyle ;
A étant phényle ou un hétérocycle aromatique à 5 ou 6 chaînons ; les atomes de cycle de l'hétérocycle aromatique comprenant outre les atomes de carbone 1, 2, 3 ou 4 hétéroatomes choisis parmi N, O et S en tant qu'atomes de cycle à la condition que l'hétérocycle ne peut pas contenir 2 atomes contigus choisis parmi O et S ; et le cycle phényle ou l'hétérocycle aromatique étant non substitué ou substitué par 1, 2, 3 ou 4 groupes R^{A} identiques ou différents ;
R^{A} étant halogène, cyano, C₁-C₆-alkyle, C₁-C₆-halogénoalkyle, C₁-C₆-alcoxy ou C₁-C₆-halogénoalcoxy;
W étant -(C=O)-NR²-#, -(C=S)-NR²-#, -S(=O)_{P}-NR²-#, -NR²-(C=O)-#, -NR²-(C=S)-#, -NR²-S(=O)ₚ-#, -NR²-(C=O)-NR²-#, -NR²-(C=S)-NR²-#, -NR²-S(=O)_{P}-NR²-#, -(C=O)-NR²-NR²-#, -(C=S)-NR²-NR²-#, -S(=O)_{P}-NR²-NR²-#, -NR²-NR²-(C=O)-#, -NR²-NR²-(C=S)-#, -NR²-NR²-S(=O)ₚ-#, -NR²-(C=O)-NR²-NR²-#, -NR²-(C=S)-NR²-NR²#, -NR²-S(=O)_{P}-NR²-NR²#, -NR²-NR²-(C=O)-NR²-#, -NR²-NR²-(C=S)-NR²-#, -NR²-NR²-S(=O)_{P}-NR²-#, -O-(C=O)-NR²-#, -O-(C=S)-NR²-#, -NR²-(C=O)-O-# ou -NR²-(C=S)-O-#, # désignant la position, qui est fixée à R¹;
p étant 0, 1 ou 2 ;
R² étant indépendamment choisi dans le groupe constitué par hydrogène, C₁-C₆-alkyle, C₂-C₆-alcényle, C₂-C₆-alcynyle, C₁-C₆-alcoxy, C₃-C₈-cycloalkyle, C₃-C₈-cycloalcényle, C₃-C₈-cycloalkyl-C₁-C₄-alkyle, phényl-C₁-C₄-alkyle, phényle, pyridinyle, C(=O)-(C₁-C₆-alkyle), C(=O)-(C₁-C₆-alcoxy) et -N(R^{2a})₂;
R^{2a} étant indépendamment choisi dans le groupe constitué par hydrogène, OH, C₁-C₆-alkyle, C₂-C₆-alcényle, C₂-C₆-alcynyle, C₃-C₈-cycloalkyle, C₃-C₈-cycloalcényle, C₁-C₆-alcoxy, C₁-C₄-alcoxy-C₁-C₄-alkyle et C₁-C₆-alkylthio;
et tous les groupes aliphatiques ou cycliques dans R² étant non substitués ou substitués par 1, 2, 3 ou jusqu'au nombre maximal possible de radicaux identiques ou différents choisis dans le groupe constitué par halogène, hydroxy, oxo, cyano, C₁-C₆-alkyle, C₁-C₆-alcoxy et C₃-C₈-cycloalkyle;
R¹ étant C₁-C₆-alkyle, C₁-C₆-alcoxy, C₃-C₈-cycloalkyle, C₃-C₈-cycloalcényle, C₂-C₆-alcényle, C₂-C₆-alcynyle, C₁-C₆-alcoxyimino-C₁-C₄-alkyle, C₂-C₆-alcényloxyimino-C₁-C₄-alkyle, C₂-C₆-alcynyloxyimino-C₁-C₄-alkyle, phényl-C₁-C₄-alkyle, phényl-C₁-C₄-alcényle, phényl-C₁-C₄-alcynyle, hétéroaryl-C₁-C₄-alkyle, phényle, naphtyle ou un hétérocycle saturé, partiellement insaturé ou aromatique, monocyclique ou bicyclique, à 3 à 10 chaînons, les atomes de cycle dudit hétérocycle monocyclique ou bicyclique comprenant outre les atomes de carbone en outre 1, 2, 3 ou 4 hétéroatomes choisis parmi N, O et S en tant qu'atomes de cycle à la condition que l'hétérocycle ne peut pas contenir 2 atomes contigus choisis parmi O et S ; et le groupe hétéroaryle dans le groupe hétéroaryl-C₁-C₄-alkyle étant un hétérocycle aromatique à 5 ou 6 chaînons, les atomes de cycle du cycle hétérocyclique comprenant outre les atomes carbone 1, 2, 3 ou 4 hétéroatomes choisis parmi N, O et S en tant qu'atomes de cycle à la condition que l'hétérocycle ne peut pas contenir 2 atomes contigus choisis parmi O et S ; et tous les groupes aliphatiques ou cycliques mentionnés ci-dessus étant non substitués ou substitués par 1, 2, 3 ou jusqu'au nombre maximal possible de groupes R^{1a} identiques ou différents ; ou
R¹ étant un carbocycle bicyclique de la formule R^{a} 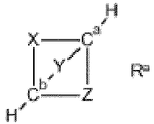
C^{a} et C^{b} étant des atomes de carbone de tête de pont ;
X étant une simple liaison directe ou un groupe divalent choisi dans le groupe constitué par -CH₂-,-CH₂-CH₂-, - (CH₂)₃-, -(CH₂)₄-, -CH=CH-, -CH₂-CH=CH-,-CH=CH-CH₂- et -CH=CH-CH=CH- ;
Y et Z indépendamment l'un de l'autre étant un groupe divalent choisi dans le groupe constitué par-CH₂-, -CH₂-CH₂-, -(CH₂)₃-, -(CH₂)₄-, -CH=CH-, -CH₂-CH=CH-, -CH=CH-CH₂- et -CH=CH-CH=CH- ;
ou R¹ étant un carbocycle tricyclique de la formule Rb 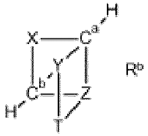
C^{a} et C^{b} étant des atomes de carbone de tête de pont ;
X étant une simple liaison directe ou un groupe divalent choisi dans le groupe constitué par -CH₂-,-CH₂-CH₂-, -(CH₂)₃-, -(CH₂)₄-, -CH=CH-, -CH₂-CH=CH-,-CH=CH-CH₂- et -CH=CH-CH=CH- ;
Y et Z indépendamment l'un de l'autre étant un groupe divalent choisi dans le groupe constitué par-CH₂-, -CH₂-CH₂-, -(CH₂)₃-, -(CH₂)₄-, -CH=CH-, -CH₂-CH=CH-, -CH=CH-CH₂- et -CH=CH-CH=CH- ; et les groupes Y et Z étant fixés aux atomes de carbone de tête de pont C^{a} et C^{b};
T étant un groupe divalent choisi dans le groupe constitué par -CH₂-, -CH₂-CH₂-, -(CH₂)₃-, -(CH₂)₄-, -CH=CH-, -CH₂-CH=CH-, -CH=CH-CH₂- et -CH=CH-CH=CH- ; et le groupe T étant fixé à un atome de carbone dans chacun des groupes Y et Z ;
et à la condition que si R¹ est un carbocycle tricyclique de la formule R^{b}, X est une simple liaison directe ou un groupe divalent -CH₂-, les groupes T et Z indépendamment l'un de l'autre sont un groupe divalent choisi dans le groupe constitué par -CH₂-CH₂-, -(CH₂)₃-, -(CH₂)₄-, -CH=CH-, -CH₂-CH=CH-, -CH=CH-CH₂- et -CH=CH-CH=CH- ; et les groupes R^{a} ou R^{b} sont reliés au groupe W par l'intermédiaire de l'un des atomes de carbone de cycle ; et les groupes R^{a} ou R^{b} sont non substitués ou substitués par 1, 2, 3, 4 ou jusqu'au nombre maximal possible de radicaux choisis dans le groupe constitué par oxo, hydroxy, halogène, C₁-C₃-alkyle, C₁-C₃-halogénoalkyle, C₃-C₆-cycloalkyle, vinylidène et dichlorovinylidène ;
ou R¹ et l'un des groupes R², conjointement avec l'atome d'azote auquel R² est fixé, et conjointement avec des groupes interjacents, le cas échéant, qui sont situés entre ledit atome d'azote et le groupe R¹, formant un hétérocycle saturé, partiellement insaturé, monocyclique ou bicyclique, à 3 à 10 chaînons, l'hétérocycle comprenant outre un atome d'azote et un ou plusieurs atomes de carbone aucun autre hétéroatome ou 1, 2 ou 3 hétéroatomes supplémentaires indépendamment choisis parmi N, O et S en tant qu'atomes de cycle à la condition que l'hétérocycle ne peut pas contenir 2 atomes contigus choisis parmi O et S ; et l'hétérocycle étant non substitué ou substitué par 1, 2, 3, 4 ou jusqu'au nombre maximal possible de groupes R^{1a} identiques ou différents ;
ou, si R² est -N(R^{2a})₂, R¹ et l'un des deux groupes R^{2a}, conjointement avec l'atome d'azote auquel R^{2a} est fixé, et conjointement avec des groupes interjacents qui sont situés entre ledit atome d'azote et le groupe R¹, formant un hétérocycle saturé ou partiellement insaturé, monocyclique ou bicyclique, à 3 à 10 chaînons, l'hétérocycle comprenant outre deux atomes d'azote et un ou plusieurs atomes de carbone aucun autre hétéroatome ou 1, 2 ou 3 hétéroatomes supplémentaires indépendamment choisis parmi N, O et S en tant qu'atomes de cycle à la condition que l'hétérocycle ne peut pas contenir 2 atomes contigus choisis parmi O et S ; et l'hétérocycle étant non substitué ou substitué par 1, 2, 3, 4 ou jusqu'au nombre maximal possible de groupes R^{1a} identiques ou différents ;
R^{1a} étant halogène, oxo, cyano, NO₂, OH, SH, NH₂, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, -NHSO₂-C₁-C₄-alkyle, (C=O)-C₁-C₄-alkyle, C(=O)-C₁-C₄-alcoxy, C₁-C₆-alkylsulfonyle, hydroxyC₁-C₄-alkyle, C(=O)-NH₂, C(=O)-NH(C₁-C₄-alkyle), C₁-C₄-alkylthio-C₁-C₄-alkyle, aminoC₁-C₄-alkyle, C₁-C₄-alkylamino-C₁-C₄-alkyle, diC₁-C₄-alkylamino-C₁-C₄-alkyle, aminocarbonyl-C₁-C₄-alkyle ou C₁-C₄-alcoxy-C₁-C₄-alkyle ;
m étant 0 ou 1 ;
R³, R⁴ indépendamment l'un de l'autre étant choisis dans le groupe constitué par hydrogène, halogène, cyano, C₁-C₄-alkyle, C₁-C₄-alcényle, C₁-C₄-alcynyle, C₁-C₄-halogénoalkyle et C₁-C₄-alcoxy ; ou
R³ et R⁴ conjointement avec l'atome de carbone auquel ils sont liés formant un carbocycle saturé à 3 à 7 chaînons ou un hétérocycle saturé à 3 à 6 chaînons ; l'hétérocycle saturé comprenant outre les atomes de carbone 1, 2 ou 3 hétéroatomes indépendamment choisis dans le groupe constitué par N, O et S en tant qu'atomes de cycle à la condition que l'hétérocycle ne peut pas contenir 2 atomes contigus choisis parmi O et S ; et ledit atome N de cycle étant substitué par le groupe R^{N} ;
R^{N} étant hydrogène, C₁-C₆-alkyle ou halogène ;
et ledit atome S de cycle étant non substitué ou substitué par 1 ou 2 radicaux oxo ; et un ou deux groupes CH₂ du carbocycle saturé ou de l'hétérocycle saturé pouvant être remplacés par un ou deux groupes indépendamment choisis parmi -C(=O)- et -C(=S)- ; et les atomes de carbone de cycle du carbocycle saturé ou de l'hétérocycle saturé étant non substitués ou substitués par nombre total de 1, 2, 3, 4 ou jusqu'au nombre maximal possible de radicaux identiques ou différents choisis dans le groupe constitué par halogène, cyano, C₁-C₆-alkyle, C₁-C₆-alcoxy et C₃-C₈-cycloalkyle.

2. Utilisation selon la revendication 1, A étant phényle dans les composés de la formule I.

3. Utilisation non thérapeutique de composés de la formule I.1, ou des N-oxydes, ou des sels acceptables sur le plan agricole correspondants, pour la lutte contre des champignons nuisibles phytopathogènes, 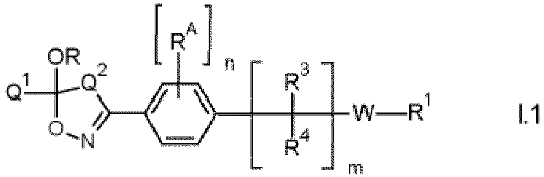 n étant 0 ou 1, et la signification des variables Q¹, Q², R, R^{A}, W, R², p, m, R³, R⁴ et R¹ étant telle que définie pour des composés de la formule I dans la revendication 1.

4. Utilisation selon l'une quelconque des revendications 1 à 3, n étant 0.

5. Utilisation selon l'une quelconque des revendications 1 à 4, W étant -(C=O)-NR²-#, -(C=S)-NR²-#, -S(=O)ₚ-NR²-#, -NR²-(C=O)-#, -NR²-(C=S)-# ou -NR²-S(=O)ₚ-#, # désignant la position qui est fixée à R¹.

6. Utilisation selon l'une quelconque des revendications 1 à 5, m étant 1 et R³ et R⁴ étant indépendamment choisis dans le groupe constitué par hydrogène, fluor, chlore, méthyle et trifluorométhyle ; ou R³ et R⁴ conjointement avec l'atome de carbone auquel ils sont liés formant un cycle cyclopropyle.

7. Utilisation selon l'une quelconque des revendications 1 à 6, R² étant indépendamment les uns des autres choisis dans le groupe constitué par hydrogène, C₁-C₆-alkyle, C₁-C₆-alcoxy, C₂-C₆-alcényle, propargyle, C₃-C₈-cycloalkyle, C₃-C₈-cycloalcényle, C₃-C₈-cycloalkyl-C₁-C₄-alkyle, phényle, C₁-C₆-alkylamino et diC₁-C₆-alkylamino ; et tous les groupes aliphatiques et/ou cycliques étant non substitués ou substitués par 1, 2, 3, 4 ou jusqu'au nombre maximal possible de radicaux identiques ou différents choisis dans le groupe constitué par halogène, cyano, C₁-C₆-alkyle et C₁-C₆-alcoxy.

8. Utilisation selon l'une quelconque des revendications 1 à 7, R étant hydrogène, méthyle ou éthyle.

9. Utilisation selon l'une quelconque des revendications 1 à 8, Q² étant -CH₂-.

10. Utilisation selon l'une quelconque des revendications 1 à 9, Q¹ étant CF₃.

11. Utilisation selon l'une quelconque des revendications 1 à 10, m étant 0.

12. Composés de la formule I tels que définis dans l'une quelconque des revendications 1 à 11, ou N-oxydes, ou sels acceptables sur le plan agricole correspondants, Q² étant -CF₂-.

13. Utilisation non thérapeutique de composés de la formule I tels que définis dans la revendication 12, ou des N-oxydes, ou des sels acceptables sur le plan agricole correspondants, pour la lutte contre des champignons nuisibles phytopathogènes.

14. Composition agrochimique, qui comprend un auxiliaire et au moins un composé de la formule I, ou un N-oxyde, ou un sel acceptable sur le plan agricole correspondant, tel que défini dans l'une quelconque des revendications 1 à 12 ; et comprenant en outre des graines, la quantité du composé de la formule I, ou d'un N-oxyde, ou d'un sel acceptable sur le plan agricole correspondant, étant de 0,1 g à 10 kg par 100 kg de graines.

15. Procédé non thérapeutique pour la lutte contre des champignons nuisibles phytopathogènes, lequel procédé comprend le traitement des champignons, des végétaux, du sol ou des graines devant être protégé(e)s contre une attaque fongique, avec une quantité efficace d'au moins un composé de la formule I, ou d'un N-oxyde, ou d'un sel acceptable sur le plan agricole correspondant, tel que défini dans l'une quelconque des revendications 1 à 12.
